(19) Europäisches Patentamt
European Patent Office
Office européen des brevets

(11) EP 0 930 288 A1

(12) **EUROPEAN PATENT APPLICATION**
published in accordance with Art. 158(3) EPC

(43) Date of publication:
**21.07.1999 Bulletin 1999/29**

(21) Application number: **97942217.7**

(22) Date of filing: **01.10.1997**

(51) Int. Cl.$^6$: **C07C 22/08**, C07C 43/172, C07C 43/192, C07C 43/225, C07D 239/26, C07D 309/04, C07D 309/06, C07D 319/06, C07D 335/02, C07D 339/08, C09K 19/30

(86) International application number:
**PCT/JP97/03503**

(87) International publication number:
**WO 98/14418 (09.04.1998 Gazette 1998/14)**

(84) Designated Contracting States:
**DE FR GB**

(30) Priority: **02.10.1996 JP 28136996**

(71) Applicant: **CHISSO CORPORATION**
**Osaka-shi Osaka 530-6591 (JP)**

(72) Inventors:
- **MATSUI, Shuichi**
  **Ichhihara-shi, Chiba 290 (JP)**
- **ANDO, Tsugumichi**
  **Minamata-shi, Kumamoto 867 (JP)**
- **MIYAZAWA, Kazutoshi**
  **Ichihara-shi, Chiba 290-01 (JP)**
- **TAKEUCHI, Hiroyuki**
  **Ichihara-shi, Chiba 290 (JP)**
- **TAKESHITA, Fusayuki**
  **Kimitsu-shi, Chiba 299-11 (JP)**
- **NAKAGAWA, Etsuo**
  **Chiba 290 (JP)**

(74) Representative: **HOFFMANN - EITLE**
**Patent- und Rechtsanwälte**
**Arabellastrasse 4**
**81925 München (DE)**

(54) **LIQUID CRYSTAL COMPOUNDS HAVING NEGATIVE PERMITTIVITY, LIQUID CRYSTAL COMPOSITIONS, AND LIQUID CRYSTAL DISPLAY DEVICE**

(57) An object of the present invention is to provide liquid crystalline compounds and liquid crystalline compositions suitable for liquid crystalline materials such as IPS mode in which negative dielectric anisotropy being required.

A liquid crystalline compound expressed by the general formula (1)

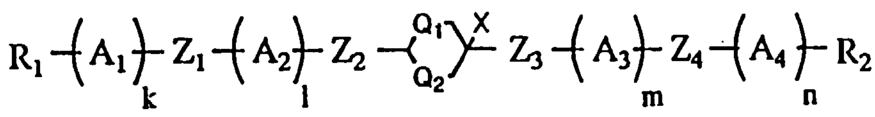

(1)

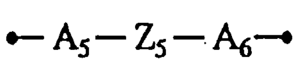

(1-1)

whrein, $R_1$ and $R_2$ being alkyl group having from 1 to 10 C atom(s) etc., $Z_1$ to $Z_4$ being $-(CH_2)_2-$ etc., $A_1$ to $A_3$ being 1,4-cyclohexylene etc., $Q_1$ and $Q_2$ being $-CH_2-$ etc., X being optionally haloalkyl or $-CH_2-$, k, l, m and n being 0 or 1, provided that $l+k+m+n \leqq 3$ , each independently.

EP 0 930 288 A1

## Description

Field of the Invention

The present invention relates to a novel liquid crystalline compounds which exhibit physical properties preferable in liquid crystal compositions mainly for TN, STN and TFT as well as liquid crystalline compostitions having preferable physical properties obtained by using the said novel liquid crystalline compounds. In particular, it relates to a novel liquid crystalline compounds showing negative and high dielectric anisotropic value ($\Delta \varepsilon$) and good miscibility with other liquid. crystalline compounds, as well as a novel liquid crystalline compositions having good characteristics constituted by using the said liquid crystalline compounds.

Background Art

Liquid crystalline display elements utilizing optical anisotropy and dielectric anisotropy possessed by liquid crystalline materials are used widely in watches, electronic computers, word processors and television sets etc., demands of which have been increased year by year. Liquid crystal phase is positioned between solid phase and liquid phase, and classified roughly into nematic phase, smectic phase and corestric phase. Among them, display elements utilizing nematic phase are most widely used.

On the other hand, there are considered many modes as display methods, and presently three types, that is, twist nematic (TN) type, supertwist nematic (STN) type and thin film transistor (TFT) type, become main currents. Although properties of liquid crystalline compounds required for these various liquid crystalline display elements are different variously according to their uses, all of liquid crystalline materials are required to be stable against external environmental factors such as moisture, air, heat and light etc., in order to show liquid crystalline phase within a wide temperature range as possible in nature centering around the room temperature, as well as to have. low viscosity and low driving voltage. However, there has not been found any single liquid crystalline material satisfying these requirements concurrently.

In the existing circumstances, liquid crystalline compositions are prepared and used for display elements by mixing several or several tens liquid crystalline compounds and optionally several non-liquid crytalline compounds if required in order to adjust suitable physical properties such as dielectric anisotropic value ($\Delta \varepsilon$), refractive anisotropic value ($\Delta$ n), viscosity ($\eta$) and elastic constant ratio K33/K11 (K33: bend elastic constant, K11: splay elastic constant) etc. required for respective display elements. Thus, it is recently required that miscibility with other liquid crystalline compounds, particularly solubility at low temperatures, should be superior owing to requirements for uses under various environments.

Thus, active matrix modes, particularly thin film transistor (TFT) mode, are recently used extensively as display modes for television sets or view finders etc. due to their display properties such as contrast, display volume and response time etc. Furthermore, STN mode which has more simple structure as to display elements and which can be prepared more inexpensively than those of active matrix mode while having large display volume is much used as displays for personal computers etc.

Recent tendency of developments in these fields has been concentrated in miniaturization, portablization, decrease of used electric power and mouse response, as well as high-speed response with the object of animation display, for example, television sets or note-type personal computers, which are characterized by small-size, light weight and portablization. So that, liquid crystalline compounds and liquid crystalline compositions having low driving voltage, that is, low threshold voltage and also low viscosity have been required. Furthermore, novel display modes have been recently invented and published in Official Patent Gazettes and academic societies owing to requirements for enlargement in viewing angles of liquid crystalline display elements.

Now, they will be illustrated in more detail. Threshold voltage (Vth) is known to be expressed by the following equation (H. J. Deuling et al., Mol. Cryst. Liq. Cryst., 27 (1975) 81):

$$Vth = \pi (K/ \varepsilon 0 \Delta \varepsilon)^{1/2}$$

In the above equation, K is elastic constant and $\varepsilon$ 0 is permittivity under vacuum. As clear easily from the equation, in order to lower threshold voltage, there may be considered two methods, that is to increase dielectric anisotropy ($\Delta \varepsilon$) or to decrease elastic constant. However, there have been studied about mutual relationship between elastic constnat and structure of compound, but there are still much unclear parts, so that it is impossible to control elastic constant at will. Generally, in the existing circumstances, the requirements are coped with by using liquid crystalline materials having high dielectric anisotopy ($\Delta \varepsilon$).

It is known that viscosity is a factor for controlling response speed against electric field of liquid crystal molecules oriented in liquid crystal panel (Phys. Lett., 39A, 69 (1972)). That is, in order to prepare liquid crystalline compositions showing high-speed response, it is preferable to use liquid crytalline compounds having very low viscosity in a large quantities to constitute liquid crystalline compositions.

Furthermore, in order to make their use within a wide temparature range possible, liquid crystalline compositions are required to show nematic phase particularly at low temperatures. It is needless to say that liquid crystalline compositions having no precipitation of crystals nor exhibition of smectic phase. As described above, in order that liquid crystalline compositions express some characteristics required for respective display elements, several or several thirties liquid crystalline compounds or non-liquid crystalline compounds are mixed and formulated. Thus, high solubility of used liquid crystalline comopunds with other liquid crystalline compounds at low temperatures is very important.

Furthermore, since liquid crystalline display elements are used under severe conditions such as high temperature, high humidity and outdoor, liquid crystalline compounds used in liquid crystalline compositions should have sufficiently high chemical stability.

Also, important matter for respective items of physical properties is temperature-dependency. Liquid crystalline display elements used actually should be required to maintain these display qualities within an extremely wide temperature range. To accomplish the object, it is necessary to use liquid crystalline compounds and liquid crystalline compositions having no or very low temperature-dependecy of respective physical properties.

Recently, In-Plane Switching (IPS) mode, Vertical Alignment (VA mode) and Optically Compensated Bend (OCB) mode which utilize TFT mode have been published in academic societies and attracted attentions as novel driving modes to overcome disadvantages of liquid crystalline displays, that is, narrowness of viewing angle (Asia Display '95 Hamamatsu, SID97 DIGEST, 845 (1997), the twenty first liquid crystalline forum).

It will be illustrated in more detail as follows. Characteristics of the above-mentioned In-Plane Switching (IPS) mode are that a comb-tooth electrode is mounted only on one side of substrate in IPS driving panel in contrast to the conventional type liquid crystalline panel wherein comb-tooth electrodes being mounted on above and below substrate, and that orientation of liquid crystalline molecules in minor axial direction in panel is preferably parallel to substrate. As advantages of IPS driving mode, the followings may be mentioned in addition to enlargement of view angle. That is,

1) cells can be made thinner than the conventional ones, since electrode being present only on one side of substrate,
2) decrease in production cost may be available, and
3) distance between electrodes is kept constant, etc.

On the other hand, as characteristics of liquid crystalline compounds required for IPS mode and VA mode which utilize the above-mentioned TFT method, there may be mentioned as follows:

1) to show high voltage holding ratio by using TFT mode, and to have low temperature-dependency, as well as
2) to have high dielectric anisotropy (negative dielectric anisotropy) in minor axial direction since major axial direction of liquid crystalline molecules being oriented preferably parallel to substrate.

Then, as known compounds having high and negative dielectric anisotropy, the following compounds have been disclosed in Official Patent Gazette.

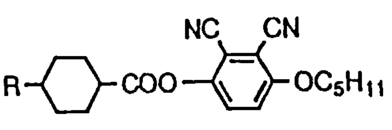

Toku-Ko-Sho 61-26899 (10)

(R denotes alkyl group in the above-mentioned structural formula).

Compounds (10) (Toku-Ko-Sho 61-26899) were reported to have partial structure of 2,3-dicyano-1,4-phenylene group in compounds and to show negative and high dielectric anisotropy. However, although compounds (10) show very high dielectric anisotropic values owing to an effect of 1,4-phenylene group substituted with two cyano groups, viscosity is eminently high so that increase of viscosity cannot be avoided when used in compositions.

Furthermore, as generally known, temperature-dependency of voltage holding ratio is very much, so that they cannot be used as liquid crystalline materials in TFT mode. Also, miscibility with other known liquid crystalline compounds is not preferable so that there are some problems about compositions such as precipitation of crystals or exhibition of smectic phase etc. when being allowed at low temperatures.

Thus, in the existing circumstances, there has not been found any liquid crystalline compound suitable for IPS mode or VA mode etc., that is, any compound showing negative and high dielectric anisotropy, high voltage holding rate,

as well as having low temperature-dependency and superior miscibility with known liquid crystalline compounds, thus a novel liquid crystalline compound and a liquid crystalline composition having improved characteristics which solve these problems are desired.

The present inventors have made extensive research in order to solve the above-mentioned problems and have found that liquid crystalline compounds expressed by the general formula (1) having partial structure of six-membered ring structure substituted with 1,4-cyclohexylene group or oxygen atom or sulfur atom at position-2 or position-6 and also substituted with halogen atom, haloalkyl group or haloalkoxy group at position-4 have negative and high dielectric anisotropy, high voltage holding ratio as well as having low temerature-dependency and superior miscibility with known liquid crystalline compounds, in another words, the said liquid crystalline compounds are suitable as liquid crystalline materials for IPS mode or VA mode, to complete the present invention.

Namely, an object of the present invention is to provide liquid crystalline compounds suitable for liquid crystalline materials mainly for IPS mode or VA mode showing negative and high dielectric anisotropy.

Disclosure of the Invention .

In order to attain the above-mentioned objects, inventions to be claimed in the present application are as follows. That is, the first item of the present invention relates to a liquid crystalline compound expressed by the general formula (1)

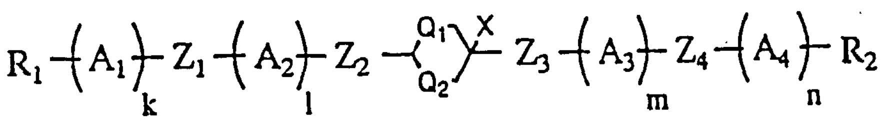 (1)

$$\bullet\!-A_5-Z_5-A_6-\!\bullet \qquad (1\text{-}1)$$

wherein, $R_1$ and $R_2$ are each independently denote alkyl group having from 1 to 10 carbon atom(s) in which one $CH_2$ group or more than one non-adjacent $CH_2$ groups may be substituted with (an) oxygen atom(s), (a) sulfur atom(s), $-SiH_2-$, -CH=CH- or -C≡C- and furthermore (an) optional hydrogen atom(s) in the group may be substituted with (a) halogen atom(s);

$Z_1$, $Z_2$, $Z_3$ and $Z_4$ each independently denote $-CH_2CH_2-$, -CH=CH-, -C≡C-, $-CH_2O-$, $-OCH_2-$, $-CF_2O-$, $-OCF_2-$, -COO-, -OCO-, $-(CH_2)_4-$or covalent bond;

$A_1$ and $A_3$ each independently denote 1,4-cyclohexylene group, 1,3-dioxane-2,5-diyl group, 1,4-phenylene group optionally substituted with one or more halogen atom(s), pyridine-2,5-diyl group, piperidine-1,4-diyl group, 1,4-bicyclo[2.2.2]octylene group, pyrimidine-2,5-diyl group, naphthalene-2,6-diyl group, decahydronaphthalene-2,6-diyl group or 1,2,3,4-tetrahydronaphthalene-2,6-diyl group;

$A_2$ and $A_4$ each independently denote 1,4-cyclohexylene group, 1,3-dioxane-2,5-diyl group, 1,4-phenylene group optionally substituted with one or more halogen atom(s), pyridine-2,5-diyl group, piperidine-1,4-diyl group, 1,4-bicyclo[2.2.2]octylene group, pyrimidine-2,5-diyl group, naphthalene-2,6-diyl group, decahydronaphthalene-2,6-diyl group, 1, 2, 3, 4-tetrahydronaphthalene-2,6-diyl group or a group expressed by the general formula (1-1);

$A_5$ and $A_6$ in the group each independently denote 1,4-cyclohexylene group, 1,3-dioxane-2,5-diyl group, 1,4-phenylene group optionally substituted with one or more halogen atom(s), pyridine-2,5-diyl group, piperidine-1,4-diyl group, 1,4-bicyclo [2.2.2] octylene group, pyrimidine-2,5-diyl group, naphthalene-2,6-diyl group, decahydronaphthalene-2,6-diyl group or 1, 2, 3, 4-tetrahydronaphthalene-2,6-diyl group;

$Z_5$ denotes $-CH_2CH_2-$, -CH=CH-, -C≡C-, $-CH_2O-$, $-OCH_2-$, $-CF_2O-$, $-OCF_2-$, -COO-, -OCO-, - $(CH_2)$ 4- or single bond;

$Q_1$ and $Q_2$ each independently denote $CH_2$ group, (an) oxygen atom (s) or (a) sulfur atom (s);

X denotes halogen atom, cyano group, haloalkyl group having from 1 to 10 carbon atom(s) or haloalkoxy group if either or both of $Q_1$ and $Q_2$ being (an) oxygen atom(s) or (a) sulfur atom(s) and denotes haloalkyl groups or haloalkoxy group having from 1 to 10 carbon atom(s) if both of $Q_1$ and $Q_2$ being $CH_2$ groups;

k, l, m and n each independently 0 or 1, provided that $l+k+m+n \leqq 3$ ,

provided that each elements constituting the compound may be substituted with their isotopic elements.

The second item of the invention relates to a liquid crystalline compound according to item 1 of the invention, wherein both of $Q_1$ and $Q_2$ being $CH_2$ groups in the general formula (1).

The third item of the invention relates to a liquid crystalline compound according to item 2 of the invention, wherein both of $Q_1$ and $Q_2$ being $CH_2$ groups and X being $CF_3$ in the general formula (1).

The fourth item of the invention relates to a liquid crystalline compound according to item 2 of the invention, wherein both of $Q_1$ and $Q_2$ being $CH_2$ groups and X being $OCF_3$ group in the general formula (1).

The fifth item of the invention relates to a liquid crystalline compound according to item 1 of the invention, wherein both of $Q_1$ and $Q_2$ being oxygen atoms in the general formula (1).

The sixth item of the invention relates to a liquid crystalline compound according to item 5 of the invention, wherein both of $Q_1$ and $Q_2$ being oxygen atoms and X being a halogen atom in the general formula (1).

The seventh item of the invention relates to a liquid crystalline compound according to item 1 of the invention, wherein both of $Q_1$ and $Q_2$ being sulfur atoms in the general formula (1).

The eighth item of the invention relates to a liquid crystalline compound according to item 1 of the invention, wherein both of $Q_1$ being $CH_2$ groups and $Q_2$ being an oxygen atom in the general formula (1).

The ninth item of the invention relates to a liquid crystalline compound according to item 1 of the invention, wherein both of $Q_1$ being $CH_2$ groups and $Q_2$ being a sulfur atom in the general formula (1).

The tenth item of the invention relates to a liquid crystalline composition characterized in that one or more compound(s) expressed by the general formula (1) is (are) contained.

The eleventh item of the invention relates to a liquid crystalline composition characterized in that one or more compound(s) according to items 1 to 9 of the invention is (are) contained as the first component, and one or more compound(s) selected from the group consisting of the general formulae (2), (3) and (4):

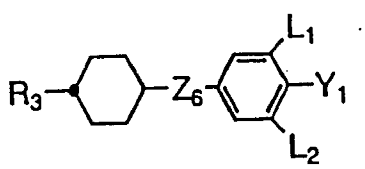

(2)

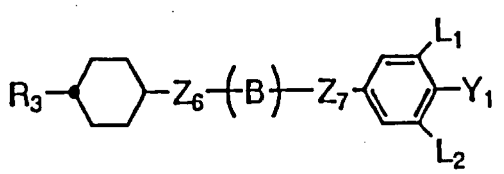

(3)

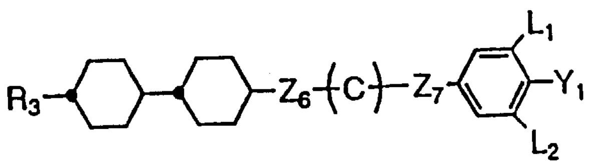

(4)

wherein, $R_3$ denotes an alkyl group having from 1 to 10 carbon atom(s) in which (an) optional non-adjacent methylene group(s) in the alkyl group may be substituted with (an) oxygen atom(s) or -CH=CH- and also (an) optional hydrogen atom(s) in the group may be substituted with (a) fluorine atom(s);
$Y_1$ denotes fluorine atom, chlorine atom, $OCF_3$, $OCF_2H$, $CF_3$, $CF_2H$, $CFH_2$, $OCF_2CF_2H$ or $OCF_2CFHCF_3$;
$L_1$ and $L_2$ each independently hydrogen atom or fluorine atom;
$Z_6$ and $Z_7$ each independently 1,2-ethylene group, 1,4-butylene group, -COO-, $-CF_2O-$, $-OCF_2-$, -CH=CH- or covalent bond;
ring B denotes trans-1,4-cyclohexylene, 1,3-dioxane-2,5-diyl or 1,4-phenylene in which (a) hydrogen atom(s) may be substituted with (a) fluorine atom(s);
ring C denotes trans-1,4-cyclohexylene or 1,4-phenylene in which (a) hydrogen atom(s) may be substituted with (a) fluorine atom(s); and
each atoms used in the formulae can include their isotopes, is(are) contained as the second component.

The twelfth item of the invention relates to a liquid crystalline composition characterized in that one or more

compound(s) according to items 1 to 9 of the invention is (are) contained as the first component, and one or more compound(s) selected from the group consisting of the general formulae (5) and (6):

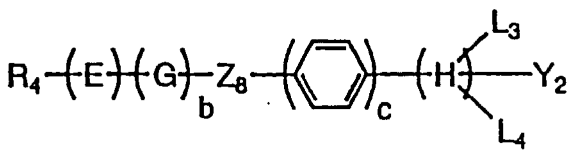

(5)

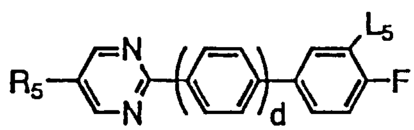

(6)

wherein, $R_4$ and $R_5$ each independently denote alkyl group having from 1 to 10 carbon atom(s) in which (an) optional non-adjacent methylene group(s) in the alkyl group may be substituted with an oxygen atom(s) or -CH=CH- and also (an) optional hydrogen atom(s) in the group may be substituted with (a) fluorine atom(s);
$Y_2$ denotes -CN group or -C≡C-CN;
ring E denotes trans-1,4-cyclohexylene, 1,4-phenylene, 1,3-dioxane-2,5-diyl or pyrimidine-2,5-diyl;
ring G denotes trans-1,4-cyclohexylene, 1,4-phenylene in which (a) hydrogen atom(s) may be substituted with (a) fluorine atom(s) or pyrimidine-2,5-diyl;
ring H denotes trans-1,4-cyclohexylene or 1,4-phenylene;
$Z_8$ denotes 1,2-ethylene group, -COO- or covalent bond;
$L_3$, $L_4$ and $L_5$ each independently donote hydrogen atom or fluorine atom;
b, c and d each independently denote 0 or 1; and
each atoms used in the formulae can include their isotopes, is(are) contained as the second component.

The thirteenth item of the invention relates to liquid crystalline composition characterized in that one or more compound(s) according to items 1 to 9 of the invention is (are) contained as the first component, and one or more compound(s) selected from the group consisting of the general formulae (7), (8) and (9):

$$R_6(I)Z_9(J)Z_{10}-R_7 \quad (7)$$

$$R_6(I)Z_9(J)Z_{10}(K)R_7 \quad (8)$$

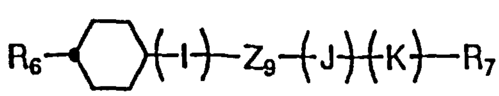

(9)

wherein, $R_6$ and $R_7$ each independently denote alkyl group having from 1 to 10 carbon atom(s) in which (an) optional non-adjacent methylene group(s) in the alkyl group may be substituted with (an) oxygen atom(s) or -CH=CH- and also (an) optional hydrogen atom(s) in the group may be substituted with (a) fluorine atom(s);
I, J and K each independently denote trans-1,4-cyclohexylene, pyrimidine-2,5-diyl or 1,4-phenylene in which (a) hydrogen atom(s) may be substituted with (a) fluorine atom(s);
$Z_4$ and $Z_5$ each independently denote -C≡C-, -COO-, $-CH_2CH_2-$, -CH=CH- or covalent bond; and

each atoms used in the formulae can include their isotopes, is(are) contained as the second component.

The fourteenth item of the invention relates to a liquid crystalline composition characterized in that one compound according to items 1 to 9 of the invention is contained as the first component, and one or more compound(s) selected from the group consisting of the general formulae (10), (11) and (12):

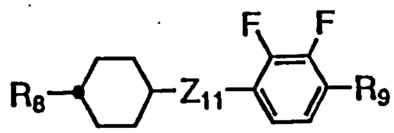

(10)

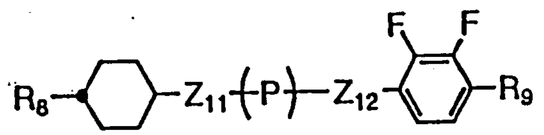

(11)

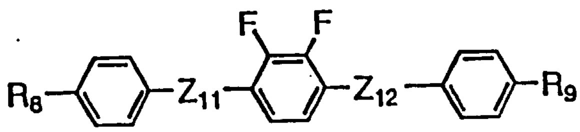

(12)

wherein, $R_8$ and $R_7$ each independently denote alkyl group having from 1 to 10 carbon atom(s) in which (an) optional non-adjacent methylene group(s) in the alkyl group may be substituted with (an) oxygen atom(s) or -CH=CH- and also (an) optional hydrogen atom(s) in the group may be substituted with (a) fluorine atom(s);
P each independently denote trans-1,4-cyclohexylene or 1,4-phenylene; $Z_{11}$ and $Z_{12}$ each independently denote -$CH_2CH_2$-, -$CH_2O$- or covalent bond; and
each atoms used in the formulae can include their isotopes, is(are) contained as the second component.

The fifteenth item of the invention relates to a liquid crystalline composition characterized in that one or more compound(s) according to items 1 to 9 of the invention is(are) contained as the first component, one or more compound(s) selected from the group consisting of the general formulae (7), (8) and (9) is (are) contained as the second component, as well as one or more compound(s) selected from the group consisting of the general formulae (10), (11) and (12) is (are) contained as the third component.

The sixteenth item of the invention relates to a liquid crystalline composition characterized in that one or more compound(s) according to items 1 to 9 of the invention is (are) contained as the first component, one or more compound(s) selected from the group consisting of the general formulae (2), (3) and (4) is (are) contained as the second component, as well as one or more compound(s) selected from the group consisting of the general formulae (7), (8) and (9) is(are) contained as the third component.

The seventeenth item of the invention relates to a liquid crystalline composition characterized in that one or more compound(s) according to items 1 to 9 of the invention is (are) contained as the first component, one or more compound(s) selected from the group consisting of the general formulae (5) and (6) is (are) contained as the second component, as well as one or more compound(s) selected from the group consisting of the general formulae (7), (8) and (9) is (are) contained as the third component.

The eighteenth item of the invention relates to a liquid crystalline composition characterized in that one or more compound(s) according to items 1 to 9 of the invention being contained as the first component, one or more compound(s) selected from the group consisting of the general formulae (2), (3) and (4) is(are) contained as the second component, one or more compound(s) selected from the group consisting of the general formulae (5) and (6) is(are) contained as the third component, as well as one or more compound(s) selected from the group consisting of the general formulae (7), (8) and (9) is(are) contained as the fourth component.

The nineteenth item of the invention relates to a liquid crystalline composition characterized in that one or more

optically active compound(s) is (are) contained in addition to any of the liquid crystalline composition according to items 10 to 18 of the invention.

The twentieth item of the invention relates to a liquid crystalline display element constituted by using the liquid crystalline composition according to any of items 10 to 19 of the invention.

## Best Mode for Carrying out the Invention

Constitution and effect of the present invention are described in detail as follows.

Compounds expressed by the general formula (1) according to the invention have high dielectric anisotropic values in minor axial direction, that is, negative values, and have high voltage holding ratio, low temperature-dependencies as well as superior characteristics such as no precipitation of crystals even at low temperatures nor exhibition of smectic phase, stable maintainance of nematic phase, and superior miscibility with other liquid crystalline compounds.

## Industrial Applicability

Furthermore, those wherein $CH_2$ being selected as $Q_1$ and $Q_2$, haloalkyl group or haloalkoxy group being selected as X in the general formula (1) are relatively low viscous but show negative and high dielectric anisotropies as well as high voltage holding ratio, so that they show preferable characteristics as liquid crystalline materials for display elements which require high reliability, as one applicability of the compounds according to the invention. On the other hand, those whrein oxygen atom or sulfur atom being selected as $Q_1$ or $Q_2$ and halogen atom, haloalkyl group or haloalkoxy group being selected as X show extremely high and negative dielectric anisotropies, and they are very useful as liquid crystalline materials for display elements such as IPS mode or VA mode which require negative dielectric anisotropies.

Above-mentioned items 13, 14 and 15 according to the invention are inventions relating to the fact that $\Delta\varepsilon$ of liquid crystalline compositions being negative. Liquid crystalline compositions wherein $\Delta\varepsilon$ being negative can be drived in various driving modes similar to liquid crystalline compositions wherein $\Delta\varepsilon$ being positive. Furtheremore, items 11, 12, 16, 17 and 18 according to the invention are inventions wherein compounds having positive $\Delta\varepsilon$ and compounds having netagive $\Delta\varepsilon$ are mixed. Voltage-transmittance curve (V-T curve) of liquid crystalline compositions can be controlled by mixing compounds having positive $\Delta\varepsilon$ and compounds having netagive $\Delta\varepsilon$. Thus, liquid crystalline compositions according to the invention can be utilized in various driving modes.

It becomes possible to provide liquid crystalline compositions suitable for various liquid crystalline display elements mainly as IPS mode or VA mode by using compounds according to the invention.

As clear from Comparative Examples in Embodiments, negative and high dielectric anisotropic values of compounds according to the invention are derived from effective action of dipole moment in minor axial direction of molecules, wherein the said dipole moment being produced by substituent X (halogen atom, haloalkyl group, haloalkoxy group) at axial positions in general formula (1). Furthermore, in the case of compounds substituted with oxygen atom or sulfur atom as $Q_1$ or $Q_2$, dipole moment produced by non-covalent electron pair of those hetero atoms contributes similarly in short axial direction of molecules, which produces negative and high dielectric anisotropic values owing to those synergic effects.

It is needless to say that all of the compounds according to the invention show preferable physical properties, but liquid crystalline compositions suitable for respective objects can be prepared by using compounds wherein $R_1$, $R_2$, $A_1$, $A_2$, $A_3$, $A_4$, $A_5$, $A_6$, $Z_1$, $Z_2$, $Z_3$, $Z_4$, $Z_5$, $Q_1$, $Q_2$, k, l, m and n being suitably selected.

That is, when used in liquid crystalline compositions wherein a liquid crystalline temperature range being a high temperature side, requirements can be satisfied by selecting four-membered ring compounds in which $k+l+m+n=3$ , and also selecting halogen atom; CN group; haloalkyl group or haloalkoxy group with short carbon chains such as $CF_3$ group, $CF_2H$ group, $CH_2F$ group, $OCF_3$ group, $OCHF_2$ group and $OCH_2F$ group etc. as substituent X. On the other hand, in the case that a liquid crystalline temperature range may not be particularly a high temperature side, two-membered ring or three-membered ring compounds may be used.

Furthermore, in the case that particularly high voltage holding ratio being necessary, requirements can be satisfied by selecting alkyl group or fluoroalkyl group as $R_1$ and $R_2$, selecting $CH_2$ group as $Q_1$ and $Q_2$, selecting other than -CH=CH-, -C≡C-, -COO- and -OCO- as $Z_1$, $Z_2$, $Z_3$, $Z_4$ and $Z_5$, and also selecting other than CN group as substituent X.

Furthermore, in order to obtain extremely high and negative dielectric anisotropic values, requirements can be satisfied by selecting oxygen atom or sulfur atom as $Q_1$ and $Q_2$, and also selecting CN group, $CF_3$ group or $CF_2H$ group as substituent X.

Refractive anisotropic values can be optionally adjusted by selecting $R_1$, $R_2$, $A_1$, $A_2$, $A_3$, $A_4$, $A_5$, $A_6$, $Z_1$, $Z_2$, $Z_3$, $Z_4$, $Z_5$, $Q_1$, $Q_2$, k, l, m and n in the generel formula (1) suitably. That is, in the case that high refractive anisotropic values being necessary, compounds wherein much 1,4-phenylene group being contained and wherein $Z_1$, $Z_2$, $Z_3$, $Z_4$ and $Z_5$ being covalent bonds may be selected, and in the case that low refractive anisotropic values being necessary, compounds wherein much trans-1,4-cyclohexylene group being contained may be selected.

In compounds expressed by the general formula (1) according to the invention, $R_1$ and $R_2$ are each independently denote alkyl groups having from 1 to 10 carbon atoms in which one $CH_2$ group or more than one non-adjacent $CH_2$ groups may be substituted with (an) oxygen atom(s), (a) sulfur atom (s), $-SiH_2-$, -CH=CH- or -C≡C- and furthermore (an) optional hydrogen atom(s) may be substituted with (a) halogen atom(s).

Concretely, above-mentioned $R_1$ and $R_2$ are alkyl group, alkoxy group, alkoxyalkyl group, alkenyl group, alkynyl group, alkenyloxy group, alkynyloxy group, halogen-substuted alkyl group, halogen-substituted alkoxy group, halogen-substituted alkoxyalkyl group, halogen-substituted alkenyl group and halogen-substituted alkynyl group.

More concretely, methyl group, ethyl group, propyl group, butyl group, pentyl group, hexyl group, heptyl group, octyl group, nonyl group, decyl group, methoxy group, ethoxy group, propoxy group, butoxy group, pentoxy group, hexyloxy group, heptyloxy group, nonyloxy group, decyloxy group, methoxymethyl group, ethoxymethyl group, propoxymethyl group, butoxymethyl group, methoxyethyl group, methoxypropyl group, methoxybutyl group, ethoxyethyl group, ethoxypropyl group, propoxyethyl group, propoxypropyl group, vinyl group, 1-propenyl group, 1-butenyl group, 1-pentenyl group, 3-butenyl group, 3-pentenyl group, allyloxy group, ethynyl group, 1-propynyl group, 1-butynyl group, 1-pentynyl group, 3-butynyl group, 3-pentynyl group, trifluoromethyl group, difluoromethyl group, difluorochloromethyl group, 2,2,2-trifluoroethyl group, 2-fluoroethyl group, 3-fluoropropyl group, 4-fluorobutyl group, 5-fluoropentyl group, 3-chloropropyl group, trifluoromethoxy group, difluoromethoxy group, difluorochloromethoxy group, pentafluoroethoxy group, 1,1,2,2-tetrafluoroethoxy group, heptafluoropropoxy group, 1,1,2,3,3,3-hexafluoropropoxy group, trifluoromethoxymethyl group, 2-fluoroethenyl group, 2,2-difluoroethenyl group, 1,2,2-trifluoroethenyl group, 3-fluoro-1-butenyl group, 4-fluoro-1-butenyl group and 3,3,3-trifluoro-1-propynyl group are preferable.

Above-mentioned X denotes halogen atom, cyano group or haloalkyl group or haloalkoxy group having 1 to 10 carbon atoms. Concretely, fluorine atom, chlorine atom, bromine atom, trifluoromethyl group, difluoromethyl group, difluorochloromethyl group, 2,2,2-trifluoroethyl group, 2-fluoroethyl group, 3-fluoropropyl group, 4-fluorobutyl group, 5-fluoropentyl group, 3-chloropropyl group, trifluoromethoxy group, difluoromethoxy group, difluorochloromethoxy group, pentafluoroethoxy group, 1,1,2,2-tetrafluoroethoxy group, heptafluoropropoxy group and 1,1,2,3,3,3-hexafluoropropoxy group are preferable.

In the first item according to the invention, preferable embodiments expressed by the general formula (1) are the following compounds expressed by the general formulae (1-2) to (1-10).

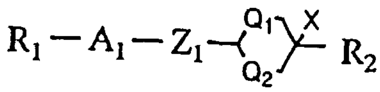
(1-2)

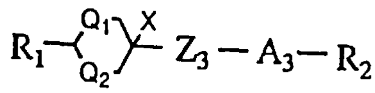
(1-3)

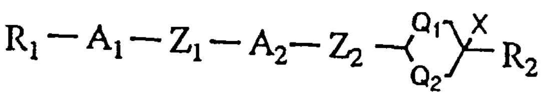
(1-4)

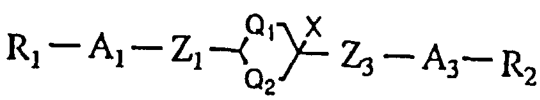
(1-5)

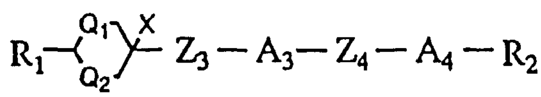
(1-6)

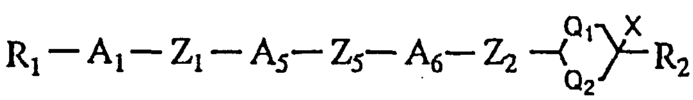
(1-7)

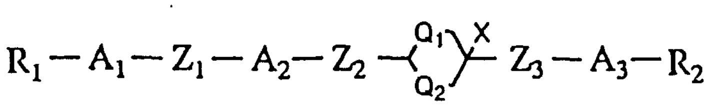
(1-8)

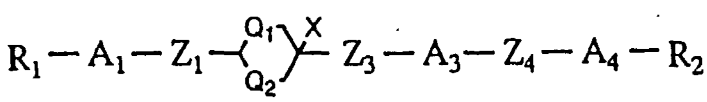
(1-9)

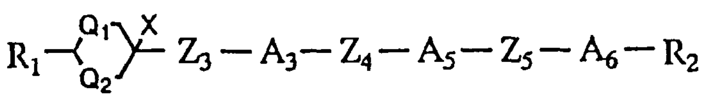
(1-10)

(in the above-mentioned general formulae, $R_1$, $R_2$, $A_1$, $A_2$, $A_3$, $A_4$, $A_5$, $A_6$, $Z_1$, $Z_2$, $Z_3$, $Z_4$, $Z_5$, $Q_1$, $Q_2$ and X denote the same meanings as above).

Furthermore, (a) to (e) shown below are preferable as saturated six-membered rings including $Q_1$ and $Q_2$, concretly.

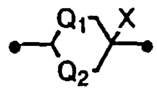

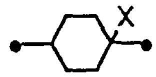 (a)

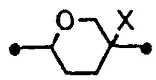 (b)

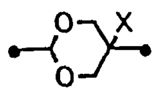 (c)

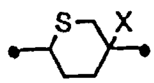 (d)

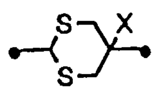 (e)

In the above-mentioned general formulae, two-ring type compounds expressed by the general formulae (1-2) and (1-3) can increase only dielectric anisotropic values of compositions negatively while maintaining viscosity when added as components of liquid crystalline compositions, and also three-ring type or four-ring type compounds expressed by the general formulae (1-4) to (1-10) can increase only dielectric anisotropic values of compositions negatively while not lowering clearing point when added as components of liquid crystalline compositions.

Liquid crystalline compositions according to the invention preferably contain 0.1 to 99.9% by weight, preferably 1 to 50% by weight, and more preferably 3 to 20% by weight, of one or more compound(s) expressed by the general formula (1), in order to obtain superior characteristics.

Liquid crystalline compositions provided by the invention are accomplished by adding the first component containing at least one compound (1) and mixing any compound selected optionally from the group of compounds expresssed by the general formulae (2) to (12) according to objects of the liquid crystalline compositions.

The following compounds may be preferably mentioned as compounds of the general formulae (2) to (4).

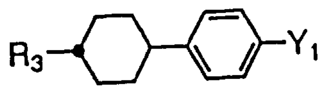
(2-1)

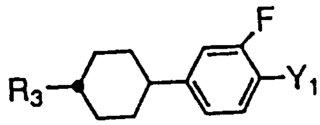
(2-2)

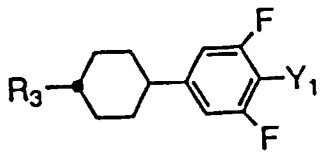
(2-3)

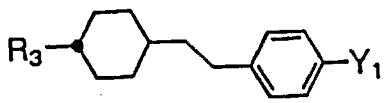
(2-4)

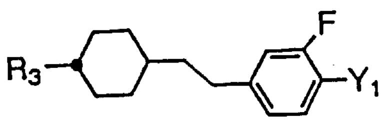
(2-5)

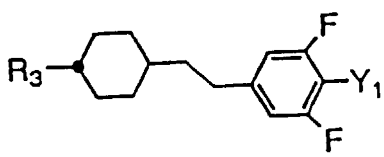
(2-6)

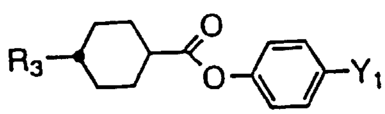
(2-7)

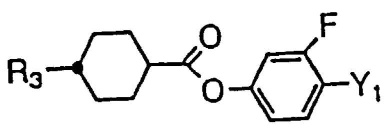
(2-8)

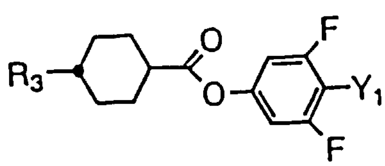
(2-9)

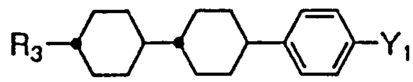
(3-1)

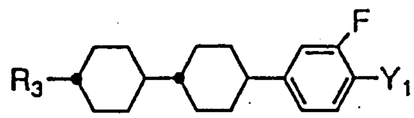
(3-2)

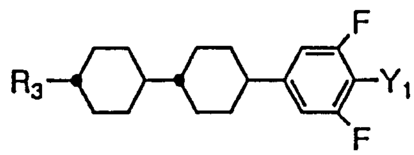
(3-3)

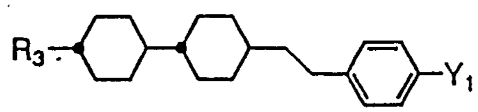
(3-4)

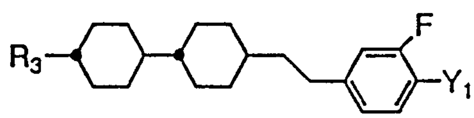
(3-5)

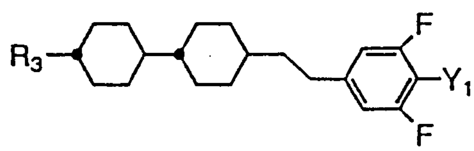
(3-6)

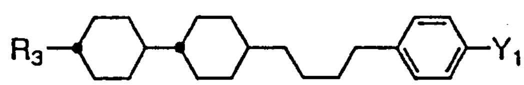
(3-7)

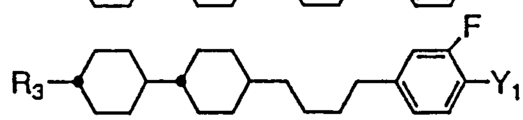
(3-8)

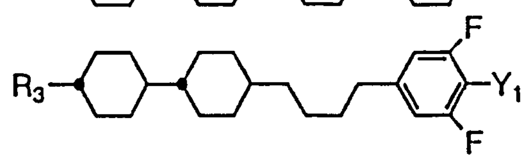
(3-9)

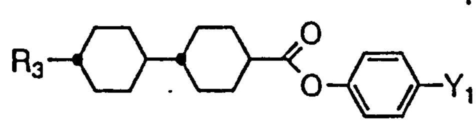
(3-10)

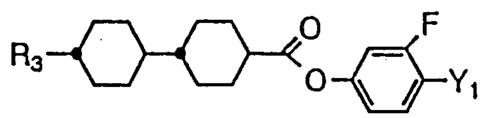
(3-11)

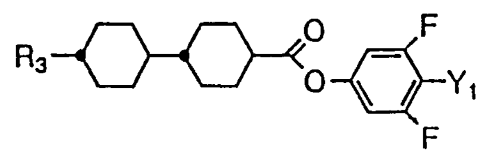
(3-12)

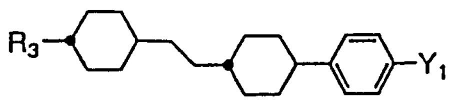

(3-13)

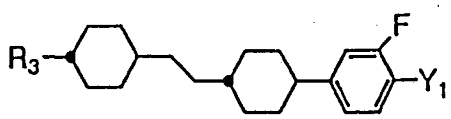

(3-14)

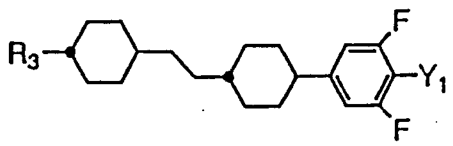

(3-15)

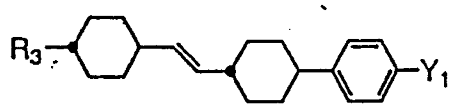

(3-16)

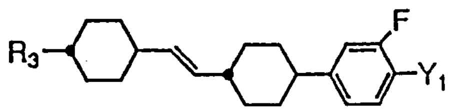

(3-17)

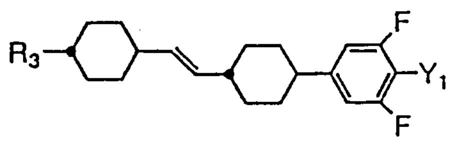

(3-18)

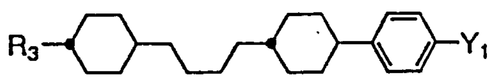

(3-19)

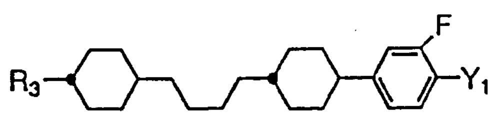

(3-20)

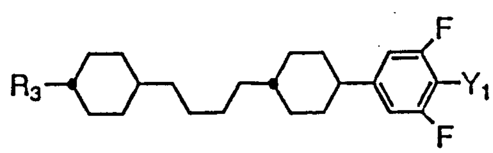

(3-21)

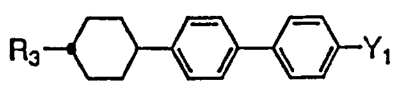

(3-22)

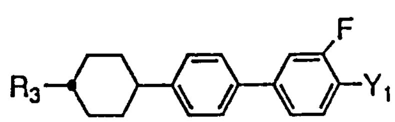

(3-23)

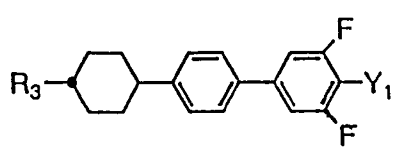

(3-24)

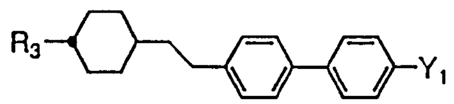

(3-25)

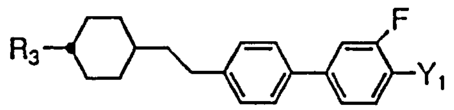

(3-26)

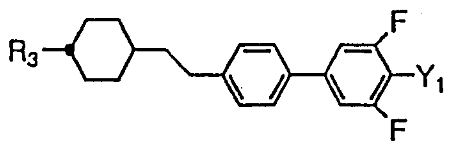

(3-27)

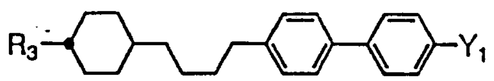

(3-28)

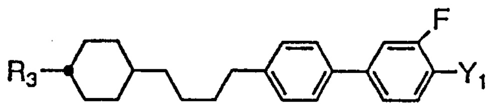

(3-29)

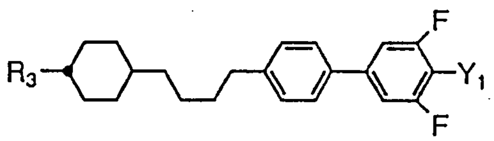

(3-30)

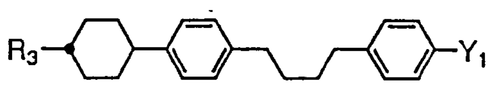

(3-31)

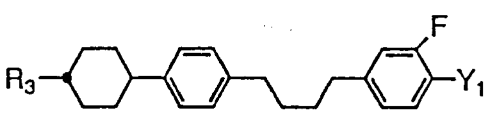

(3-32)

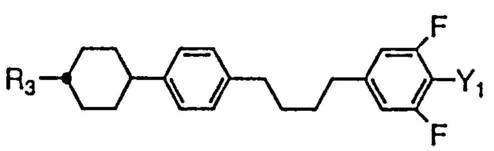

(3-33)

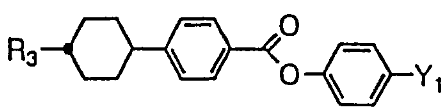

(3-34)

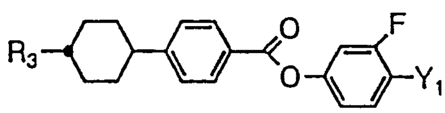

(3-35)

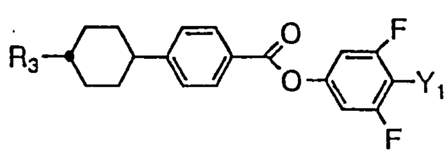

(3-36)

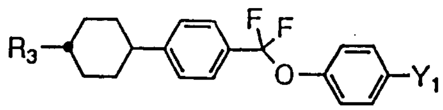
(3-37)

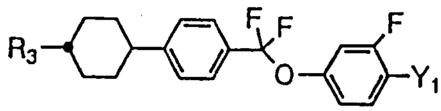
(3-38)

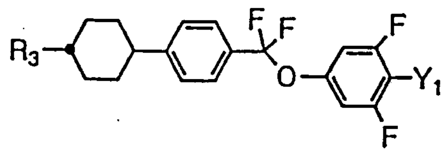
(3-39)

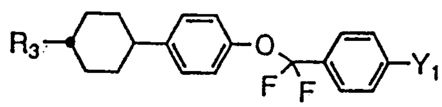
(3-40)

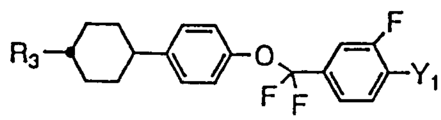
(3-41)

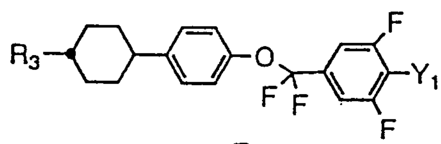
(3-42)

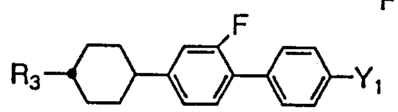
(3-43)

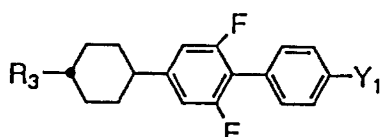
(3-44)

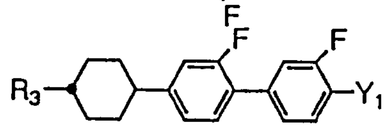
(3-45)

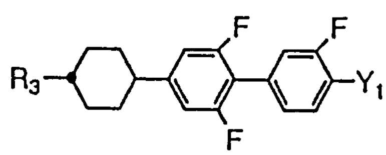
(3-46)

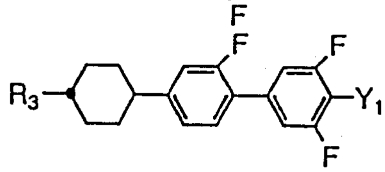
(3-47)

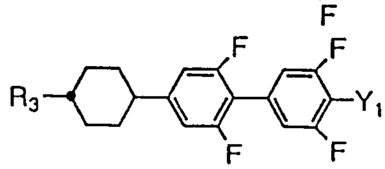
(3-48)

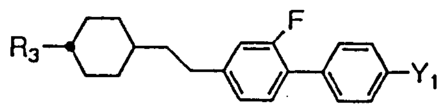

(3-49)

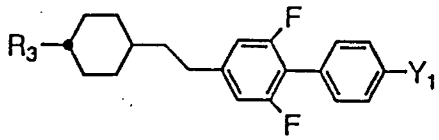

(3-50)

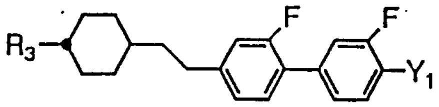

(3-51)

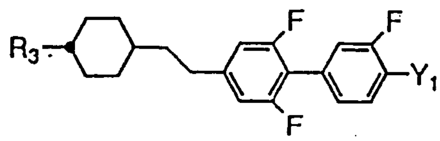

(3-52)

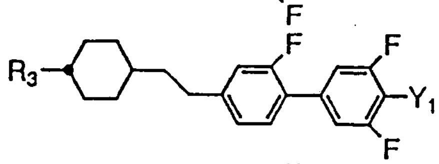

(3-53)

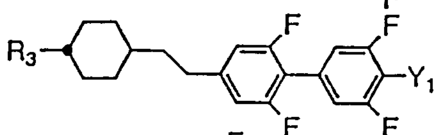

(3-54)

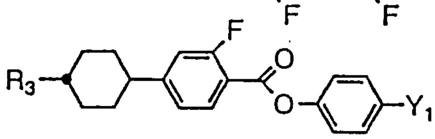

(3-55)

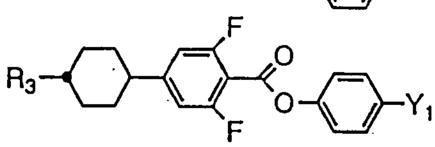

(3-56)

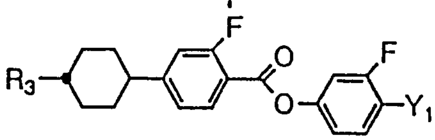

(3-57)

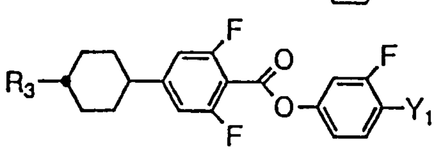

(3-58)

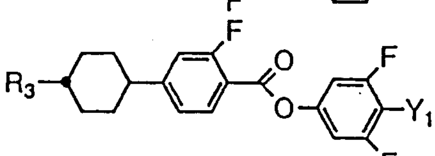

(3-59)

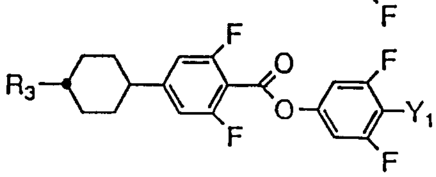

(3-60)

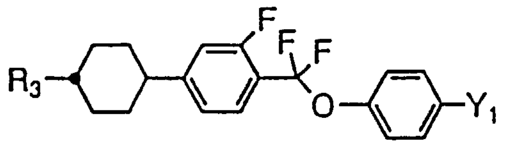

(3-61)

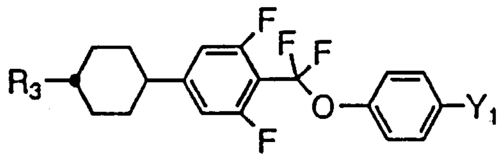

(3-62)

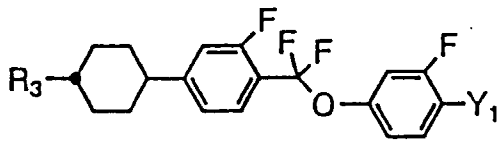

(3-63)

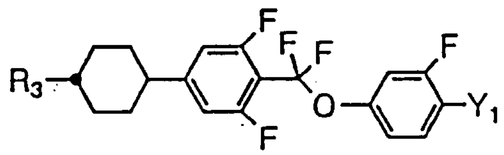

(3-64)

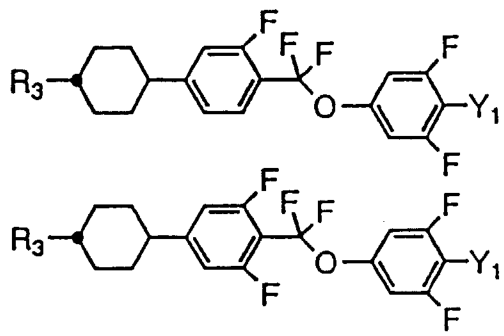

(3-65)

(3-66)

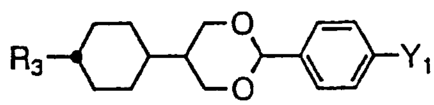

(3-67)

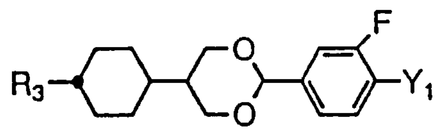

(3-68)

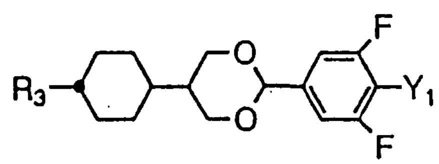

(3-69)

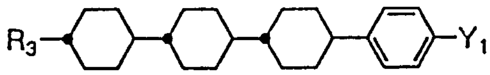
(4-1)

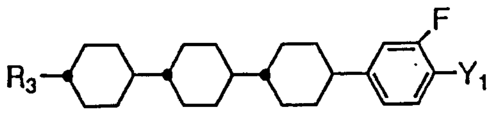
(4-2)

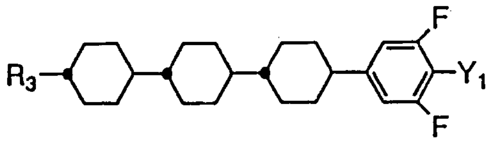
(4-3)

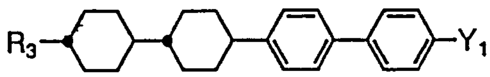
(4-4)

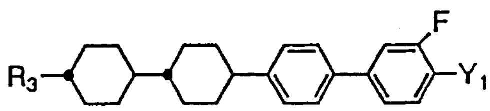
(4-5)

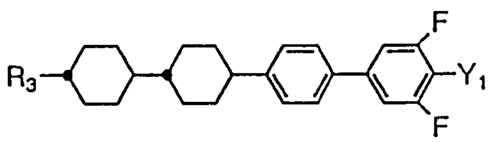
(4-6)

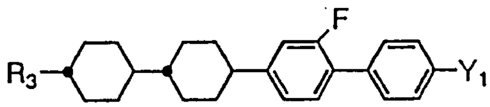
(4-7)

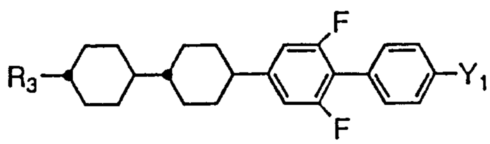
(4-8)

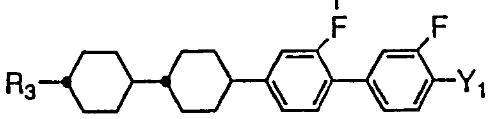
(4-9)

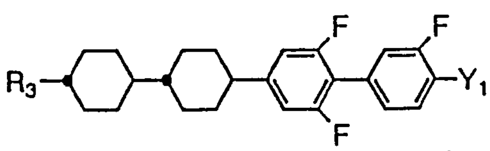
(4-10)

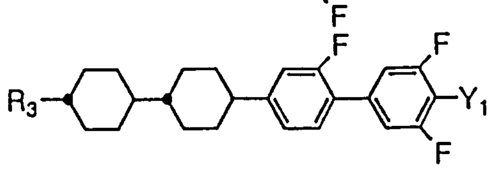
(4-11)

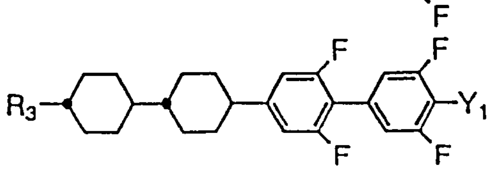
(4-12)

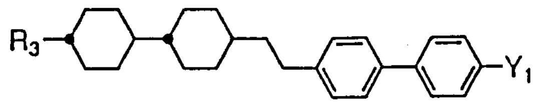

(4-13)

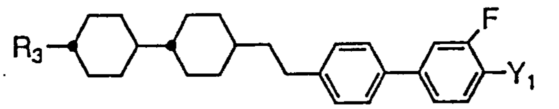

(4-14)

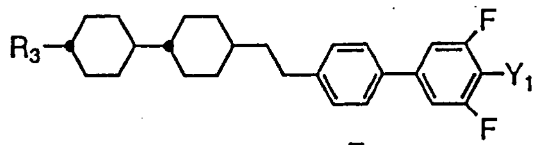

(4-15)

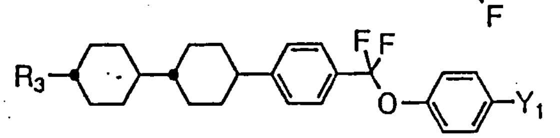

(4-16)

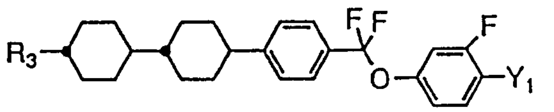

(4-17)

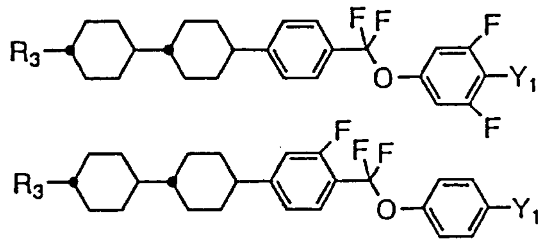

(4-18)

(4-19)

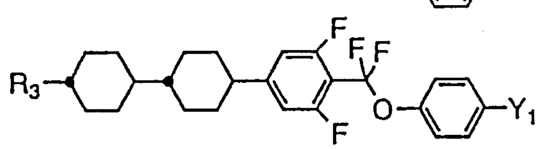

(4-20)

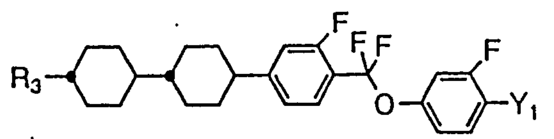

(4-21)

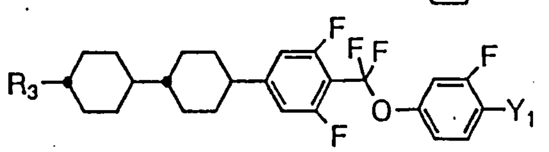

(4-22)

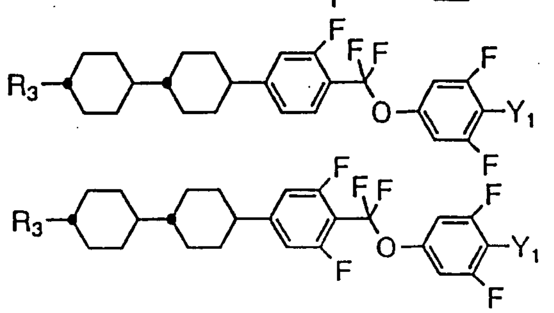

(4-23)

(4-24)

($R_3$ and $Y_1$ denote the same meanings as above.)

The compounds of the general formulae (2) to (4) are indispensable compounds when preparing liquid crystalline compositions for TFT (AM-LCD), in which dielectric anisotropic values being positive, thermal stability and chemical stability being superior and also high reliabilities such as high voltage holding ratio (or high specific resistant values)

being required.

The used amounts of the compounds of the general formulae (2) to (4) may be at will within a range of 1 to 99% by weight, preferably 10 to 97% by weight, more preferably 40 to 95% by weight, relative to the total weight of the liquid crystalline composition. Furthermore, compounds of (7) to (9) may be contained in such cases. In the cases that liquid crystalline compositions for STN display mode and TN display mode being prepared, the compounds of the general formulae (2) to (4) may be used. In such cases, the used amount of less than 50% by weight is preferable since effects to decrease threshold voltage of liquid crystalline compositions are less than compounds of general formulae (5) and (6).

As compounds of general formulae (5) and (6), the following compounds may be preferably mentioned.

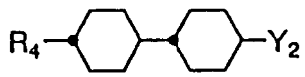
(5-1)

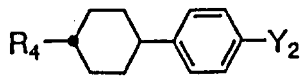
(5-2)

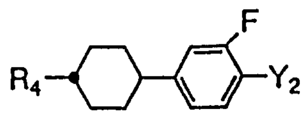
(5-3)

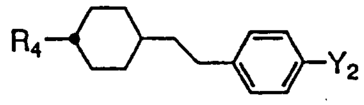
(5-4)

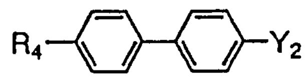
(5-5)

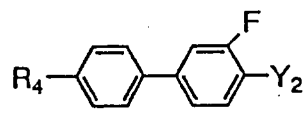
(5-6)

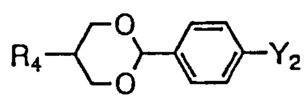
(5-7)

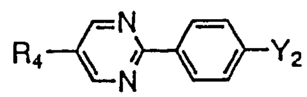
(5-8)

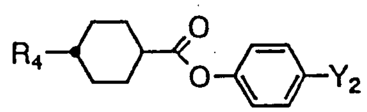
(5-9)

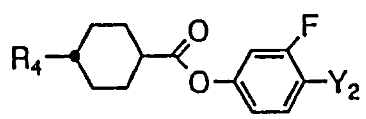
(5-10)

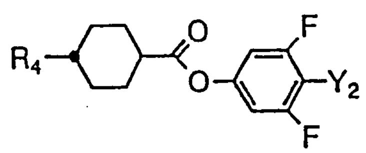
(5-11)

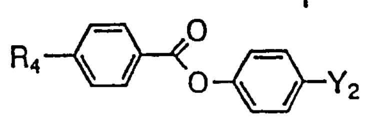
(5-12)

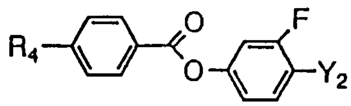 (5-13)

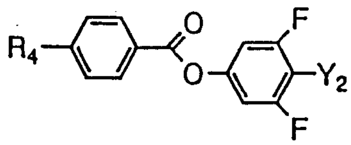 (5-14)

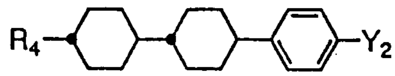 (5-15)

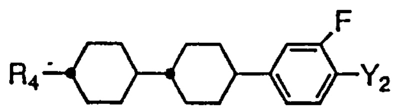 (5-16)

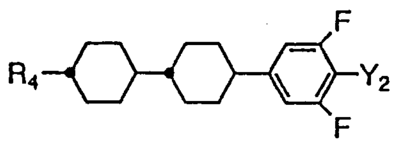 (5-17)

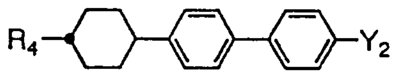 (5-18)

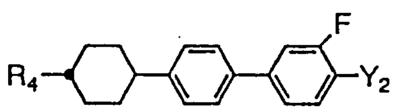 (5-19)

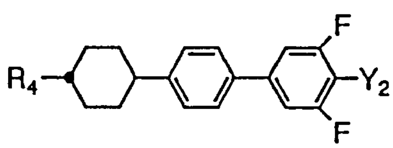 (5-20)

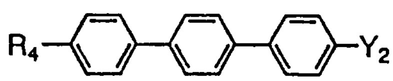 (5-21)

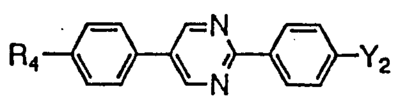 (5-22)

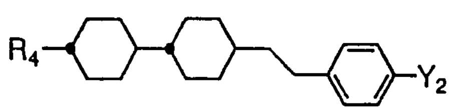 (5-23)

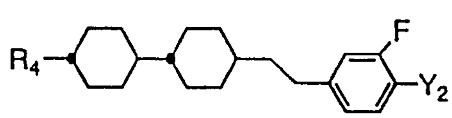 (5-24)

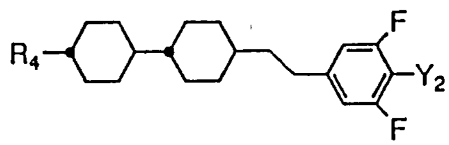
(5-25)

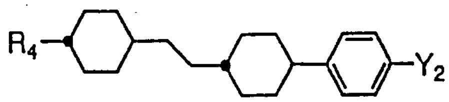
(5-26)

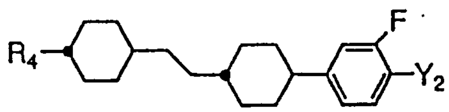
(5-27)

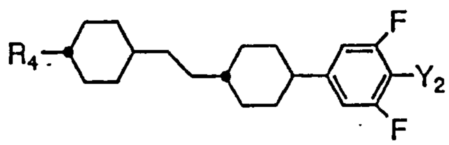
(5-28)

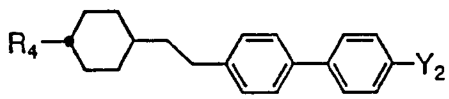
(5-29)

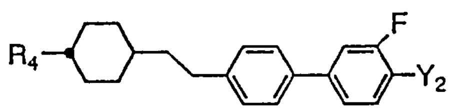
(5-30)

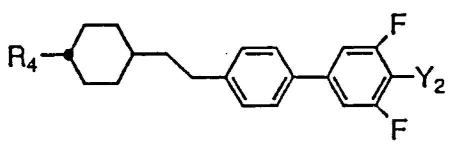
(5-31)

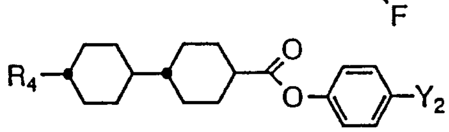
(5-32)

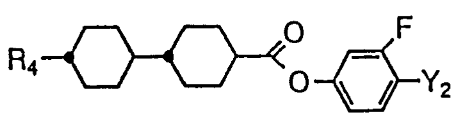
(5-33)

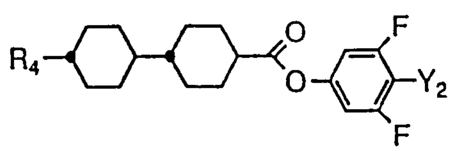
(5-34)

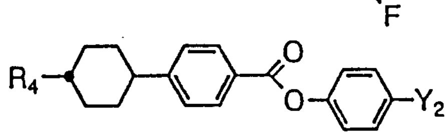
(5-35)

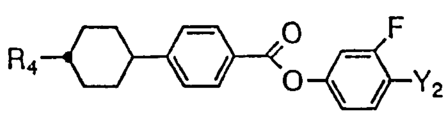
(5-36)

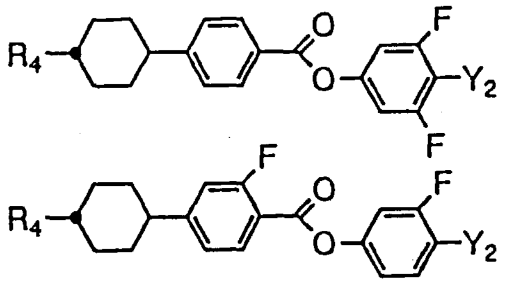

(5-37)

(5-38)

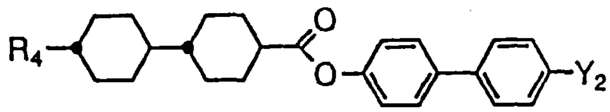

(5-39)

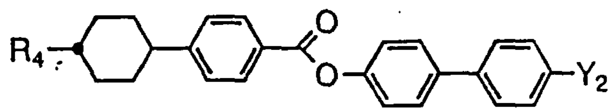

(5-40)

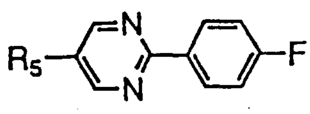

(6-1)

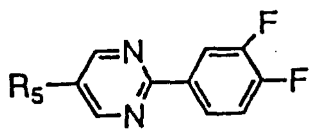

(6-2)

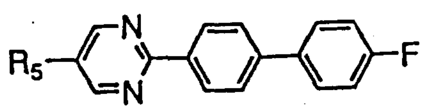

(6-3)

($R_4$, $R_5$ and $Y_2$ denote the same meanings as above.)

The compounds of the general formulae (5) and (6) have positive and high dielectric anisotropic values and they are used particularly for an object to decrease threshold voltage. Also, they are used for an object to enlarge nematic range, for example, adjustment of refractive anisotropic values and increase of clearing points. Furthermore, they are used for an object to improve sharpness of threshold voltage in liquid crystalline compositions for STN display mode and TN display mode.

The compounds of the general formulae (5) and (6) are indispensable compounds when preparing liquid crystalline compositions particularly for STN display mode and TN display mode.

When the used amounts of compounds of the general formulae (5) and (6) are increased, threshold voltage of the liquid crystalline compositions becomes low and viscosity becomes high. Thus, it is advantageous to use them in large quantities because of drivability at low voltage, provided that viscosity of the liquid crystalline compositions satisfies required characteristics. The used amounts of compounds of the general formulae (5) and (6) may be optionally within a range of 0.1 to 99.9% by weight, preferably 10 to 97% by weight, more preferably 40 to 95% by weight, relative to the total weight of the liquid crystalline composition.

As compounds of general formulae (7) to (9), the following compounds may be preferably mentioned.

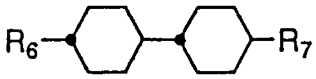
(7-1)

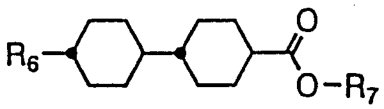
(7-2)

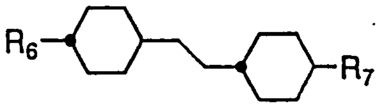
(7-3)

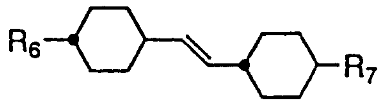
(7-4)

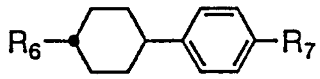
(7-5)

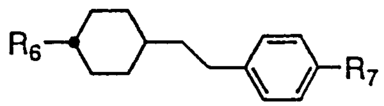
(7-6)

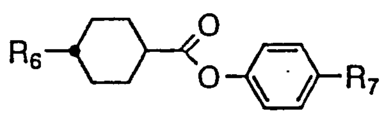
(7-7)

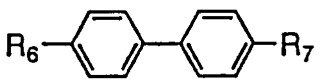
(7-8)

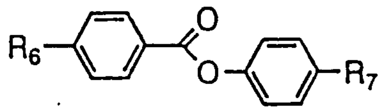
(7-9)

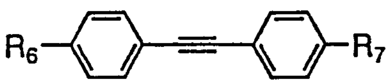
(7-10)

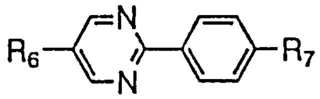
(7-11)

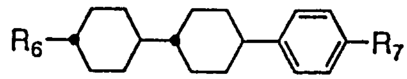

(8-1)

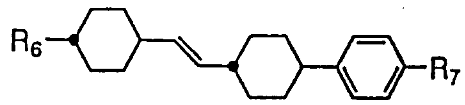

(8-2)

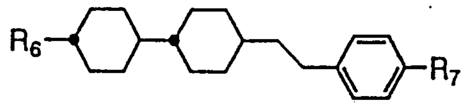

(8-3)

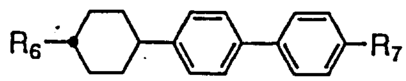

(8-4)

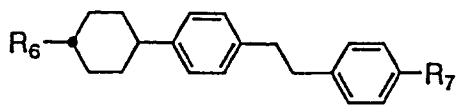

(8-5)

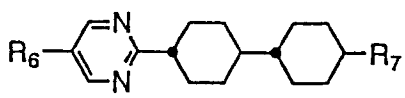

(8-6)

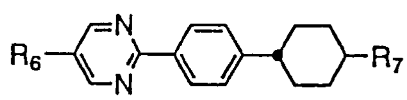

(8-7)

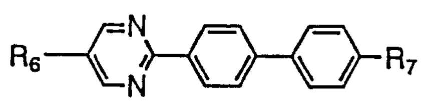

(8-8)

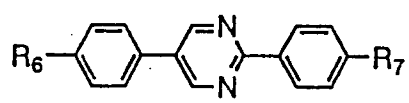

(8-9)

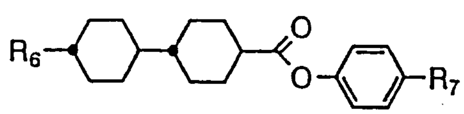

(8-10)

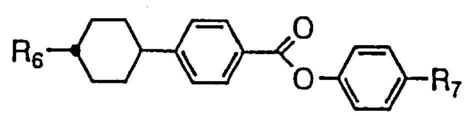

(8-11)

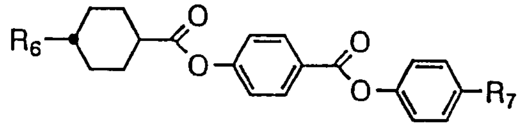

(8-12)

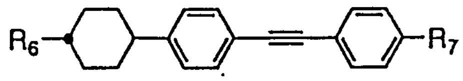
(8-13)

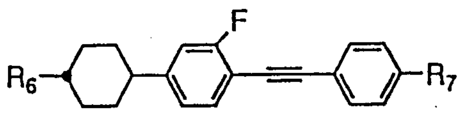
(8-14)

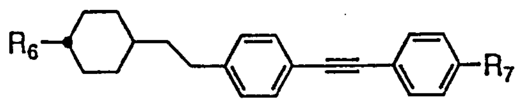
(8-15)

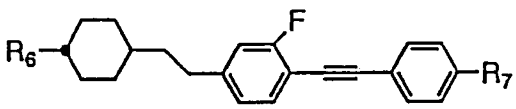
(8-16)

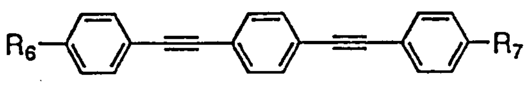
(8-17)

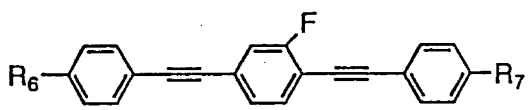
(8-18)

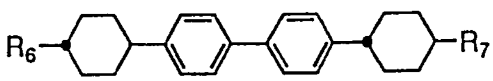
(9-1)

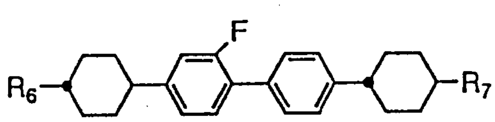
(9-2)

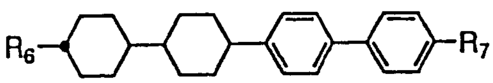
(9-3)

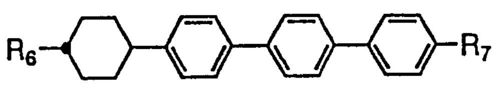
(9-4)

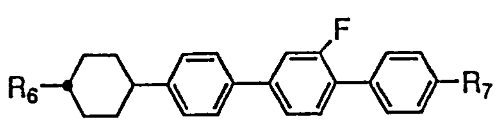
(9-5)

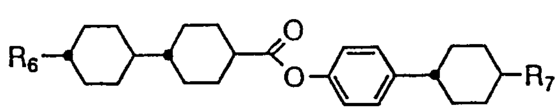
(9-6)

($R_6$ and $R_7$ denote the same meanings as above.)

The compounds of the general formulae (7) to (9) are compounds having low and nearly nuetral absolute values of dielectric anisotropy. The compounds of the general formula (7) are used for objects to adjust viscosity and to

adjust refractive anisotropic values. Also, the compounds of the general formulae (8) and (9) are used for objects to enlarge nematic range, for example, increase of clearing points and to adjust refractive anisotropic values.

When the used amounts of compounds of the general formulae (7) to (9) are increased, threshold voltage of the liquid crystalline compositions becomes high and viscosity becomes low. Thus, it is advantageous to use them in large quantities, provided that threshold voltage of liquid crystalline composition satisfies required values. The used amounts of the compounds of the general formulae (7) to (9) are preferably less than 40% by weight, more preferably less than 35% by weight, in the case of preparing liquid crystalline compositions for TFT. Also, they are preferably less than 70% by weight, more preferably less than 60% by weight, in the case of preparing liquid crystalline compositions for STN display mode or TN display mode.

As the compounds of general formulae (10) to (12), the following compounds may be preferably mentioned.

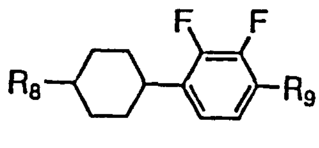
(10-1)

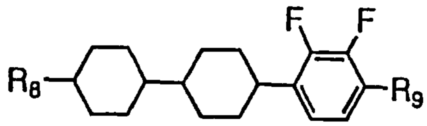
(11-1)

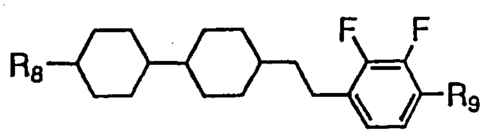
(11-2)

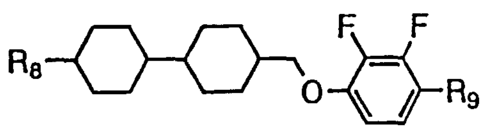
(11-3)

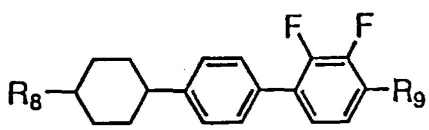
(11-4)

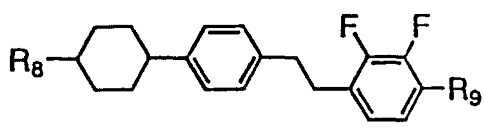
(11-5)

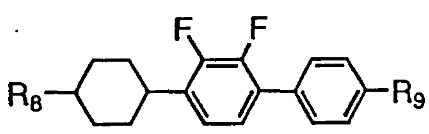
(12-1)

($R_8$ and $R_9$ denote the same meanings as above.)

The compounds of the general formulae (10) to (12) are compounds having negative and nearly nuetral dielectric anisotropy. Furthermore, the used amounts of the compounds of the general formulae (10) to (12) may be at will within a range of 0.1 to 99.9% by weight, 10 to 97% by weight, more preferably 10 to 60% by weight, in the case of preparing liquid crystalline compositions for TFT display mode, for STN display mode or TN display mode.

Furthermore, optically active compounds are generally added for objects to adjust necessary twist angle by driving helical structure of the liquid crystallline compositions and to inhibit reverse twist, except for special cases such as liquid crytalline compositions for OCB (Optically Compensated Birefringence) mode. As optically active compounds according to the invention, all of known optically active compounds may be used for the said objects, but the following

optically active compounds may be preferably mentioned.

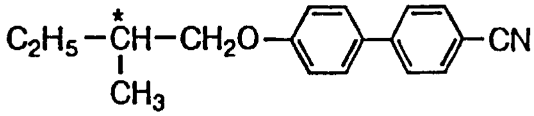

[記号：C15]

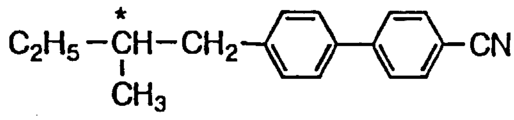

[記号：CB15]

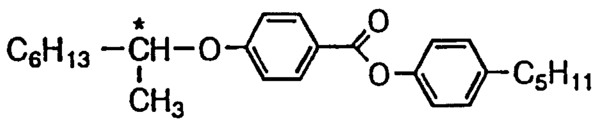

[記号：CM21]

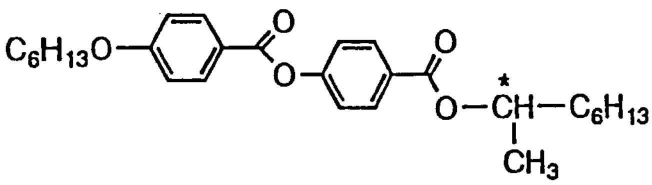

[記号：CM33]

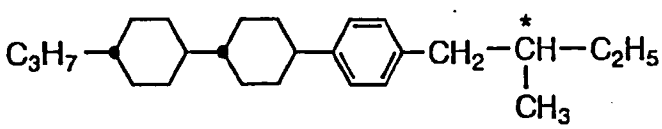

[記号：CM44]

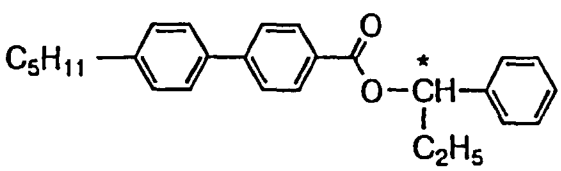

[記号：CM45]

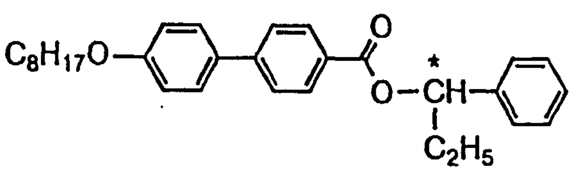

[記号：CM47]

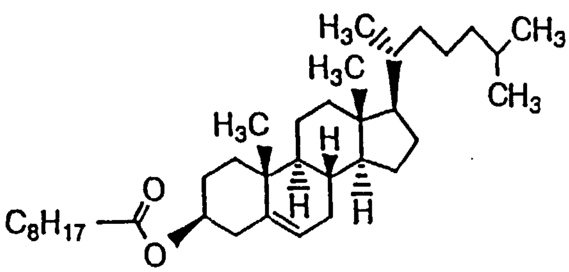

[記号：CN]

Liquid crytalline compositions according to the invention are adjusted in pitch length of twist by adding these

optically active compounds. Pitch length is preferably adjusted within a range of 40 to 200μm in the case of liquid crytalline compositions for TFT and TN. It is preferably adjusted within a range of 6 to 20μm in the case of liquid crytalline compositions for STN. Also, it is preferably adjusted within a range of 1.5 to 4 μm in the case of Bistable TN mode. Furthermore, for an object to adjust temperature dependency of pitch length, two or more optically active compounds may be added.

The liquid crytalline compositions prepared according to the invention are made by any of conventional methods. Generally, there may be took a mothod to dissolve various components mutually at high temperatures.

Furthermore, liquid crytalline compositions according to the invention may be used as liquid crytalline compositions for guest-host (GH) mode by adding bichromic dyes such as merocyanine type, styryl type, azo type, azomethine type, azoxy type, quinophthalone type, anthraquinone type and tetrazine type etc. Or, they may be used as liquid crystalline compositions for NCAD prepared by microcapsulating nematic liquid crystals or polymer dispersed type liquid crystalline display elements (PDLCD), for example, polymer network liquid crystalline display elements (PNLCD) having three-dimensional network macromolecules in liquid crystals. Additionally, they may be used as liquid crystalline compositions for effective controlling birefringent (ECB) mode and dinamic scattering (DS) mode.

Furthermore, the following Use Examples (Use Examples 1 to 29) can be shown as nematic liquid crystalline compositions containing compounds according to the invention. Herein, the compounds in Use Examples are experssed by abbreviations according to definitions shown in Table 1. Furthermore, as to the abbreviations in Use Examples, the case in which hydrogen atoms of trans-1,4-cyclohexylene group are substituted with deuterium atoms in $Q_3$, $Q_4$ and $Q_5$ in the following partial structures is shown by symbol H [3D, 4D, 5D], and the case in which they are substituted with deuterium atoms in $Q_7$, $Q_8$ and $Q_9$ is shown by H [7D, 8D, 9D], that is, positions substituted with deuterium are shown by numbers in [].

Table 1 Expression modes of compounds using symbole

$R-(A_1)-Z_1 \cdots\cdots\cdots Z_n-(A_n)-X$

| 1) left terminal group R- | symbol |
|---|---|
| $C_nH_{2n+1}-$ | n— |
| $C_nH_{2n+1}O-$ | nO— |
| $C_nH_{2n+1}OC_mH_{2m}-$ | nOm— |
| $CH_2=CH-$ | V— |
| $CH_2=CHC_nH_{2n}-$ | Vn— |
| $C_nH_{2n+1}CH=CHC_mH_{2m}-$ | nVm— |
| $C_nH_{2n+1}CH=CHC_mH_{2m}CH=CHC_kH_{2k}-$ | nVmVk— |

| 3) bonding group $-Z_1-$, $-Z_n-$ | symbol |
|---|---|
| $-C_2H_4-$ | 2 |
| $-C_4H_8-$ | 4 |
| —COO— | E |
| —C≡C— | T |
| —CH=CH— | V |
| $-CF_2O-$ | CF2O |
| $-OCF_2-$ | OCF2 |

| 2) ring structure $-(A_1)-$, $-(A_n)-$ | symbol |
|---|---|
| 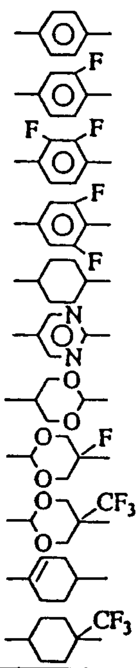 | B |
| | B(F) |
| | B(2F,3F) |
| | B(F,F) |
| | H |
| | Py |
| | d |
| | D(F) |
| | D(CF3) |
| | Ch |
| | H(CF3) |

| 4) right terminal group -X | symbol |
|---|---|
| —F | —F |
| —Cl | —CL |
| —CN | —C |
| $-CF_3$ | —CF3 |
| $-OCF_3$ | —OCF3 |
| $-OCF_2H$ | —OCF2H |
| $-C_nH_{2n+1}$ | —n |
| $-OC_nH_{2n+1}$ | —On |
| $-COOCH_3$ | —EMe |
| $-C_nH_{2n}CH=CH_2$ | —nV |
| $-C_mH_{2m}CH=CHC_nH_{2n+1}$ | —mVn |
| $-C_mH_{2m}CH=CHC_nH_{2n}F$ | —mVnF |
| $-CH=CF_2$ | —VFF |
| $-C_nH_{2n}CH=CF_2$ | —nVFF |
| —C≡C-CN | —TC |

5) Expression example

example 1 3-H2B(F,F)B(F)-F

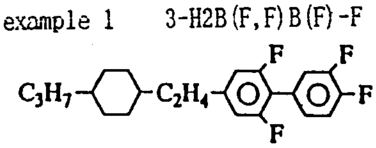

example 2 3-HB(F)TB-2

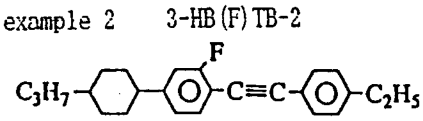

example 3 1V2-BEB(F,F)-C

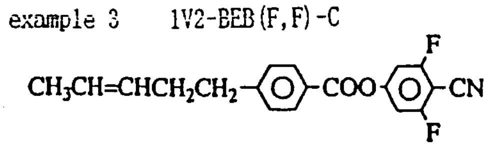

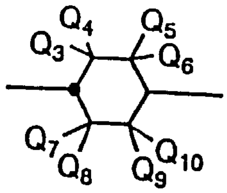

Use Example 1

| | |
|---|---|
| 5-HH(CF3)B-2 (No. 169) | 7.0% |
| 1V2-BEB(F,F)-C | 5.0% |
| 3-HB-C | 30.0% |
| 1-BTB-3 | 5.0% |
| 2-BTB-1 | 5.0% |
| 3-HH-4 | 11.0% |
| 3-HHB-1 | 4.0% |
| 3-HHB-3 | 9.0% |
| 3-H2BTB-2 | 4.0% |
| 3-H2BTB-3 | 4.0% |
| 3-H2BTB-4 | 4.0% |
| 3-HB(F)TB-2 | 6.0% |
| 3-HB(F)TB-3 | 6.0% |
| CM33 | 0.8 parts |

Use Example 2

| | |
|---|---|
| 5-HH(CF3)B(2F,3F)-02 (No. 171) | 5.0% |
| V2-HB-C | 12.0% |
| IV2-HB-C | 12.0% |
| 3-HB-C | 15.0% |
| 3-H[1D,2D,3D]-C | 9.0% |
| 3-HB(F)C | 5.0% |
| 2-BTB-1 | 2.0% |
| 3-HH-4 | 3.0% |
| 3-HH-VFF | 6.0% |

(continued)

| | |
|---|---|
| 3-H[1D,2D,3D]-HB-C | 3.0% |
| 3-HHB-C | 6.0% |
| 3-HB(F)TB-2 | 8.0% |
| 3-H2BTB-2 | 5.0% |
| 3-H2BTB-3 | 5.0% |
| 3-H2BTB-4 | 4.0% |

Use Example 3

| | |
|---|---|
| 3-HD(CF3)B(2F,3F)-2 (No. 198) | 7.0% |
| 201-BEB(F)-C | 5.0% |
| 301-BEB(F)-C | 15.0% |
| 401-BEB(F)-C | 13.0% |
| 501-BEB(F)-C | 13.0% |
| 2-HHB(F)-C | 15.0% |
| 3-HHB(F)-C | 15.0% |
| 3-HB(F)TB-2 | 4.0% |
| 3-HB(F)TB-3 | 4.0% |
| 3-HB(F)TB-4 | 4.0% |
| 3-HHB-1 | 3.0% |
| 3-HHB-01 | 2.0% |

Use Example 4

| | |
|---|---|
| 3-HD(F)B(2F,3F)-2 (No. 226) | 7.0% |
| 5-PyB-F | 4.0% |
| 3-PyB(F)-F | 4.0% |
| 2-BBC | 5.0% |
| 4-BBC | 4.0% |
| 5-BBC | 5.0% |
| 2-PyB-2 | 2.0% |
| 3-PyB-2 | 2.0% |
| 4-PyB-2 | 2.0% |
| 6-PyB-05 | 3.0% |
| 6-PyB-06 | 3.0% |

(continued)

| | |
|---|---|
| 6-PyB-07 | 3.0% |
| 6-PyB-08 | 3.0% |
| 3-PyBB-F | 6.0% |
| 4-PyBB-F | 6.0% |
| 5-PyBB-F | 6.0% |
| 3-HBB-1 | 7.0% |
| 2-H2BTB-2 | 4.0% |
| 2-H2BTB-3 | 4.0% |
| 2-H2BTB-4 | 5.0% |
| 3-H2BTB-2 | 5.0% |
| 3-H2BTB-3 | 5.0% |
| 3-H2BTB-4 | 5.0% |

TN1=90.3( °C)
η = 36.5(mPa.s)
Δ n = 0.200
Δ ε = 6.4
Vth=2.28(V)

Use Example 5

| | |
|---|---|
| 5-HD(F)H-2 (No.211) | 7.0% |
| 3-dB-C | 10.0% |
| 4-dB-C | 10.0% |
| 2-BEB-C | 12.0% |
| 3-BEB-C | 4.0% |
| 3-PyB(F)-F | 6.0% |
| 3-HEB-04 | 8.0% |
| 4-HEB-02 | 6.0% |
| 5-HEB-01 | 6.0% |
| 3-HEB-02 | 5.0% |
| 5-HEB-5 | 5.0% |
| 4-HEB-5 | 5.0% |
| 10-BEB-2 | 4.0% |
| 3-HHB-1 | 3.0% |
| 3-HHEBB-C | 3.0% |
| 3-HBEBB-C | 3.0% |

(continued)

| | |
|---|---|
| 5-HBEBB-C | 3.0% |

Use Example 6

| | |
|---|---|
| 30-HD(CF3)B-2 (No.207) | 8.0% |
| 3-HB-C | 18.0% |
| 7-HB-C | 3.0% |
| 101-HB-C | 10.0% |
| 3-HB(F)-C | 10.0% |
| 2-PyB-2 | 2.0% |
| 3-PyB-2 | 2.0% |
| 4-PyB-2 | 2.0% |
| 101-HH-3 | 7.0% |
| 2-BTB-01 | 7.0% |
| 3-HHB-1 | 7.0% |
| 3-HHB-F | 4.0% |
| 3-HHB-01 | 4.0% |
| 3-H2BTB-2 | 3.0% |
| 3-H2BTB-3 | 3.0% |
| 2-PyBH-3 | 4.0% |
| 3-PyBH-3 | 3.0% |
| 3-PyBB-2 | 3.0% |

Use Example 7

| | |
|---|---|
| 3-HD(F)B-2 (No. 229) | 10.0% |
| 201-BEB(F)-C | 5.0% |
| 301-BEB(F)-C | 12.0% |
| 501-BEB(F)-C | 4.0% |
| 1V2-BEB(F,F)-C | 10.0% |
| 3-HH-EMe | 10.0% |
| 3-HB-02 | 18.0% |
| 7-HEB-F | 2.0% |
| 3-HHEB-F | 2.0% |

(continued)

| | |
|---|---|
| 5-HHEB-F | 2.0% |
| 3-HBEB-F | 4.0% |
| 201-HBEB(F)-C | 2.0% |
| 3-HB(F)EB(F)-C | 2.0% |
| 3-HBEB(F,F)-C | 2.0% |
| 3-HHB-F | 4.0% |
| 3-HHB-01 | 4.0% |
| 3-HHB-3 | 3.0% |
| 3-HEBEB-F | 2.0% |
| 3-HEBEB-1 | 2.0% |

TN1=71.5 ( °C)
$\eta$ = 37.5 (mPa.s)
$\Delta$ n = 0.113
$\Delta$ $\varepsilon$ = 24.2
Vth=0.98 (V)

Use Example 8

| | |
|---|---|
| 3-HD(F)B-2 (No. 229) | 7.0% |
| V-HD(F)B-3 (No. 232) | 7.0% |
| 5-BEB(F)-C | 5.0% |
| V-HB-C | 16.0% |
| 5-PyB-C | 6.0% |
| 4-BB-3 | 6.0% |
| 3-HH-2V | 10.0% |
| 5-HH-V | 11.0% |
| V-HHB-1 | 7.0% |
| V2-HHB-1 | 8.0% |
| 3-HHB-1 | 2.0% |
| IV2-HBB-2 | 10.0% |
| 3-HHEBH-3 | 5.0% |

TN1=82.2( °C)
$\eta$ = 20.8(mPa.s)
$\Delta$ n = 0.111
$\Delta$ $\varepsilon$ = 5.1
Vth=2.31(V)

Use Example 9

| | |
|---|---|
| 5-HD(F)H-2 (No. 211) | 3.0% |
| 3-HD(F)B(2F,3F)-2 (No.226) | 3.0% |
| 3-HD(F)B-2 (No.229) | 9.0% |
| 201-BEB(F)-C | 5.0% |
| 301-BEB(F)-C | 12.0% |
| 501-BEB(F)-C | 4.0% |
| IV2-BEB(F,F)-C | 16.0% |
| 3-HB-02 | 10.0% |
| 3-HH-4 | 3.0% |
| 3-HHB-F | 3.0% |
| 3-HHB-1 | 8.0% |
| 3-HHB-01 | 4.0% |
| 3-HBEB-F | 4.0% |
| 3-HHEB-F | 7.0% |
| 5-HHEB-F | 7.0% |
| 3-H2BTB-2 | 4.0% |
| 3-H2BTB-3 | 4.0% |
| 3-H2BTB-4 | 4.0% |
| 3-HB(F)TB-2 | 5.0% |

Use Example 10

| | |
|---|---|
| 5-HH(CF3)B-2 (No. 169) | 7. 0% |
| 2-BEB-C | 12.0% |
| 3-BEB-C | 4.0% |
| 4-BEB-C | 6.0% |
| 3-HB-C | 28.0% |
| 3-HEB-04 | 12.0% |
| 4-HEB-02 | 8.0% |
| 5-HEB-01 | 8.0% |
| 3-HEB-02 | 6.0% |
| 5-HEB-02 | 5.0% |
| 3-HHB-1 | 2.0% |

(continued)

| | |
|---|---|
| 3-HHB-01 | 2.0% |

Use Example 11

| | |
|---|---|
| 3-HD(CF3)B(2F,3F)-2 (No. 198) | 5.0% |
| 2-BEB-C | 10.0% |
| 5-BB-C | 12.0% |
| 7-BB-C | 7.0% |
| 1-BTB-3 | 7.0% |
| 2-BTB-1 | 10.0% |
| 10-BEB-2 | 10.0% |
| 10-BEB-5 | 12.0% |
| 2-HHB-1 | 4.0% |
| 3-HHB-F | 4.0% |
| 3-HHB-1 | 7.0% |
| 3-HHB-01 | 4.0% |
| 3-HHB-3 | 8.0% |

Use Example 12

| | |
|---|---|
| 30-HD(CF3)B-2 (No. 207) | 4.0% |
| 3-HD(F)B(2F,3F)-2 (No. 226) | 4.0% |
| 1V2-BEB(F,F)-C | 8.0% |
| 3-HB-C | 10.0% |
| V2V-HB-C | 20.0% |
| V2V-HH-3 | 13.0% |
| 3-HB-02 | 4.0% |
| 3-HHB-1 | 10.0% |
| 3-HHB-3 | 7.0% |
| 3-HB(F)TB-2 | 4.0% |
| 3-HB(F)TB-3 | 4.0% |
| 3-H2BTB-2 | 4.0% |
| 3-H2BTB-3 | 4.0% |
| 3-H2BTB-4 | 4.0% |

Use Example 13

| | |
|---|---|
| 5-HH(CF3)B-2) (No.169) | 3.0% |
| 5-HH(CF3)B(2F,3F)-2 (No. 171) | 3.0% |
| 5-BTB(F)TB-3 | 10.0% |
| V2-HB-TC | 10.0% |
| 3-HB-TC | 10.0% |
| 3-HB-C | 10.0% |
| 5-HB-C | 7.0% |
| 5-BB-C | 3.0% |
| 2-BTB-1 | 10.0% |
| 2-BTB-01 | 5.0% |
| 3-HH-4 | 5.0% |
| 3-HHB-1 | 10.0% |
| 3-HHB-3 | 5.0% |
| 3-H2BTB-2 | 3.0% |
| 3-H2BTB-3 | 3.0% |
| 3-HB(F)TB-2 | 3.0% |

Use Example 14

| | |
|---|---|
| 5-HD(F)H-2 (No.211) | 4.0% |
| 3-HD(F)B(2F,3F)-2 (No.226) | 4.0% |
| 1V2-BEB(F,F)-C | 6.0% |
| 3-HB-C | 18.0% |
| 2-BTB-1 | 10.0% |
| 5-HH-VFF | 30.0% |
| 1-BHH-VFF | 8.0% |
| 1-BHH-2VFF | 3.0% |
| 3-H2BTB-2 | 5.0% |
| 3-H2BTB-3 | 4.0% |
| 3-H2BTB-4 | 4.0% |
| 3-HHB-1 | 4.0% |

Use Example 15

| | |
|---|---|
| 30-HD(CF3)B-2 (No. 207) | 4.0% |
| 3-HD(F)B-2 (No. 229) | 4.0% |
| V-HD(F)B-3 (No. 232) | 4.0% |
| 2-HB-C | 5.0% |
| 3-HB-C | 12.0% |
| 3-HB-02 | 15.0% |
| 2-BTB-1 | 3.0% |
| 3-HHB-1 | 10.0% |
| 3-HHB-F | 4.0% |
| 3-HHB-01 | 5.0% |
| 3-HHEB-F | 4.0% |
| 5-HHEB-F | 4.0% |
| 2-HHB(F)-F | 7.0% |
| 3-HHB(F)-F | 7.0% |
| 5-HHB(F)-F | 7.0% |
| 3-HHB(F,F)-F | 5.0% |

Use Example 16

| | |
|---|---|
| 5-HH(CF3)B-2 (No. 169) | 5.0% |
| 2-HHB(F)-F | 17. 0% |
| 3-HHB(F)-F | 17.0% |
| 5-HHB(F)-F | 16.0% |
| 2-H2HB(F)-F | 10.0% |
| 3-H2HB(F)-F | 5.0% |
| 5-H2HB(F)-F | 5.0% |
| 2-HBB(F)-F | 6.0% |
| 3-HBB(F)-F | 6.0% |
| 3-HBB(F)-F | 13.0% |
| CN | 0.3 parts |

Use Example 17

| | |
|---|---|
| 5-HH(CF3)B-2 (No. 169) | 4.0% |
| 5-HH(CF3)B(2F,3F)-02 (No. 171) | 4.0% |
| 7-HB(F)-F | 5.0% |
| 5-H2B(F)-F | 5.0% |
| 3-HB-02 | 10.0% |
| 3-HH-4 | 2.0% |
| 3-HH[5D,6D,7D]-4 | 3.0% |
| 2-HHB(F)-F | 10. 0% |
| 3-HHB(F)-F | 10.0% |
| 5-HH[5D,6D,7D]B(F)-F | 10.0% |
| 3-H2HB(F)-F | 5.0% |
| 2-HBB(F)-F | 3.0% |
| 3-HBB(F)-F | 3.0% |
| 5-HBB(F)-F | 6.0% |
| 2-H2BB(F)-F | 5.0% |
| 3-H2BB(F)-F | 6.0% |
| 3-HHB-1 | 4.0% |
| 3-HHB-01 | 5.0% |

Use Example 18

| | |
|---|---|
| 5-HH(CF3)B(2F,3F)-02 (No.171) | 4.0% |
| 3-HH(CF3)B-2 (No. 173) | 4.0% |
| 7-HB(F,F)-F | 3.0% |
| 3-HB-02 | 7.0% |
| 2-HHB(F)-F | 10.0% |
| 3-HHB(F)-F | 10.0% |
| 5-HHB(F)-F | 10.0% |
| 2-HBB(F)-F | 9.0% |
| 3-HBB(F)-F | 9.0% |
| 5-HBB(F)-F | 16.0% |
| 2-HBB-F | 3.0% |
| 3-HBB(F,F)-F | 5.0% |

(continued)

| | |
|---|---|
| 5-HBB(F,F)-F | 10.0% |

Use Example 19

| | |
|---|---|
| 5-HH(CF3)B(2F,3F)-02 (No. 171) | 5.0% |
| 7-HB(F,F)-F | 3.0% |
| 3-H2HB(F,F)-F | 12.0% |
| 4-H2HB(F,F)-F | 10.0% |
| 5-H2HB(F,F)-F | 10.0% |
| 3-HHB(F,F)-F | 5.0% |
| 4-HHB(F,F)-F | 5.0% |
| 3-HH2B(F,F)-F | 15.0% |
| 5-HH2B(F,F)-F | 5.0% |
| 3-HBB(F,F)-F | 12.0% |
| 5-HBB(F,F)-F | 12.0% |
| 3-HBCF20B(F,F)-F | 6.0% |

Use Example 20

| | |
|---|---|
| 5-HD(F)H-2 (No.211) | 5.0% |
| 3-HD(F)B-2 (No.229) | 5.0% |
| 7-HB(F,F)-F | 5.0% |
| 3-H2HB(F,F)-F | 12.0% |
| 3-HHB(F,F)-F | 10.0% |
| 4-HHB(F,F)-F | 5.0% |
| 3-HBB(F,F)-F | 10.0% |
| 3-HHEB(F,F)-F | 10.0% |
| 4-HHEB(F,F)-F | 3.0% |
| 5-HHEB(F,F)-F | 3.0% |
| 2-HBEB(F,F)-F | 3.0% |
| 3-HBEB(F,F)-F | 5.0% |
| 5-HBEB(F,F)-F | 3.0% |
| 3-HdB(F,F)-F | 15.0% |
| 3-HHBB(F,F)-F | 6.0% |

Use Example 21

| | |
|---|---|
| 3-HD(F)B-2 (No. 229) | 4.0% |
| V-HD(F)B-3 (No. 232) | 4.0% |
| 3-HB-CL | 10.0% |
| 5-HB-CL | 4.0% |
| 7-HB-CL | 6.0% |
| 101-HH-5 | 3.0% |
| 2-HBB(F)-F | 8.0% |
| 3-HBB(F)-F | 8.0% |
| 5-HBB(F)-F | 14.0% |
| 4-HHB-CL | 8.0% |
| 3-H2HB(F)-CL | 4.0% |
| 3-HBB(F,F)-F | 10.0% |
| 5-H2BB(F,F)-F | 9.0% |
| 3-HB(F)VB-2 | 4.0% |
| 3-HB(F)VB-3 | 4.0% |

TN1=86.7 ( °C)
$\eta$ =22.7 (mPa.s)
$\Delta$ n = 0.126
$\Delta$ $\varepsilon$ = 4.7
Vth=2.34 (V)

Use Example 22

| | |
|---|---|
| 5-HH(CF3)B(2F,3F)-02 (No.171) | 4.0% |
| 3-HD(F)B-2 (No.229) | 4.0% |
| 3-HHB(F,F)-F | 9.0% |
| 3-H2HB(F,F)-F | 8.0% |
| 4-H2HB(F,F)-F | 8.0% |
| 3-HBB(F,F)-F | 21.0% |
| 5-HBB(F,F)-F | 20.0% |
| 3-H2BB(F,F)-F | 10.0% |
| 5-HHBB(F,F)-F | 3.0% |
| 5-HHEBB-F | 2.0% |

(continued)

| | |
|---|---|
| 3-HH2BB(F,F)-F | 3.0% |
| 101-HBBH-4 | 4.0% |
| 101-HBBH-5 | 4.0% |

Use Example 23

| | |
|---|---|
| 5-HD(F)H-2 (No.211) | 5.0% |
| 5-HB-F | 12.0% |
| 6-HB-F | 9.0% |
| 7-HB-F | 7.0% |
| 2-HHB-OCF3 | 7.0% |
| 3-HHB-OCF3 | 7.0% |
| 4-HHB-OCF3 | 7.0% |
| 3-HH2B-OCF3 | 4.0% |
| 5-HH2B-OCF3 | 4.0% |
| 3-HHB(F,F)-OCF3 | 5.0% |
| 3-HBB(F)-F | 10.0% |
| 5-HBB(F)-F | 10.0% |
| 3-HH2B(F)-F | 3.0% |
| 3-HB(F)BH-3 | 3.0% |
| 5-HBBH-3 | 3.0% |
| 3-HHB(F,F)-OCF2H | 4.0% |

Use Example 24

| | |
|---|---|
| V-HD(F)B-3 (No.232) | 5.0% |
| 5-H4HB(F,F)-F | 7.0% |
| 5-H4HB-OCF3 | 15.0% |
| 3-H4HB(F,F)-CF3 | 8.0% |
| 5-H4HB(F,F)-CF3 | 5.0% |
| 3-HB-CL | 6.0% |
| 5-HB-CL | 4.0% |
| 2-H2BB(F)-F | 5.0% |
| 3-H2BB(F)-F | 10.0% |

(continued)

| | |
|---|---|
| 5-HVHB(F,F)-F | 5.0% |
| 3-HHB-OCF3 | 5.0% |
| 3-H2HB-OCF3 | 5.0% |
| V-HHB(F)-F | 5.0% |
| 3-HHB(F)-F | 5.0% |
| 5-HHEB-OCF3 | 2.0% |
| 3-HBEB (F, F)-F | 5.0% |
| 5-HH-V2F | 3.0% |

TN1=72.0( °C)
$\eta$ = 24.5(mPa.s)
$\Delta$ n = 0.094
$\Delta$ $\varepsilon$ = 7.8
Vth=1.79(V)

Use Example 25

| | |
|---|---|
| 5-HH(CF3)B(2F,3F)-02 (No.171) | 7.0% |
| 2-HHB(F)-F | 2.0% |
| 3-HHB(F)-F | 2.0% |
| 5-HHB(F)-F | 2.0% |
| 2-HBB(F)-F | 6.0% |
| 3-HBB(F)-F | 6.0% |
| 5-HBB(F)-F | 10.0% |
| 2-H2BB(F)-F | 9.0% |
| 3-H2BB(F)-F | 9.0% |
| 3-HBB(F,F)-F | 18.0% |
| 5-HBB(F,F)-F | 19.0% |
| 101-HBBH-4 | 5.0% |
| 101-HBBH-5 | 5.0% |

Use Example 26

| | |
|---|---|
| 3-HH(CF3)B-2 (No.173) | 4.0% |
| 5-HD(F)H-2 (No. 211) | 3.0% |

(continued)

| | |
|---|---|
| 3-HD(F)B-2 (No.229) | 4.0% |
| 5-HB-CL | 12.0% |
| 3-HH-4 | 3.0% |
| 3-HB-02 | 20.0% |
| 3-H2HB(F,F)-F | 4.0% |
| 3-HHB(F,F)-F | 8.0% |
| 3-HBB(F,F)-F | 6.0% |
| 2-HHB(F)-F | 5.0% |
| 3-HHB(F)-F | 5.0% |
| 5-HHB(F)-F | 5.0% |
| 2-H2HB(F)-F | 2.0% |
| 3-H2HB(F)-F | 1.0% |
| 5-H2HB(F)-F | 2.0% |
| 3-HHBB(F,F)-F | 4.0% |
| 3-HBCF20B-OCF3 | 4.0% |
| 5-HBCF20B(F,F)-CF3 | 4.0% |
| 3-HHB-01 | 4.0% |

Use Example 27

| | |
|---|---|
| 5-HH(CF3)B-2 (No.169) | 5.0% |
| 3-HH(CF3)B-2 (No.173) | 5.0% |
| 5-HH(CF3)B(2F,3F)-02 (No.171) | 5.0% |
| 3-HD(F)B-2 (No.229) | 5.0% |
| 3-HB-02 | 10.0% |
| 3-HB-04 | 10.0% |
| 3-HEB-04 | 16.0% |
| 4-HEB-02 | 13.0% |
| 5-HEB-01 | 13.0% |
| 3-HEB-02 | 10.0% |
| 5-HEB-02 | 8.0% |

Use Example 28

| | |
|---|---|
| 5-HH(CF3)B-2 (No.169) | 5.0% |

(continued)

| | |
|---|---|
| 3-HH(CF3)B-2 (No.173) | 5.0% |
| 5-HH(CF3)B(2F,3F)-02 (No. 171) | 5.0% |
| 3-HD(F)B-2 (No.229) | 10.0% |
| 5-HD(F)H-2 (No.211) | 5.0% |
| 3-HD(F)B(2F,3F)-2 (No.226) | 10.0% |
| 3-HB(2F,3F)-02 | 20.0% |
| 5-HHB(2F,3F)-02 | 10.0% |
| 5-HBB(2F,3F)-2 | 10.0% |
| 5-HBB(2F,3F)-02 | 10.0% |
| 5-HHB(2F,3F)-101 | 5.0% |
| 5-HBB(2F,3F)- 101 | 5.0% |

Use Example 29

| | |
|---|---|
| 5-HH(CF3)B-2 (No.169) | 5.0% |
| 5-HH(CF3)B(2F,3F)-02 (No.171) | 5.0% |
| 3-HD(F)B(2F,3F)-2 (No.226) | 5.0% |
| 3-HB-02 | 15.0% |
| 3-HB-04 | 10.0% |
| 3-HEB-04 | 10.0% |
| 4-HEB-02 | 7.0% |
| 5-HEB-01 | 7.0% |
| 3-HEB-02 | 6.0% |
| 5-HEB-02 | 5.0% |
| 3-HB(2F,3F)-02 | 7.0% |
| 5-HHB(2F,3F)-02 | 5.0% |
| 5-HBB(2F,3F)-2 | 5.0% |
| 5-HBB(2F,3F)-02 | 4.0% |
| 5-BB(2F,3F)-3 | 4.0% |

Preparation methods of compounds

Compounds expressed by the general formula (1) according to the invention can be prepared easily by using conventional organic synthetic methods. For example, they may be synthesized easily by selecting and combining methods such as described in Organic Synthesis, Organic Reactions and Experimental Chemical Course (published by Maruzen Kabushiki Kaisha).

That is, in the case of compounds expressed by the general formula (1-a) wherein $Q_1$ and $Q_2$ on saturated six-membered ring being both $CH_2$ groups and substituent X being trifluoromethoxy group in general formula (1), the objective compounds expressed by the general formula (1-a) can be prepared by reacting alcohol derivatives (11) which can be prepared according to the method described in Official Patent Gazette of Toku-Ko-Sho 62-39136 with sodium hydride, carbon disulfide and methyl iodide according to the method described in Journal of Synthetic Organi c Chemistry JAPAN, Vol 51, No. 12, p 22 (Manabu Kurohoshi et al. ,Survey) to make xanthate (12), and then reacting HF-pyridine in the presence of 1,3-dibromo-5,5-dimethylhydantoin (hereinafter, referredto DBH) with using the xanthate as an oxidant.

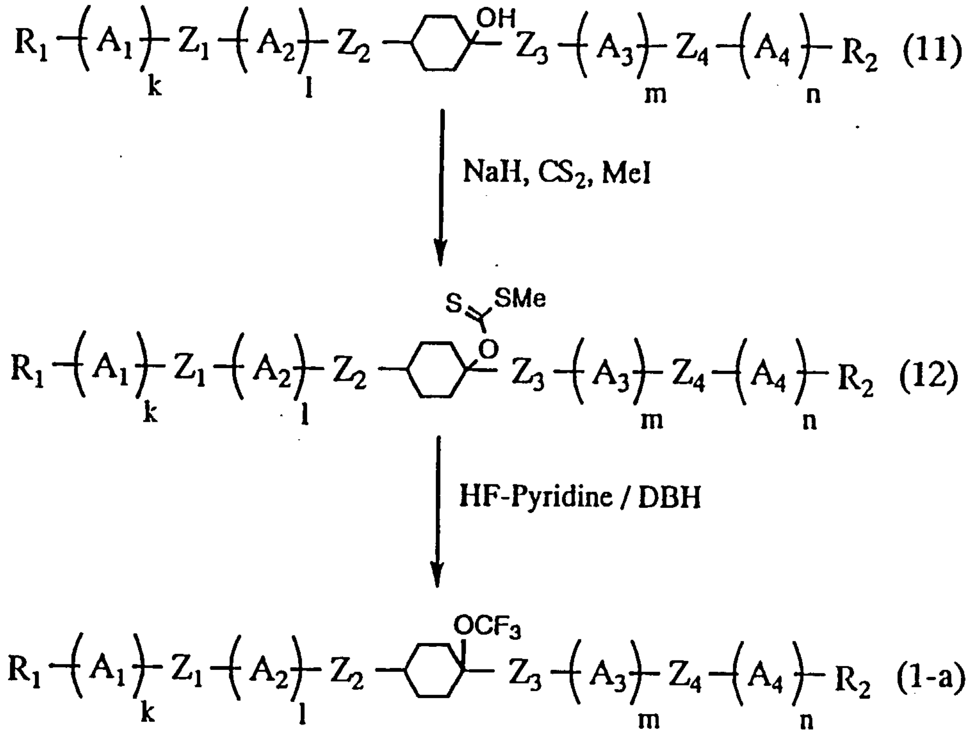


(In the above mentioned formulae, $R_1$, $R_2$, $A_1$, $A_2$, $A_3$, $A_4$, $Z_1$, $Z_2$, $Z_3$, $Z_4$, k, l, m and n have the same meanings as above.)

Furthermore, compounds expressed by the general formula (1-b) wherein $Q_1$ and $Q_2$ on saturated six-membered ring being both $CH_2$ groups, $Z_3$ being covalent bond, m=1, $A_3$ being 1,4-phenylene group optionally substituted with (a) halogen atom(s) and substituent X being trifluoromethyl group in the general formula (1) can be preferably prepared according to the following method.

Namely, similar to the above-mentioned (11), an alcohol derivative (11) which can be prepared according to the method described in Official Patent Gazette of Toku-Ko-Sho 62-39136 is reacted with carbon tetrahalide in the presence of triphenyl phosphine according to the method of J. G. Calzada et al. (Org. Synth., 54, 63 (1974)) to prepare a halobenzyl derivative (14).

Then, according to the trifluoromethylation method reported by Qing-Yun Chen et al. in J. Chem. Soc. Chem. Commun., 1989, 709, (14) is reacted with methyl fluorosulfonyl(difluoro)acetica cid methyl ester with using cupper iodide as a catalyst, to prep are the objectivederivative expressed by the general formula (1-b).

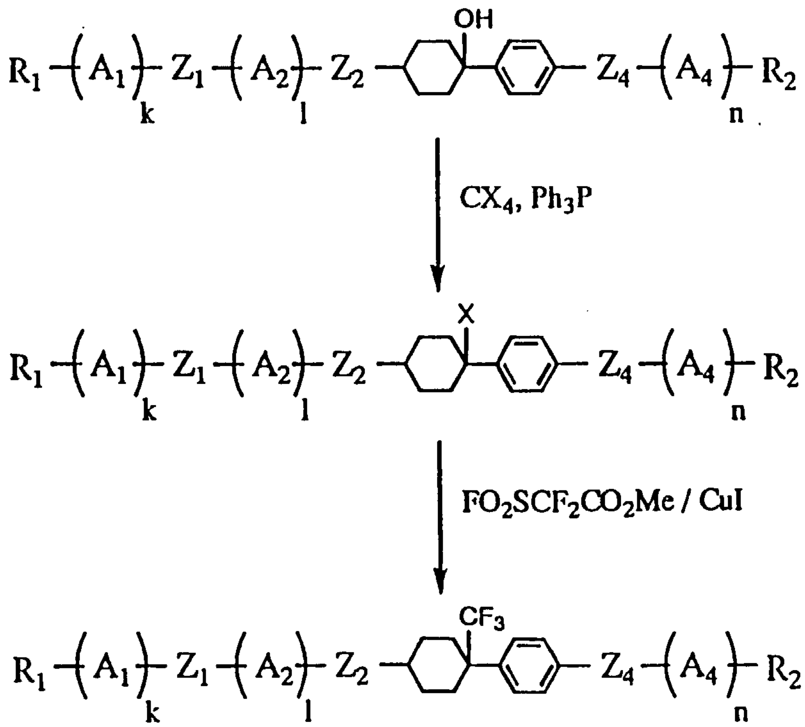


(13)

(14)

(1-b)

(In the above mentioned formulae, $R_1$, $R_2$, $A_1$, $A_2$, $A_4$, $Z_1$, $Z_2$, $Z_4$, k, l and n have the same meanings as above, and X denotes halogen atom.)

Furthermore, compounds expressed by the general formula (1-c) wherein $Q_1$ and $Q_2$ on saturated six-membered ring being both oxygen atoms and substituent X being halogen atom in the general formula (1), can be preferably prepared according to the following method. Namely, ester derivative expressed by the general formula (15) is reacted with lithium diisopropylamide (hereinafter, referred to LDA) to lithiate $\alpha$-position of ester carbonyl, and then reacted with alkyl chloroformate to prepare a malonic diester derivative (16).

Then, compound (16) is reacted with a base such as sodium alkoxide, sodium hydride or LDA etc. to remove hydrogen at $\alpha$-position of carbonyl group and form carbanion, and thereafter reacted with lithium halide, sodium halide or potassium halide to prepare compound (17) wherein $\alpha$-position of carbonyl group being substituted with halogen atom. (17) is then reduced with lithium aluminium hydride and converted to a propylene glycol derivative (18), and threafter reacted with an aldehyde derivative expressed by the general formula (19) in the presence of an acid catalyst to prepare the objective compound (1-c) wherein 5 position on dioxane ring being substituted with halogen atom.

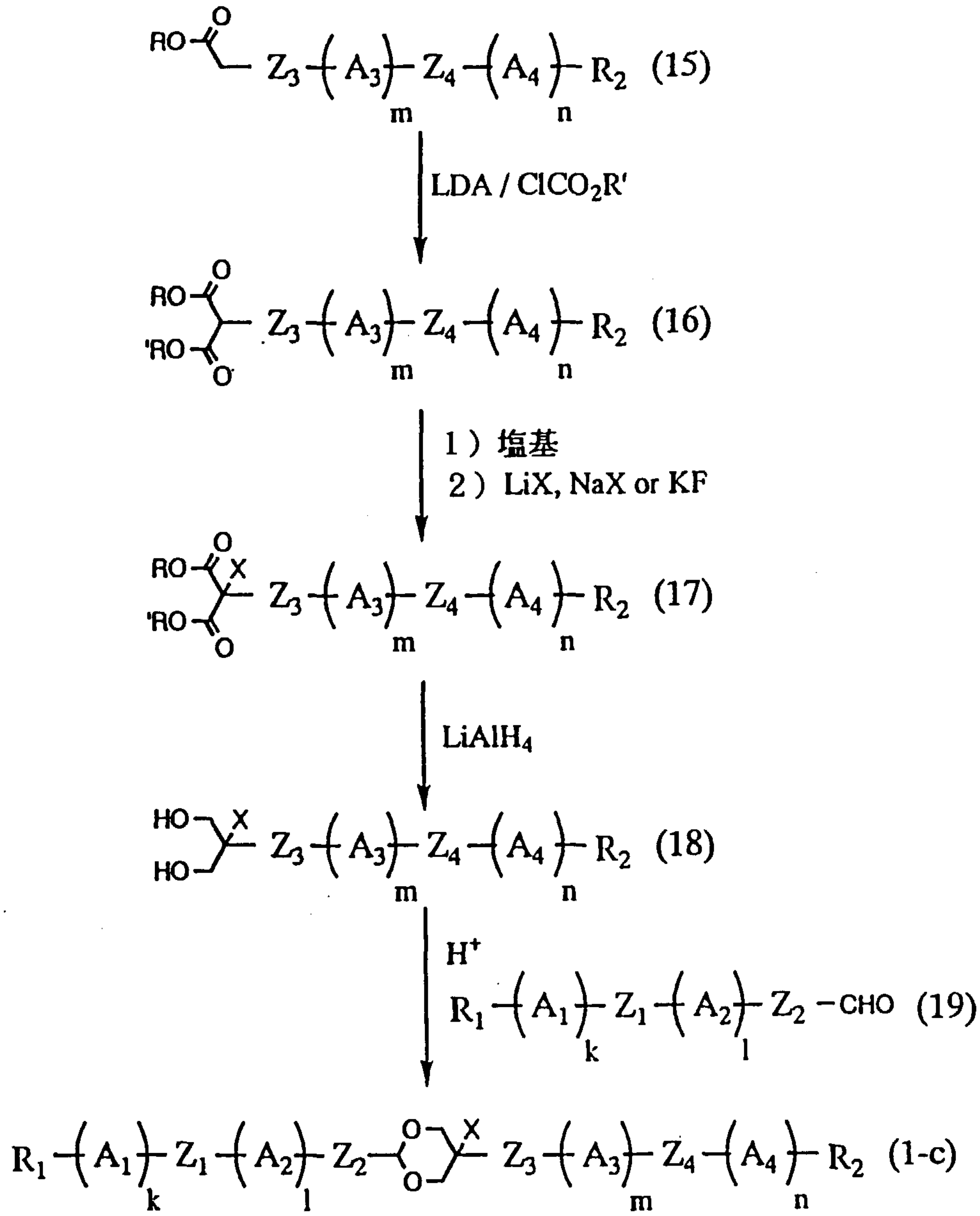


(15)
(16)
(17)
(18)
(19)
(1-c)

(In the above-mentioned formulae, $R_1$, $R_2$, $A_1$, $A_2$, $A_3$, $A_4$, $Z_1$, $Z_2$, $Z_3$, $Z_4$, k, l, m and n have the same meanings as above, X denotes halogen atom, R and R' denote alkyl groups.)

Furthermore, compounds expressed by the general formula (1-d) wherein $Q_1$ and $Q_2$ on saturated six-membered ring being both oxygen atoms, $Z_3$ being covalent bond, m=1, $A_3$ being 1,4-phenylene group optionally substituted with (a) halogen atom(s), and substituent X being $CF_3$ in the general formula (1) can be preferably prepared according to the following method. Namely, a halogen substituted compound (17a) which can be prepared similar to the above-mentioned compound (17) is reacted with fluorosulfonyl(difluoro)aceticacid methylester with using cupper iodide as a catalyst according to the trifluoromethylation method by Qing-Yun Chen et al., to prepare a trifluoromethylated compound (20), then by the method similar for preparing (1-c) from the above-mentioned (17), to prepare the compound (1-d) wherein 5 position on dioxane ring being substituted with trifluoromethyl group.

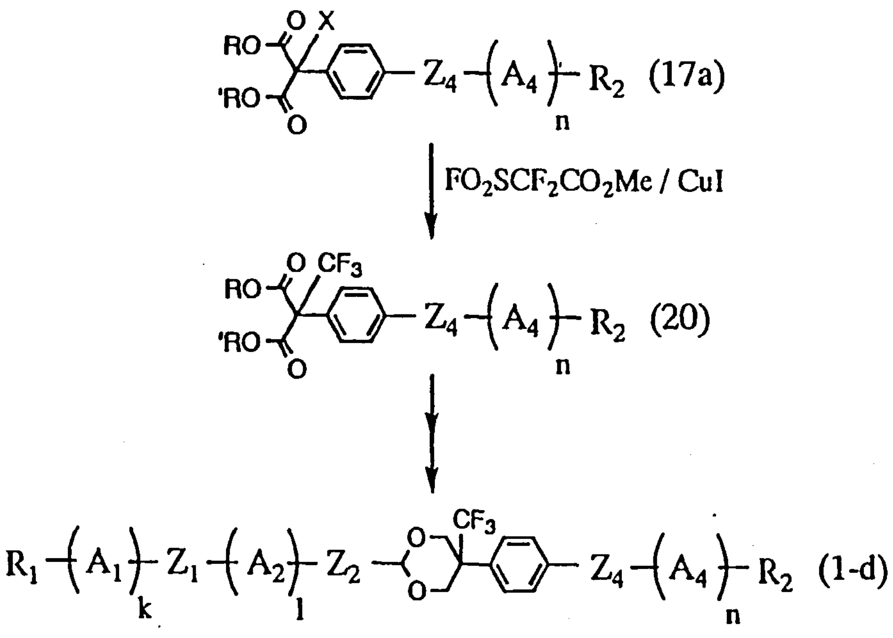


(17a)

(20)

(1-d)

(In the above mentioned formulae, $R_1$, $R_2$, $A_1$, $A_2$, $A_4$, $Z_1$, $Z_2$, $Z_4$, k, l and n have the same meanings as above, X denotes halogen atom, R and R' denote alkyl group.)

Furthermore, compounds expressed by the general formula (1-e) wherein $Q_1$ and $Q_2$ on saturated six-membered ring being both oxygen atoms, $Z_3$ being covalent bond, m=1, $A_3$ being 1,4-cyclohexylene group and substituent X being $OCF_3$ in the general formula (1) can be preferably prepared according to the following method. Namely, a cyclohexanone derivative (21) is condensed with diethyl malonate in the persence of titanium tetrachloride and pyridine to prepare compound (22). Then, compound (22) is hydroborated according to the method reported by W.Lehner et al. described in Tetrahedron, 24, 5701 (1968) to prepare an alcohol derivative (23). (23) is then converted into xanthate according to the method described in (Journal of Synthetic Organic Chemistry JAPAN) Vol 51, No. 12, p 22 (Manabu Kurohoshi et al., Survey) , thereafter reacted with HF-pyridine in the presence of DBH to prepare a trifluoromethoxylated compound (24). Furthermore, by the method similar for preparing (1-c) from the above-mentioned (17), the compound (1-e) wherein 5-position on dioxane ring being substituted with trifluoromethoxy group can be prepared.

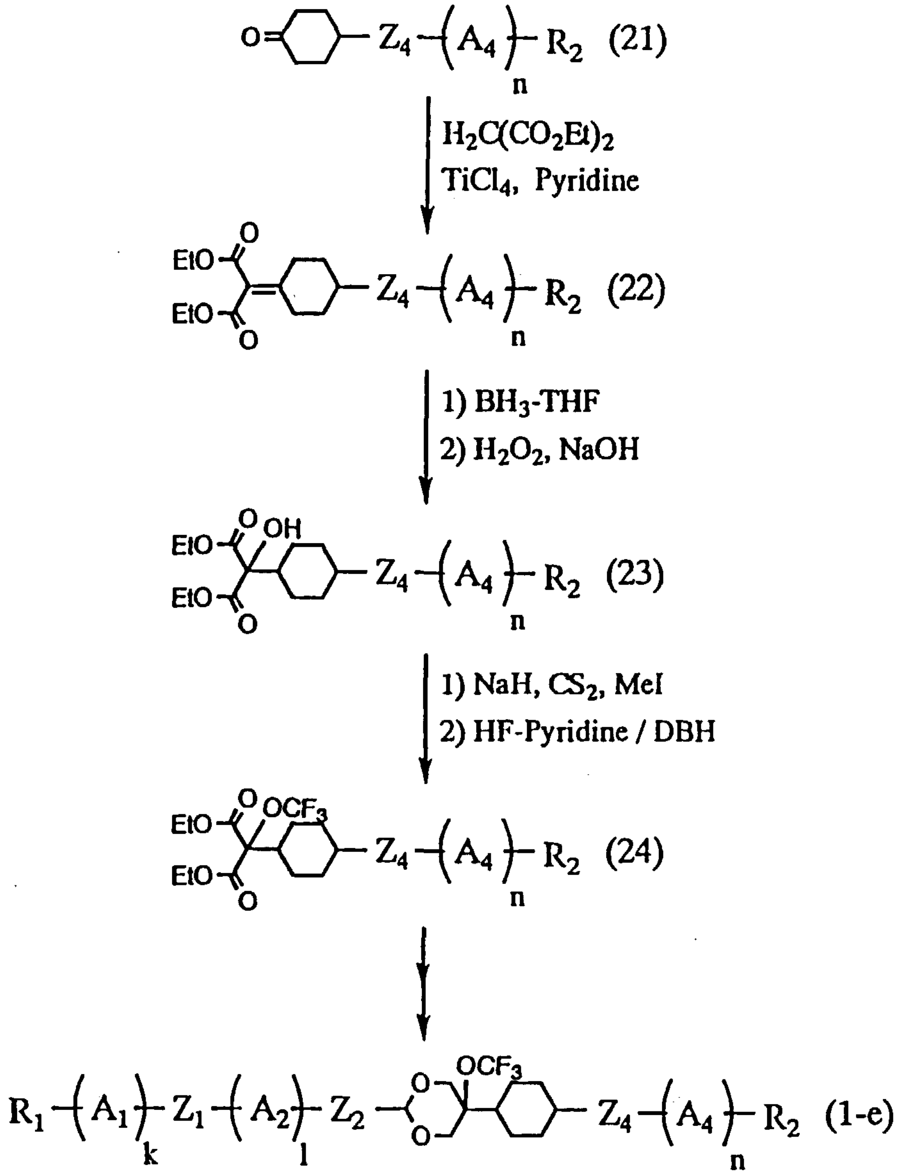


(In the above-mentioned formulae, $R_1$, $R_2$, $A_1$, $A_2$, $A_4$, $Z_1$, $Z_2$, $Z_4$, k, l and n have the same meanings as above.)

Derivatives not described above wherein $Q_1$ and $Q_2$ being oxygen atom or sulfur atom can be prepared with referring to literatures such as COMPREHENSIVE HETEROCYCLIC CHEMISTRY Vol. 3 (PERGAMON PRESS), Japanese Chemical Society ed. - Novel Experimental Chemical Course 14 (IV) etc.

Furthermore, various bonding groups may be prepared with referring to preparation methods described in Official Patent Gazettes etc. For example, compounds having 1,2-ethylene group as bonding group can be prepared according to Official Patent Gazette of Toku-Ko-Hei 3-03643 or Official Patent Gazette of Toku-Kai-Sho 59-25338. Also, compounds having 1,4-butylene group as bonding group can be prepared according to Official Patent Gazettes of Toku-Kai-Hei 3-66632, Toku-Kai-Hei 4-501575 or Toku-Kai-Hei 5-310605. Furthermore, compounds having 1,2-ethenyl group as bonding group can be prepared according to Official Patent Gazette of Toku-Kai-Sho 61-215336 or Toku-Kai-Hei 3-127748, and compounds having 1,2-ethynyl group as bonding group can be prepared according to Official Patent Gazette of Toku-Kai-Sho 61-280441 or Toku-Kai-Hei 1-502908. Furthermore, compounds having difluoromethyleneoxy group ($-CF_2O-$) can be prepared according to Official Patent Gazette of Toku-Kai-Hei 5-112778 or Toku-Kai-Hei 5-255165.

## Embodiments

The preparation methods and use examples of the compounds according to the present invention are

explained in more detail as follows, but the present invention is not limited by these examples. Therein, Cr denotes crystals, N denotes nematic phase, Sm denotes smectic phase, Iso denotes isotropic liquid, and units of phase transition temperatures are all in °C.

Example 1

Preparation of r-1-trifluoromethyl-1-(4-ethylphenyl)-cis-4-(trans-4-pentylcyclohexyl)cyclohexane (Compound No. 169) (In the general formula (1), k=m=l, l=n=0, $A_1$ being trans-1,4-cyclohexylene group, $A_3$ being 1,4-phenylene group, $R_1$=n-$C_5H_{11}$, $R_2$=$C_2H_5$, $Z_1$, $Z_2$, $Z_3$ and $Z_4$ being covalent bonds, $Q_1$ and $Q_2$ being $CH_2$ group, X being $CF_3$ group)

The preparation procedure can be roughly divided into 1) preparation of a cyclohexanol derivative, 2) preparation of r-1-bromo-1-(4-ethylphenyl)-cis-4-(trans-4-pentylcyclohexyl) cyclohexane and 3) preparation of r-1-trifluoromethyl-1-(4-ethylphenyl)-cis-4-(trans-4-pentylcyclohexyl)cyclohexane. Each preparation steps are described separatedly in detail as follows.

1) preparation of cyclohexanol derivative the first step

In a 500ml three-necked flask equipped with a stirrer, a thermometer and a cooling pipe, 1.9g (75.5mmol) of heated and activated magnesium turnings were suspended in 20ml of tetrahydrofuran (referred to THF hereinafter) and 13.3g (71.9mmol) of 4-ethylbromobenzene was added dropwise for 15 minutes with maintaining an inside temperature at below 50°C. After dropwise addition, stirring was carried out for 2 hours with maintaining 50°C on a warm bath to ripen Grignard reagent.

Then, to the above-mentioned prepared Grignard reagent, 15.0g (59.9mmol) of 4-(trans-4-pentylcyclohexyl)cyclohexanone THF solution 40ml was added dropwise for 13 minutesat the room temperature, and thereafter stirred for 2 hours with maintaining 50°C on a warm bath for aging reaction. The reaction was completed by adding dropwise 50ml of an aqueous saturated ammonium chloride solution to the reaction solution, the reaction solution was extracted with 250ml of diethyl ether, washed with water (150ml×2), and dried with anhydrous magnesium sulfate. By distilling off the solvent and concentrating, 21.1g of the reaction product was obtained. Concentrated residue was purified by silica gel column chromatography using heptane/ethyl acetate type mixed solvent as a developing solvent, to obtain 16.5g of colorless crystals. The said product is a cyclohexanol derivative.

2) Preparation of r-1-bromo-1-(4-ethylphenyl)-cis-4-(trans-4-pentylcyclohexyl)cyclohexane the second step

In a 500ml three-necked flask equipped with a stirrer, a thermometer and a cooling pipe, 16.5g (46.3mmol) of cyclohexanol derivative obtained from the above-mentioned operation and 23.0g (69.4mol) of carbon tetrabromide were dissolved in 80ml of N,N-dimethyl formamide (referred to DMF hereinafter), and 18.2g (69.4mmol) of triphenylphosphine was added dropwise for 20 minutes. After end of the dropwise addition, the reaction solution was heated to 50 °C on a warm bath and stirred for 2 hours. After the reaction solution was cooled to the room temperature, 100ml of water was added to complete the reaction. The reaction solution was extracted with 300ml of toluene, and the extracted layer was washed with water (200ml×3) and thereafter dried with anhydrous magnesium sulfate. After distilling off the solvent and concentrating, the concentrated residue was purified by silica gel column chromatography using heptane/toluene type mixed solvent as a developing solvent and recrystallized from heptane, to obtain 12.6g of colorless crystals. The said product is r-1-bromo-1-(4-ethylphenyl)-cis-4-(trans-4-pentylcyclohexyl)cyclohexane.

3) Preparation of r-1-trifluoromethyl-1-(4-ethylphenyl)-cis-4-(trans-4-pentylcyclohexyl)cyclohexane the third step

In a 500ml three-necked flask equipped with a stirrer, a thermometer, a nitrogen introducing pipe and a cooling pipe, 12.6g (30.0mmol) of r-1-bromo-1-(4-ethylphenyl)-cis-4-(trans-4-pentylcyclohexyl)cyclohexane obtained above , 28.9g (150.4mmol) of fluorosulfonyl (difluoro)aceticaid methylester and 5.7g (30.1 mmol) of cuprous iodide were dissolved in 200ml of DMF and heated with stirring at 80°C for 8 hours. The reaction solution was filtered off insolubles with Celite, and threafter filtrate was extracted with 300ml of ethyl acetate, washed subsequently with 100ml of 6 normal hydrochloric acid, water (150ml×2), 150ml of aqueous saturated sodium hydrogen carbonate solution and water (150ml×2), dried with anhydrous magnesium sulfate, distilled off the solvent under a reduced pressure, to obtain 12.3g of a reaction product as brown solid.

The obtained reaction product was purified by silica gel column chromatography using heptane solvent as a developing solvent and recrystallized from heptane, to obtain 2.1g of colorless crystals. The said product is the object, that is, r-1-trifluoromethyl-1-(4-ethylphenyl)-cis-4-(trans-4-pentylcyclohexyl)cyclohexane. The determined results of various spectra data support its construction. GC-MS(EI):$M^+$408

According to the above-mentioned preparation method, compounds having Compounds Nos. 57-70, 170-182, 253-266, 323-336, 379-392 and 435-448 can be prepared by substituting 4-(trans-4-pentylcyclohexyl)cyclohexanone and 4-ethylbromobenzene with cyclohexanone derivatives and bromobenzene derivatives having different substituents respectively.

Example 2

Preparation of r-5-fluoro-5-(trans-4-pentylcyclohexyl)-cis-2-(trans-4-propylcyclohexyl)1,3-dioxane (Compound No. 216)(In the general formula (1), k=m=l , l=n=0 , both of $A_1$ and $A_3$ being trans-1,4-cyclohexylene groups, $R_1$=n-$C_3H_7$, $R_2$=$C_5H_{11}$, $Z_1$, $Z_2$, $Z_3$ and $Z_4$ being covalent bonds, $Q_1$ and $Q_2$ being oxygen atoms, X being fluorine atom)

The preparation procedure can be roughly divided into two parts, that is, 1) preparation of dimethyl α-fluoro-α-(trans-4-pentylcyclohexyl)malonate and 2) preparation of r-5-fluoro-5-trans-4-pentylcyclohexyl)-cis-2-(trans-4-propylcyclohexyl)1,3-dioxane. Each preparation steps are described separatedly in detail as follows.

1) preparation of dimethyl α-fluoro-α-(trans-4-pentylcyclohexyl)malonate

the first step

In a 500ml three-necked flask equipped with a stirrer, a thermometer and a nitrogen introducing pipe, 2.1g (44.1mmol) of 50%-sodium hydride was suspended in 50ml of DMF at the room temperature under a nitrogen atmosphere, and a solution of 10.0g (36.8mmol) of dimethyl α-(trans-4-pentylcyclohexyl)malonate in 40ml of DMF was added dropwise for 50 minutes at the room temperature. After addition and stirring for 30 minutes, 30ml of a solution of 6.4g (110.3mmol) of dissolved potassium fluoride in DMF was added dropwise for 20 minutes and stiring for 2 hours. 100ml of water was added to the reaction solution to complete the reaction, and thereafter extracted with 300ml of ethyl acetate. The extracted layer was washed with water (150ml×4), thereafter dried with anhydrous magnesium sulfate, and then distilled off the solvent under a reduced pressure and concentrated, to obtain 10.1g of brown solid.

The obtained reaction product was purified by silica gel column chromatography using heptane-ethyl acetate type mixed solvent as a developing solvent, to obtain 6.9g (23.9mmol) of colorless crystals. The said product is dimethyl α-fluoro-α-(trans-4-pentylcyclohexyl)malonate.

2) Preparation of r-5-fluoro-5-(trans-4-pentylcyclohexyl)-cis-2-(trans-4-propylcyclohexyl)1,3-dioxane

the first step

In a 500ml three-necked flask equipped with a stirrer, a thermometer, a dropping funnel and a nitrogen introducing pipe, 1.36g (35.8mmol) of lithium aluminium hydride was suspended in 60ml of THF with manitaining below 10 °C with ice cooling, and then a 40ml of THF solution containing 6.9g (23.9mmol) of dimethyl α-fluoro-α-(trans-4-pentylcyclohexyl)malonate obtained by the above-metnioned operation was added dropwise for 20 minutes with stirring at the same temperature.

After dropwise addition, the reaction solution was stirred further for 2 hours with maintaining at 5 to 10°C. After adding 100ml of ethyl acetate and 30ml of 6 normal hydrochloric acid to complete the reaction, the reaction solution was extracted with ethyl acetate (150ml×2). The extracted layer was washed with water (150ml×2) and dried with anhydrous magnesium sulfate, distilled off the solvent under a reduced pressure, and concentrated, to obtain 5.2g of colorless solid product. The said product is 2-fluoro-2-(trans-4-pentylcyclohexyl)propylene glycol.

the second step

In a 300ml three-necked flask equipped with a stirrer, a thermometer and a Dean-Stark cooling pipe, 5.2g (21.3mmol) of 2-fluoro-2-(trans-4-pentylcyclohexyl)propylene glycol obtained above first step, 3.1g (20.2mmol) of trans-4-propylcyclohexyl carbaldehyde and 0.3g (1.7mmol) of p-toluene sulfonic acid were dissolved in 100ml of toluene and heated under reflux for 3 hours with dehydrating. After cooling to the room temperature, 100ml of water was added and extracted with ethyl acetate (100ml ×2).

The extracted layer was washed subsequently with 50ml of aqueous saturated sodium carbonate solution and water (100ml× 2), threafter dried with anhydrous magnesium sulfate, distilled off the solvent under a reduced pressure and concetrated, to obtain 7.9g of yellowish brown solid. The reaction product was purified by silica gel column chromatography using toluene/ethyl acetate type mixed solvent as a developing solvent and recrystallized from mixed solvent of heptane-ethanol, to obtain 2.1g of colorless crystals.

The said product is the objective r-5-fluoro-5-(trans-4-pentylcyclohexyl)-cis-2-(trans-4-propylcyclohexyl)1,3-dioxane. The determined results of various spectra data support its construction.
GC-MS(EI):$M^{+}$382

According to the above-mentioned preparation method, compounds having Compounds No. 155-168, 211-215, 217-238, 281-294, 351-364, 407-420 and 463-476 can be prepared by substituting dimethyl α-(trans-4-pentylcyclohexyl)malonate and trans-4-propylcyclohexyl carbaldehyde with various malonic ester derivatives and aldehyde derivatives respectively.

Example 3

Preparation of r-5-trifluoromethyl-5-(4-pentylphenyl)-cis-2-(trans-4-propylcyclohexyl)1,3-dioxane (Compound No. 202) (In the general formula (1) , k=m=l , l=n=0 , $A_1$ being 1,4-cyclohexylene group, $A_3$ being 1,4-phenylene group, $R_1$=n-$C_3H_7$, $R_2$=$C_5H_{11}$, $Z_1$, $Z_2$, $Z_3$ and $Z_4$ being covalent bonds, $Q_1$ and $Q_2$ being oxygen atoms, X being trifluoromethyl group)

The preparation procedure can be roughly divided into two parts, that is, 1) preparation of dimethyl α-trifluoromethyl-α-(4-pentylphenyl)malonate and 2) preparation of r-5-trifluoromethyl-5-(4-pentylphenyl)-cis-2-(trans-4- propylcyclohexyl)1,3-dioxane. Each preparation steps are described separatedly in detail as follows.

1) preparation of dimethyl α-trifluoromethyl- α-(4-pentylphenyl) malonate

the first step

In a 500ml three-necked flask equipped with a stirrer, a thermometer and a nitrogen introducing pipe, 2.0g (43.1mmol) of 50%-sodium hydride was suspended in 50ml of DMF with stirring at the room temperature under a nitrogen atmosphere, and a solution of 10.0g (35.9mmol) of dimethyl α-(4-pentylphenyl)malonate in 50ml of DMF was added dropwise for 1 hour at the room temperature. After adding and stirring for 30 minutes, a solution of 4.4g (43.1mmol) of sodium bromide dissolved in 20ml of DMF was added dropwise for 15 minutes at the room temperature and stirred at the same temperature further for 2 hours.
100ml of water was added to the reaction solution to complete the reaction, and thereafter extracted with 300ml of ethyl acetate. The extracted layer was washed with water (150ml×4), thereafter dried with anhydrous magnesium sulfate, and then distilled off the solvent under a reduced pressure and concentrated, to obtain 12.5g of brown solid. The obtained reaction product was purified by silica gel column chromatography using toluene-ethyl acetate type mixed solvent as a developing solvent, to obtain 8.8g of colorless crystals. The said product is dimethyl α-bromo- α-(4-pentylphenyl)malonate.

the second step

In a 500ml three-necked flask equipped with a stirrer, a thermometer, a nitrogen introducing pipe and a cooling pipe, 8.8g (24.8mmol) of dimethyl α-bromo- α-(4-pentylphenyl) malonate obtained in the above-mentioned first step, 14.3g (74.4 mmol) of fluorosulfonyl (difluoro) aceticacid methylester and 2.1g (11.2mmol) of cuprous iodide were dissolved in 100ml of DMF, and heated at 80°C with stirring for 8 hours. The reaction solution was filtered off insolubles with Celite, threafter the filtrate was extracted with 200ml of ethyl acetate, washed subsequently with 50ml of 6 normal hydrochloric acid, water (100ml×2), 50ml of aqueous saturated sodium hydrogen carbonate and water (100ml × 2), dried with anhydrous magnesium sulfate, and distilled off the solvent under a reduced pressure, to obtain 8.6g of colorless product as brown solid.
The obtained reaction product was purified by silica gel column chromatography using touluene-ethyl acetate type mixed solvent as a developing solvent, to obtain 4.2g of colorless crystals. The said product is dimethyl α-trifluoromethyl- α-(4-pentylphenyl)malonate.

2) preparation of r-5-trifluoromethyl-5-(4-pentylphenyl)-cis-2-(trans-4-propylcyclohexyl)1,3-dioxane.

the third step

In a 500ml three-necked flask equipped with a stirrer, a thermometer, a dropping funnel and a nitrogen introducing pipe, 0.6g (15.8mmol) of lithium aluminium hydride was suspended in 20ml of THF with maintaining below 10°C under ice cooling, and a 20ml of THF solution of 4.2g (12.1mmol) of dimethyl α-trifluoromethyl-α-(4-pentylphenyl)malonate obtained by the above-mentioned operation was added dropwise at the same temperature for 8 minutes

with stirring. After dropwise addition, the solution was stirred further for 2 hours with maintaining 5 to 10°C.

50ml of ethyl acetate and 20ml of 6 normal hydrochloric acid were added dropwise to the reaction solution to complete the reaction, and thereafter the reaction solution was extracted with ethyl acetate (100ml×2). The extracted layer was washed with water (50ml×2), then dried with anhydrous magnesium sulfate, distilled off the solvent under a reduced pressure and concentrated, to obtain 3.2g of colorless solid product. The said product is 2-trifluoromethyl-2-(4-pentylphenyl)propylene glycol.

the fourth step

In a 300ml three-necked flask equipped with a stirrer, a thermometer and a Dean-Stark cooling pipe, 3.2 g (11.1mmol) of 2-trifluoromethyl-2-(4-pentylphenyl)propylene glycol obtained in the above-mentioned third step, 2.0g (12.9mmol) of trans-4-propylcyclohexyl carbaldehyde and 0.2g (1.5mmol) of p-toluene sulfonic acid were dissolved in 100ml of toluene and heated under reflux for 3 hours with dehydrating. After cooling to the room temperature, 100ml of water was added and extracted with ethyl acetate (100ml×2).

The extracted layer was washed subsequently with 50ml of aqueous saturated sodium carbonate solution and water (100ml×2), thereafter dried with anhydrous magnesium sulfate, distilled off the solvent under a reduced pressure and concetrated, to obtain 4.6g of yellowish brown solid. The reaction product was purified by silica gel column chromatography using toluene/ethyl acetate type mixed solvent as a developing solvent and recrystallized from mixed solvent of heptane-ethanol, to obtain 1.5g of colorless crystals. The said product is the object, that is, r-5-trifluoromethyl-5-(4-pentylphenyl)-cis-2-(trans-4-propylcyclohexyl)1,3-dioxane. The determined results of various spectra data support its construction.

GC-MS(EI):$M^+$426

According to the above-mentioned preparation method, compounds having Compounds No. 99-140, 197-201, 203-210, 295-308, 365-378, 421-434 and 477-490 can be prepared by substituting dimethyl α-(4-pentylphenyl)malonate and trans-4-propylcyclohexyl carbaldehyde with various malonic ester derivatives and aldehyde derivatives respectively.

Example 4

According to the above-mentioned Examples 1-3 or by selecting and combining various reactions described in Open-laid Patent Official Gazettes or organic synthetic literatures, the compounds (Compounds Nos. 1-574) shown below can be prepared.

| Compound No. | $R_1-$ | $-(A_1)_k-Z_1-(A_2)_l-Z_2-$ | 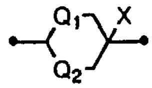 | $-Z_3-(A_3)_m-Z_4-(A_4)_n-$ | $-R_2$ |
|---|---|---|---|---|---|
| 1 | $C_2H_5-$ | 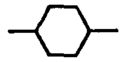 | 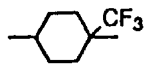 | | $-C_2H_5$ |
| 2 | $C_3H_7-$ | 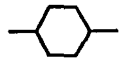 | 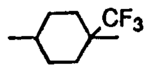 | | $-C_2H_5$ |
| 3 | $C_5H_{11}-$ | 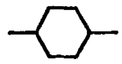 | 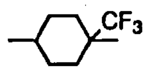 | | $-C_2H_5$ |
| 4 | $C_3H_7-$ | 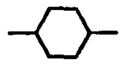 | 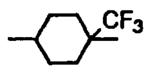 | | $-C_3H_7$ |
| 5 | $C_3H_7-$ | 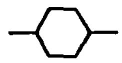 | 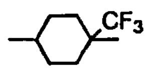 | | $-C_5H_{11}$ |
| 6 | $C_5H_{11}-$ | 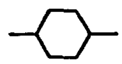 | 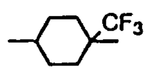 | | $-C_5H_{11}$ |
| 7 | 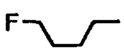 | 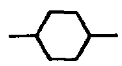 | 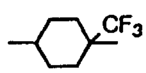 | | $-C_2H_5$ |
| 8 | 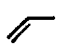 | 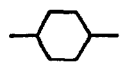 | 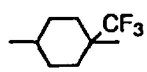 | | $-C_3H_7$ |
| 9 | 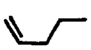 | 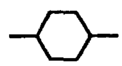 | 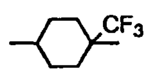 | | $-C_2H_5$ |
| 10 | 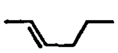 | 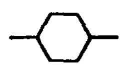 | 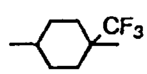 | | $-C_2H_5$ |
| 11 | $C_3H_7O-$ | 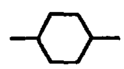 | 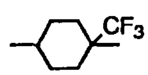 | | $-C_2H_5$ |
| 12 | 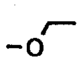 | 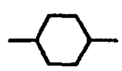 | 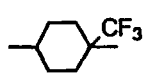 | | $-C_2H_5$ |
| 13 | $C_3H_7-$ | 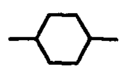 | 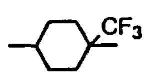 | | $-OCF_2CFHCF_3$ |
| 14 | $C_3H_7-$ | 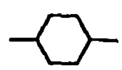 | 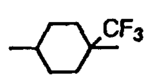 | | $-OCF_2CF_2Cl$ |

| Compound No. | R1—• | •(A1)k Z1 (A2)l Z2—• | 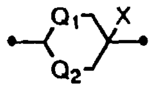 | •—Z3 (A3)m Z4 (A4)n• | •—R2 |
|---|---|---|---|---|---|
| 15 | $C_2H_5$— | 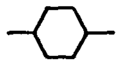 | 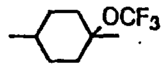 | | $-C_2H_5$ |
| 16 | $C_3H_7$— | 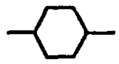 | 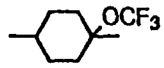 | | $-C_2H_5$ |
| 17 | $C_5H_{11}$— | 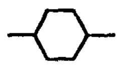 | 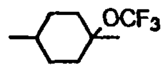 | | $-C_2H_5$ |
| 18 | $C_3H_7$— | 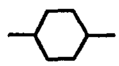 | 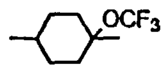 | | $-C_3H_7$ |
| 19 | $C_3H_7$— | 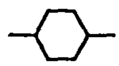 | 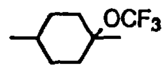 | | $-C_5H_{11}$ |
| 20 | $C_5H_{11}$— | 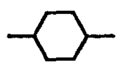 | 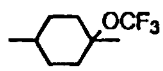 | | $-C_5H_{11}$ |
| 21 | 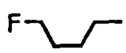 | 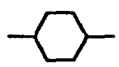 | 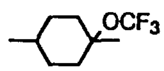 | | $-C_2H_5$ |
| 22 | 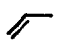 | 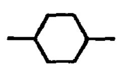 | 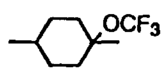 | | $-C_3H_7$ |
| 23 | 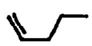 | 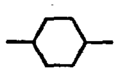 | 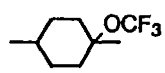 | | $-C_2H_5$ |
| 24 | 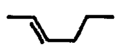 | 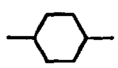 | 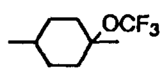 | | $-C_2H_5$ |
| 25 | $C_3H_7O$— | 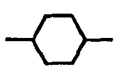 | 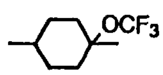 | | $-C_2H_5$ |
| 26 | 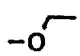 | 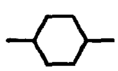 | 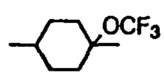 | | $-C_2H_5$ |
| 27 | $C_3H_7$— | 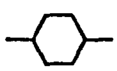 | 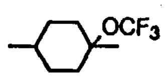 | | $-OCF_2CFHCF_3$ |
| 28 | $C_3H_7$— | 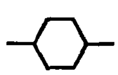 | 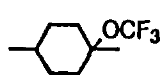 | | $-OCF_2CF_2Cl$ |

| Compound No. | $R_1$—• | •$-(A_1)_k-Z_1-(A_2)_l-Z_2-$• | 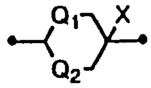 | •$-Z_3-(A_3)_m-Z_4-(A_4)_n-$• | •$-R_2$ |
|---|---|---|---|---|---|
| 29 | $C_2H_5-$ | | 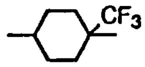 | 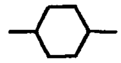 | $-C_2H_5$ |
| 30 | $C_3H_7-$ | | 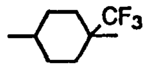 | 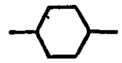 | $-C_2H_5$ |
| 31 | $C_5H_{11}-$ | | 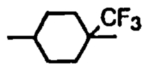 | 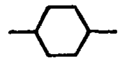 | $-C_2H_5$ |
| 32 | $C_3H_7-$ | | 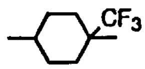 | 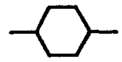 | $-C_3H_7$ |
| 33 | $C_3H_7-$ | | 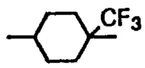 | 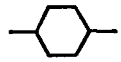 | $-C_5H_{11}$ |
| 34 | $C_5H_{11}-$ | | 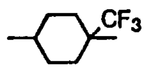 | 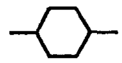 | $-C_5H_{11}$ |
| 35 | 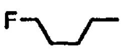 | | 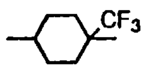 | 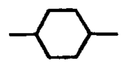 | $-C_2H_5$ |
| 36 | 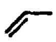 | | 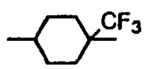 | 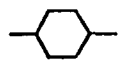 | $-C_3H_7$ |
| 37 | 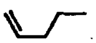 | | 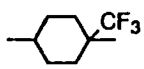 | 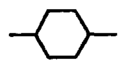 | $-C_2H_5$ |
| 38 | 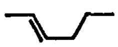 | | 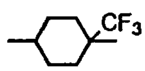 | 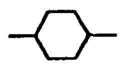 | $-C_2H_5$ |
| 39 | $C_3H_7O-$ | | 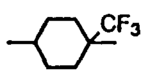 | 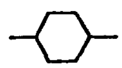 | $-C_2H_5$ |
| 40 | 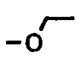 | | 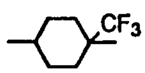 | 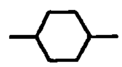 | $-C_2H_5$ |
| 41 | $C_3H_7-$ | | 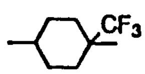 | 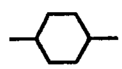 | $-OCF_2CFHCF_3$ |
| 42 | $C_3H_7-$ | | 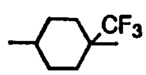 | 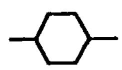 | $-OCF_2CF_2Cl$ |

| Compound No. | $R_1$— | 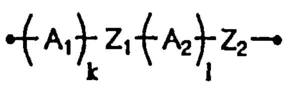 | 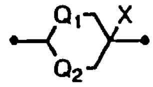 | 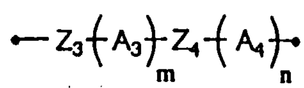 | —$R_2$ |
|---|---|---|---|---|---|
| 43 | $C_2H_5$— | | 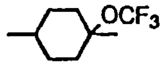 | 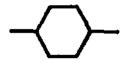 | —$C_2H_5$ |
| 44 | $C_3H_7$— | | 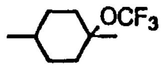 | 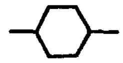 | —$C_2H_5$ |
| 45 | $C_5H_{11}$— | | 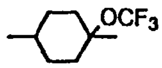 | 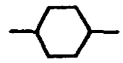 | —$C_2H_5$ |
| 46 | $C_3H_7$— | | 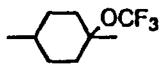 | 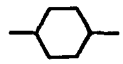 | —$C_3H_7$ |
| 47 | $C_3H_7$— | | 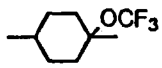 | 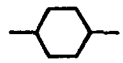 | —$C_5H_{11}$ |
| 48 | $C_5H_{11}$— | | 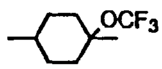 | 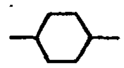 | —$C_5H_{11}$ |
| 49 | 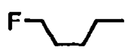 | | 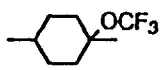 | 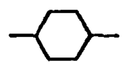 | —$C_2H_5$ |
| 50 | 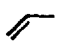 | | 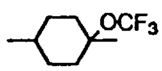 | 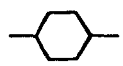 | —$C_3H_7$ |
| 51 | 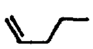 | | 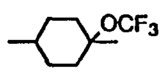 | 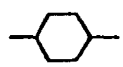 | —$C_2H_5$ |
| 52 | 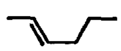 | | 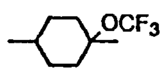 | 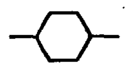 | —$C_2H_5$ |
| 53 | $C_3H_7O$— | | 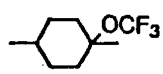 | 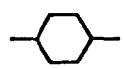 | —$C_2H_5$ |
| 54 | 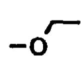 | | 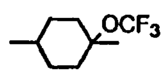 | 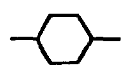 | —$C_2H_5$ |
| 55 | $C_3H_7$— | | 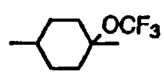 | 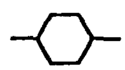 | —$OCF_2CFHCF_3$ |
| 56 | $C_3H_7$— | | 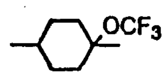 | 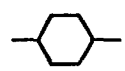 | —$OCF_2CF_2Cl$ |

| Compound No. | $R_1$— | $-(A_1)_k-Z_1-(A_2)_l-Z_2-$ | 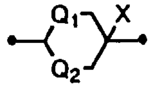 | $-Z_3-(A_3)_m-Z_4-(A_4)_n-$ | $-R_2$ |
|---|---|---|---|---|---|
| 57 | $C_2H_5$— | | 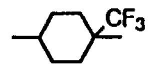 | 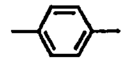 | $-C_2H_5$ |
| 58 | $C_3H_7$— | | 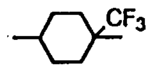 | 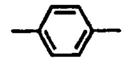 | $-C_2H_5$ |
| 59 | $C_5H_{11}$— | | 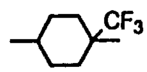 | 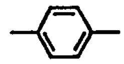 | $-C_2H_5$ |
| 60 | $C_3H_7$— | | 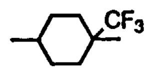 | 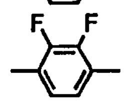 | $-C_3H_7$ |
| 61 | $C_3H_7$— | | 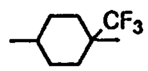 | 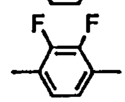 | $-OC_5H_{11}$ |
| 62 | $C_5H_{11}$— | | 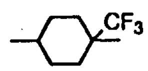 | 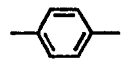 | $-C_5H_{11}$ |
| 63 | 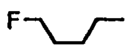 | | 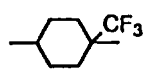 | 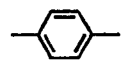 | $-C_2H_5$ |
| 64 | 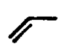 | | 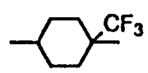 | 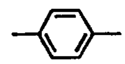 | $-C_3H_7$ |
| 65 | 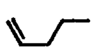 | | 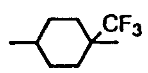 | 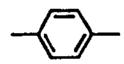 | $-C_2H_5$ |
| 66 | 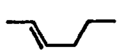 | | 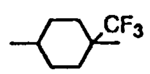 | 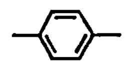 | $-C_2H_5$ |
| 67 | $C_3H_7O$— | | 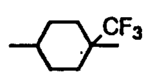 | 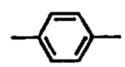 | $-C_2H_5$ |
| 68 | 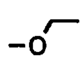 | | 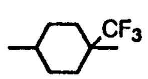 | 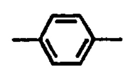 | $-C_2H_5$ |
| 69 | $C_3H_7$— | | 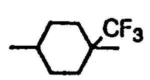 | 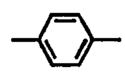 | $-OCF_2CFHCF_3$ |
| 70 | $C_3H_7$— | | 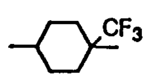 | 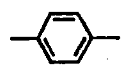 | $-OCF_2CF_2Cl$ |

| Compound No. | $R_1$—• | •$-(A_1)_k-Z_1-(A_2)_l-Z_2$—• | 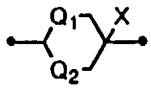 | •—$Z_3-(A_3)_m-Z_4-(A_4)_n$• | •—$R_2$ |
|---|---|---|---|---|---|
| 71 | $C_2H_5$— | | 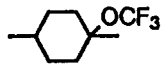 | 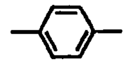 | $-C_2H_5$ |
| 72 | $C_3H_7$— | | 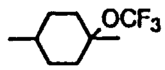 | 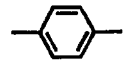 | $-C_2H_5$ |
| 73 | $C_5H_{11}$— | | 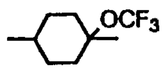 | 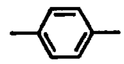 | $-C_2H_5$ |
| 74 | $C_3H_7$— | | 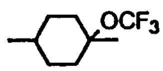 | 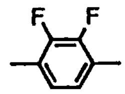 | $-C_3H_7$ |
| 75 | $C_3H_7$— | | 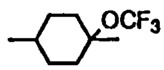 | 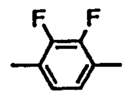 | $-OC_5H_{11}$ |
| 76 | $C_5H_{11}$— | | 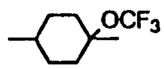 | 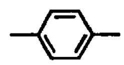 | $-C_5H_{11}$ |
| 77 | 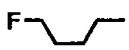 | | 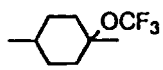 | 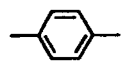 | $-C_2H_5$ |
| 78 | 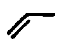 | | 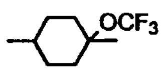 | 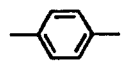 | $-C_3H_7$ |
| 79 | 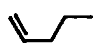 | | 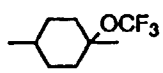 | 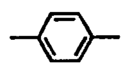 | $-C_2H_5$ |
| 80 | 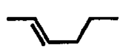 | | 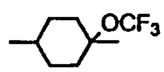 | 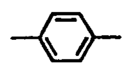 | $-C_2H_5$ |
| 81 | $C_3H_7O$— | | 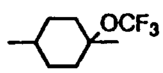 | 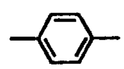 | $-C_2H_5$ |
| 82 | 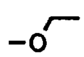 | | 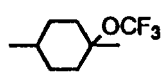 | 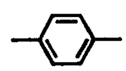 | $-C_2H_5$ |
| 83 | $C_3H_7$— | | 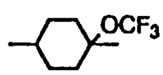 | 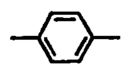 | $-OCF_2CFHCF_3$ |
| 84 | $C_3H_7$— | | 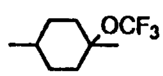 | 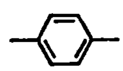 | $-OCF_2CF_2Cl$ |

| Compound No. | $R_1-$ | $-(A_1)_k-Z_1-(A_2)_l-Z_2-$ | 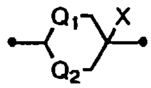 | $-Z_3-(A_3)_m-Z_4-(A_4)_n-$ | $-R_2$ |
|---|---|---|---|---|---|
| 85 | $C_2H_5-$ | 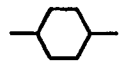 | 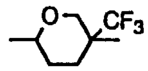 | | $-C_2H_5$ |
| 86 | $C_3H_7-$ | 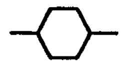 | 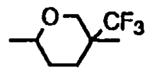 | | $-C_2H_5$ |
| 87 | $C_5H_{11}-$ | 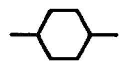 | 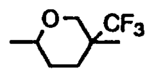 | | $-C_2H_5$ |
| 88 | $C_3H_7-$ | 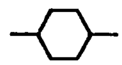 | 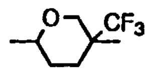 | | $-C_3H_7$ |
| 89 | $C_3H_7-$ | 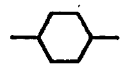 | 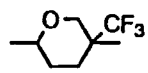 | | $-C_5H_{11}$ |
| 90 | $C_5H_{11}-$ | 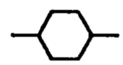 | 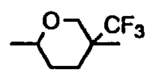 | | $-C_5H_{11}$ |
| 91 | 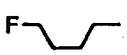 | 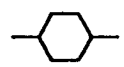 | 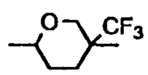 | | $-C_2H_5$ |
| 92 | 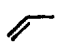 | 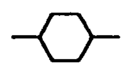 | 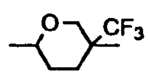 | | $-C_3H_7$ |
| 93 | 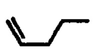 | 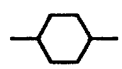 | 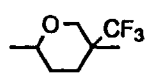 | | $-C_2H_5$ |
| 94 | 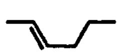 | 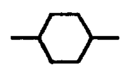 | 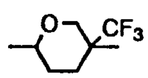 | | $-C_2H_5$ |
| 95 | $C_3H_7O-$ | 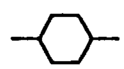 | 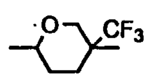 | | $-C_2H_5$ |
| 96 | 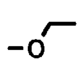 | 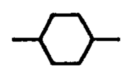 | 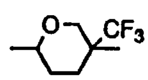 | | $-C_2H_5$ |
| 97 | $C_3H_7-$ | 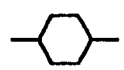 | 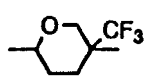 | | $-OCF_2CFHCF_3$ |
| 98 | $C_3H_7-$ | 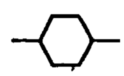 | 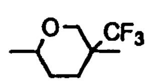 | | $-OCF_2CF_2Cl$ |

| Compound No. | $R_1$—• | •$-(A_1)_k-Z_1-(A_2)_l-Z_2-$• | 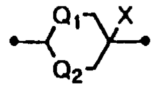 | •$-Z_3-(A_3)_m-Z_4-(A_4)_n-$• | •$-R_2$ |
|---|---|---|---|---|---|
| 99 | $C_2H_5-$ | 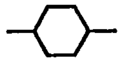 | 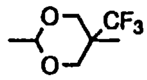 | | $-C_2H_5$ |
| 100 | $C_3H_7-$ | 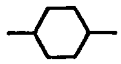 | 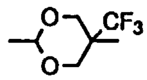 | | $-C_2H_5$ |
| 101 | $C_5H_{11}-$ | 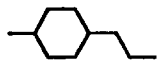 | 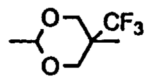 | | $-C_2H_5$ |
| 102 | $C_3H_7-$ | 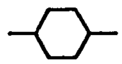 | 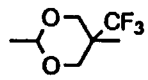 | | $-C_3H_7$ |
| 103 | $C_3H_7-$ | 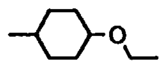 | 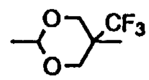 | | $-C_5H_{11}$ |
| 104 | $C_5H_{11}-$ | 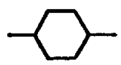 | 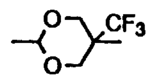 | | $-C_5H_{11}$ |
| 105 | 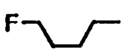 | 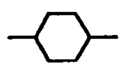 | 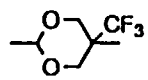 | | $-C_2H_5$ |
| 106 | 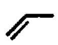 | 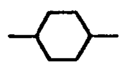 | 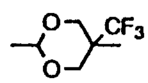 | | $-C_3H_7$ |
| 107 | 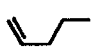 | 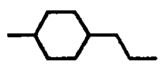 | 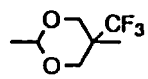 | | $-C_2H_5$ |
| 108 | 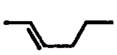 | 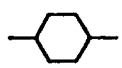 | 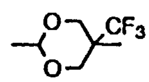 | | $-C_2H_5$ |
| 109 | $C_3H_7O-$ | 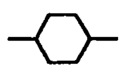 | 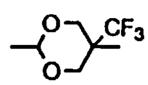 | | $-C_2H_5$ |
| 110 | 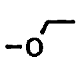 | 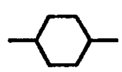 | 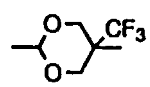 | | $-C_2H_5$ |
| 111 | $C_3H_7-$ | 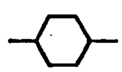 | 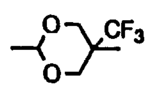 | | $-OCF_2CFHCF_3$ |
| 112 | $C_3H_7-$ | 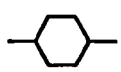 | 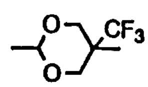 | | $-OCF_2CF_2Cl$ |

| Compound No. | 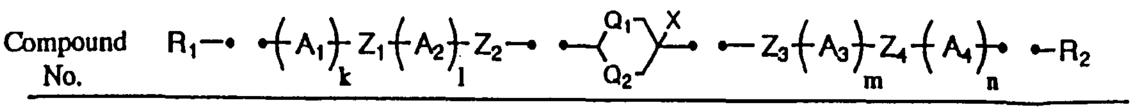 | | | |
|---|---|---|---|---|
| 113 | $C_2H_5-$ | 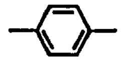 | 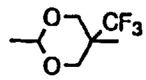 | $-C_2H_5$ |
| 114 | $C_3H_7-$ | 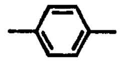 | 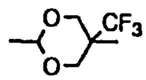 | $-C_2H_5$ |
| 115 | $C_5H_{11}-$ | 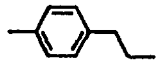 | 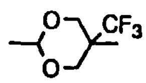 | $-C_2H_5$ |
| 116 | $C_3H_7-$ | 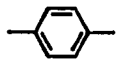 | 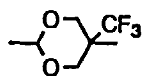 | $-C_3H_7$ |
| 117 | $C_3H_7-$ | 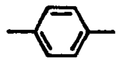 | 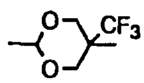 | $-C_5H_{11}$ |
| 118 | $C_5H_{11}-$ | 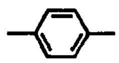 | 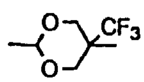 | $-C_5H_{11}$ |
| 119 | 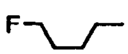 | 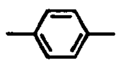 | 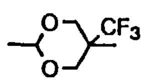 | $-C_2H_5$ |
| 120 | 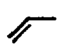 | 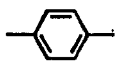 | 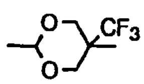 | $-C_3H_7$ |
| 121 | 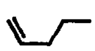 | 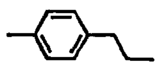 | 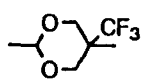 | $-C_2H_5$ |
| 122 | 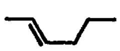 | 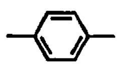 | 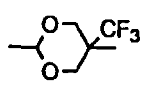 | $-C_2H_5$ |
| 123 | $C_3H_7O-$ | 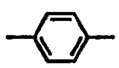 | 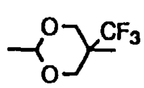 | $-C_2H_5$ |
| 124 | 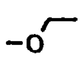 | 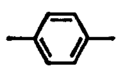 | 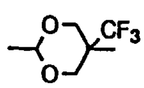 | $-C_2H_5$ |
| 125 | $C_3H_7-$ | 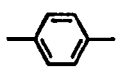 | 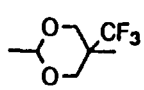 | $-OCF_2CFHCF_3$ |
| 126 | $C_3H_7-$ | 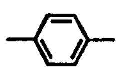 | 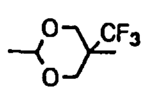 | $-OCF_2CF_2Cl$ |

| Compound No. | $R_1$—• | •$-(A_1)_k-Z_1-(A_2)_l-Z_2$—• | 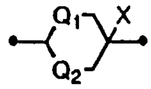 | •—$Z_3-(A_3)_m-Z_4-(A_4)_n$-• | •—$R_2$ |
|---|---|---|---|---|---|
| 127 | $C_2H_5$— | | 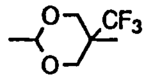 | 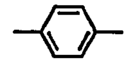 | $-C_2H_5$ |
| 128 | $C_3H_7$— | | 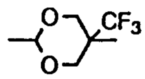 | 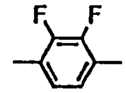 | $-C_2H_5$ |
| 129 | $C_5H_{11}$— | | 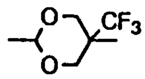 | 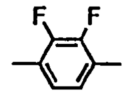 | $-OC_2H_5$ |
| 130 | $C_3H_7$— | | 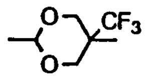 | 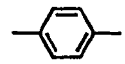 | $-C_3H_7$ |
| 131 | $C_3H_7$— | | 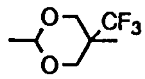 | 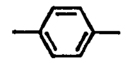 | $-C_5H_{11}$ |
| 132 | $C_5H_{11}$— | | 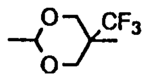 | 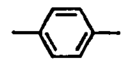 | $-C_5H_{11}$ |
| 133 | 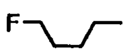 | | 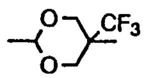 | 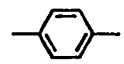 | $-C_2H_5$ |
| 134 | 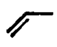 | | 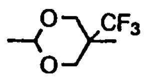 | 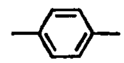 | $-C_3H_7$ |
| 135 | 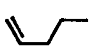 | | 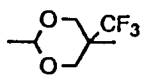 | 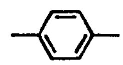 | $-C_2H_5$ |
| 136 | 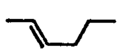 | | 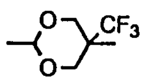 | 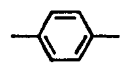 | $-C_2H_5$ |
| 137 | $C_3H_7O$— | | 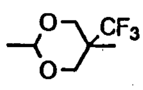 | 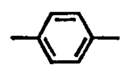 | $-C_2H_5$ |
| 138 | 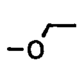 | | 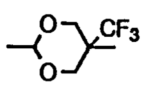 | 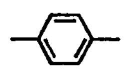 | $-C_2H_5$ |
| 139 | $C_3H_7$— | | 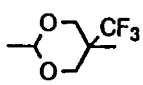 | 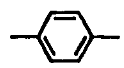 | $-OCF_2CFHCF_3$ |
| 140 | $C_3H_7$— | | 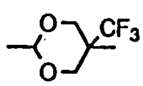 | 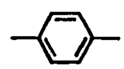 | $-OCF_2CF_2Cl$ |

| Compound No. | $R_1$—• | •$(A_1)_k$ $Z_1$ $(A_2)_l$ $Z_2$—• | 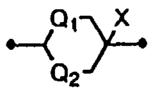 | •—$Z_3$ $(A_3)_m$ $Z_4$ $(A_4)_n$• | •—$R_2$ |
|---|---|---|---|---|---|
| 141 | $C_2H_5$— | | 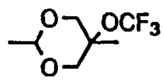 | 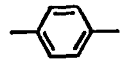 | $-C_2H_5$ |
| 142 | $C_3H_7$— | | 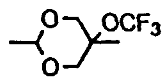 | 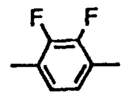 | $-C_2H_5$ |
| 143 | $C_5H_{11}$— | | 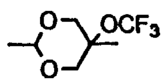 | 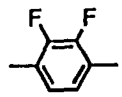 | $-OC_2H_5$ |
| 144 | $C_3H_7$— | | 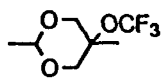 | 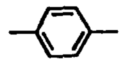 | $-C_3H_7$ |
| 145 | $C_3H_7$— | | 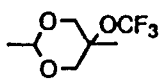 | 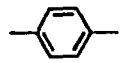 | $-C_5H_{11}$ |
| 146 | $C_5H_{11}$— | | 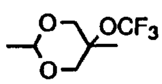 | 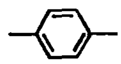 | $-C_5H_{11}$ |
| 147 | 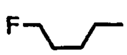 | | 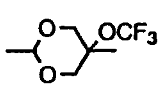 | 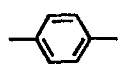 | $-C_2H_5$ |
| 148 | 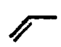 | | 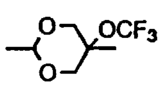 | 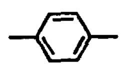 | $-C_3H_7$ |
| 149 | 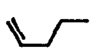 | | 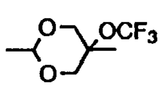 | 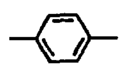 | $-C_2H_5$ |
| 150 | 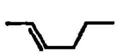 | | 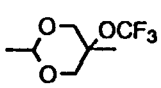 | 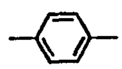 | $-C_2H_5$ |
| 151 | $C_3H_7O$— | | 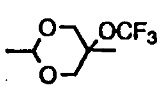 | 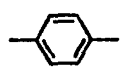 | $-C_2H_5$ |
| 152 | 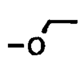 | | 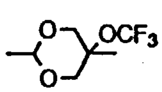 | 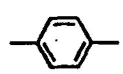 | $-C_2H_5$ |
| 153 | $C_3H_7$— | | 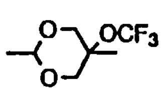 | 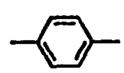 | $-OCF_2CFHCF_3$ |
| 154 | $C_3H_7$— | | 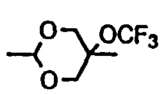 | 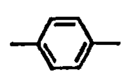 | $-OCF_2CF_2Cl$ |

| Compound No. | 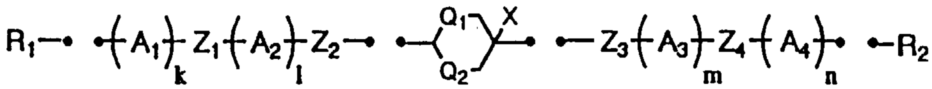 | | | | |
|---|---|---|---|---|---|
| 155 | $C_2H_5$— | | 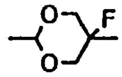 | 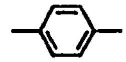 | $-C_2H_5$ |
| 156 | $C_3H_7$— | | 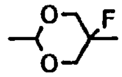 | 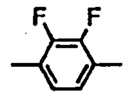 | $-C_2H_5$ |
| 157 | $C_5H_{11}$— | | 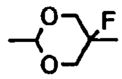 | 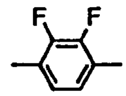 | $-OC_2H_5$ |
| 158 | $C_3H_7$— | | 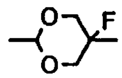 | 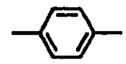 | $-C_3H_7$ |
| 159 | $C_3H_7$— | | 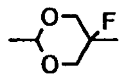 | 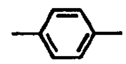 | $-C_5H_{11}$ |
| 160 | $C_5H_{11}$— | | 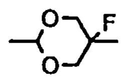 | 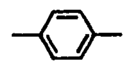 | $-C_2H_5$ |
| 161 | 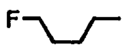 | | 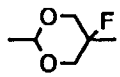 | 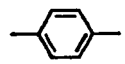 | $-C_2H_5$ |
| 162 | 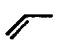 | | 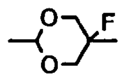 | 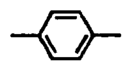 | $-C_3H_7$ |
| 163 | 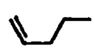 | | 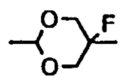 | 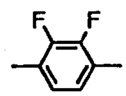 | $-C_2H_5$ |
| 164 | 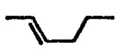 | | 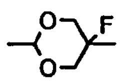 | 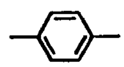 | $-C_2H_5$ |
| 165 | $C_3H_7O$— | | 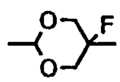 | 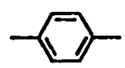 | $-C_2H_5$ |
| 166 | 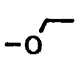 | | 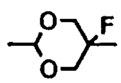 | 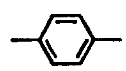 | $-C_2H_5$ |
| 167 | $C_3H_7$— | | 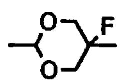 | 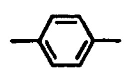 | $-OCF_2CFHCF_3$ |
| 168 | $C_3H_7$— | | 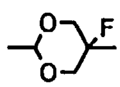 | 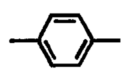 | $-OCF_2CF_2Cl$ |

| Compound No. | R₁ | 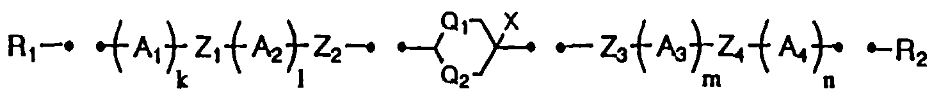 | R₂ |
|---|---|---|---|
| 169 | $C_5H_{11}-$ | 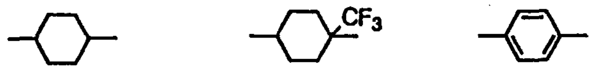 | $-C_2H_5$ |
| 170 | $C_3H_7-$ | 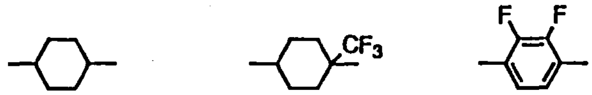 | $-C_2H_5$ |
| 171 | $C_5H_{11}-$ | 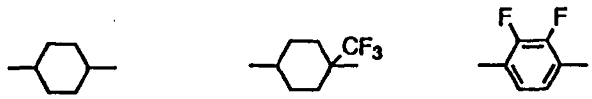 | $-OC_2H_5$ |
| 172 | $C_3H_7-$ | 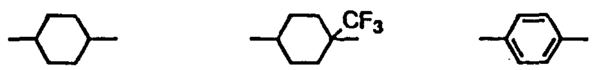 | $-C_3H_7$ |
| 173 | $C_3H_7-$ | 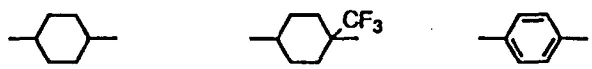 | $-C_2H_5$ |
| 174 | $C_3H_7-$ | 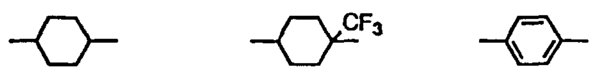 | $-C_5H_{11}$ |
| 175 | 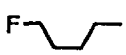 | 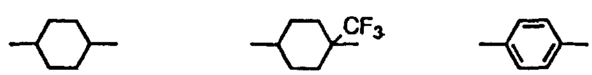 | $-C_2H_5$ |
| 176 | 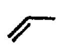 | 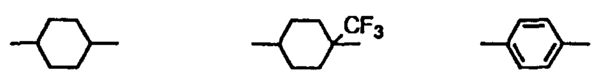 | $-C_3H_7$ |
| 177 | 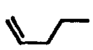 | 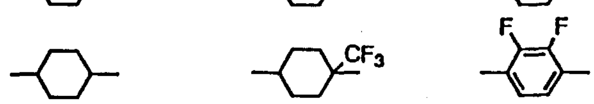 | $-C_2H_5$ |
| 178 | 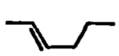 | 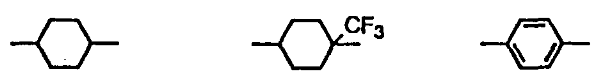 | $-C_2H_5$ |
| 179 | $C_3H_7O-$ | 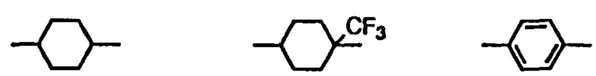 | $-C_2H_5$ |
| 180 | 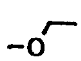 | 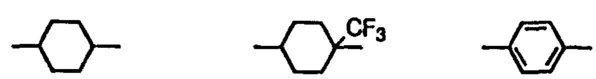 | $-C_2H_5$ |
| 181 | $C_3H_7-$ | 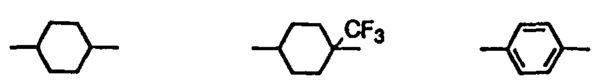 | $-OCF_2CFHCF_3$ |
| 182 | $C_3H_7-$ | 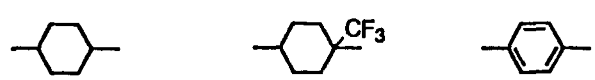 | $-OCF_2CF_2Cl$ |

| Compound No. | $R_1$—• | •$\{A_1\}_k$ $Z_1$ $\{A_2\}_l$ $Z_2$—• | 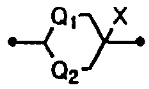 | •— $Z_3$ $\{A_3\}_m$ $Z_4$ $\{A_4\}_n$• | •—$R_2$ |
|---|---|---|---|---|---|
| 183 | $C_5H_{11}$— | 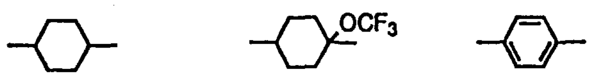 | | | $-C_2H_5$ |
| 184 | $C_3H_7$— | 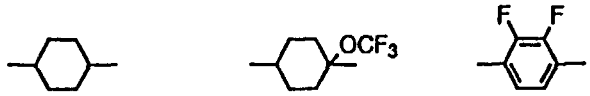 | | | $-C_2H_5$ |
| 185 | $C_5H_{11}$— | 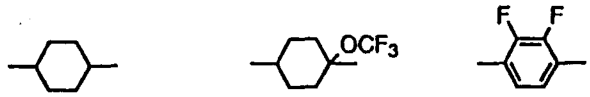 | | | $-OC_2H_5$ |
| 186 | $C_3H_7$— | 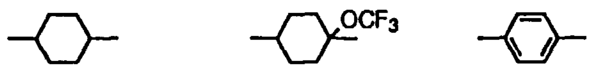 | | | $-C_3H_7$ |
| 187 | $C_3H_7$— | 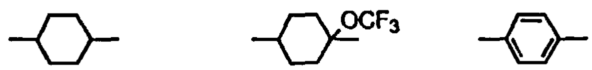 | | | $-C_2H_5$ |
| 188 | $C_3H_7$— | 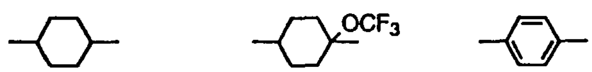 | | | $-C_5H_{11}$ |
| 189 | 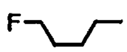 | 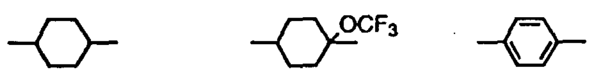 | | | $-C_2H_5$ |
| 190 | 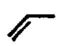 | 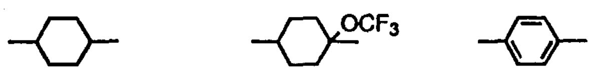 | | | $-C_3H_7$ |
| 191 | 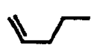 | 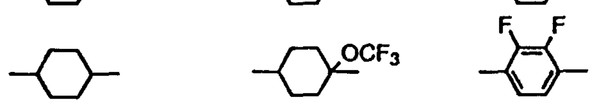 | | | $-C_2H_5$ |
| 192 | 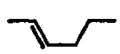 | 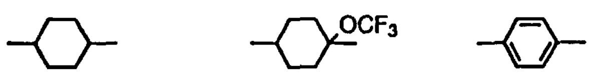 | | | $-C_2H_5$ |
| 193 | $C_3H_7O$— | 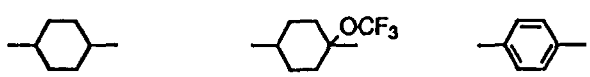 | | | $-C_2H_5$ |
| 194 | 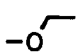 | 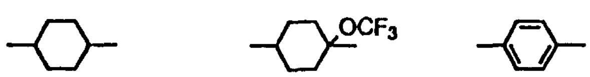 | | | $-C_2H_5$ |
| 195 | $C_3H_7$— | 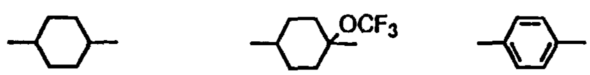 | | | $-OCF_2CFHCF_3$ |
| 196 | $C_3H_7$— | 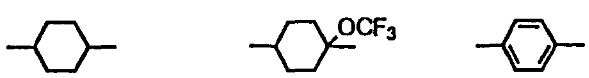 | | | $-OCF_2CF_2Cl$ |

| Compound No. | $R_1$—• | •$(A_1)_k$-$Z_1$-$(A_2)_l$-$Z_2$—• | 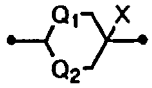 | •—$Z_3$-$(A_3)_m$-$Z_4$-$(A_4)_n$• | •—$R_2$ |
|---|---|---|---|---|---|
| 197 | $C_5H_{11}$— | 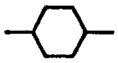 | 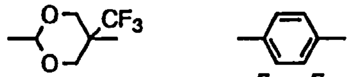 | | $-C_2H_5$ |
| 198 | $C_3H_7$— | 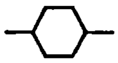 | 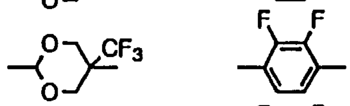 | | $-C_2H_5$ |
| 199 | $C_5H_{11}$— | 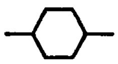 | 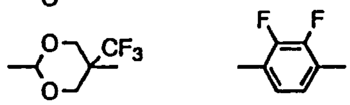 | | $-OC_2H_5$ |
| 200 | $C_3H_7$— | 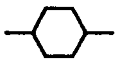 | 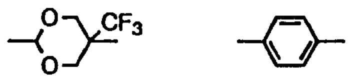 | | $-C_3H_7$ |
| 201 | $C_3H_7$— | 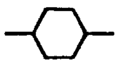 | 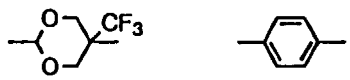 | | $-C_2H_5$ |
| 202 | $C_3H_7$— | 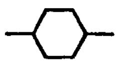 | 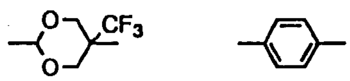 | | $-C_5H_{11}$ |
| 203 | 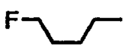 | 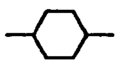 | 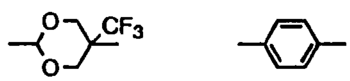 | | $-C_2H_5$ |
| 204 | 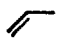 | 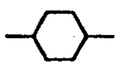 | 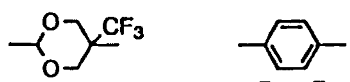 | | $-C_3H_7$ |
| 205 | 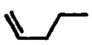 | 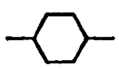 | 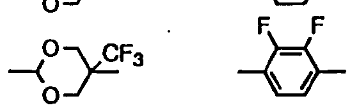 | | $-C_2H_5$ |
| 206 | 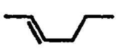 | 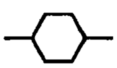 | 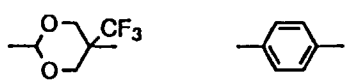 | | $-C_2H_5$ |
| 207 | $C_3H_7O$— | 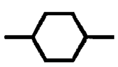 | 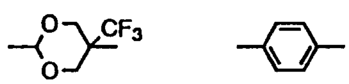 | | $-C_2H_5$ |
| 208 | 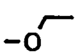 | 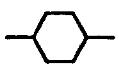 | 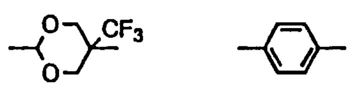 | | $-C_2H_5$ |
| 209 | $C_3H_7$— | 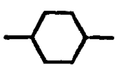 | 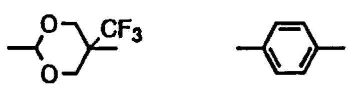 | | $-OCF_2CFHCF_3$ |
| 210 | $C_3H_7$— | 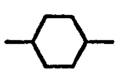 | 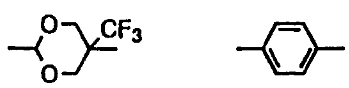 | | $-OCF_2CF_2Cl$ |

| Compound No. | $R_1$—• | •$-(A_1)_k-Z_1-(A_2)_l-Z_2$—• | | •—$Z_3-(A_3)_m-Z_4-(A_4)_n$-• | •—$R_2$ |
|---|---|---|---|---|---|
| | | | 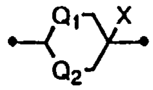 | | |
| 211 | $C_5H_{11}$— | 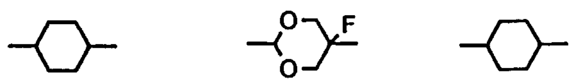 | | | $-C_2H_5$ |
| 212 | $C_3H_7$— | 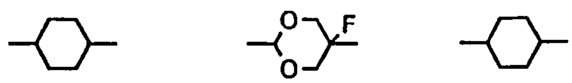 | | | $-C_2H_5$ |
| 213 | $C_5H_{11}$— | 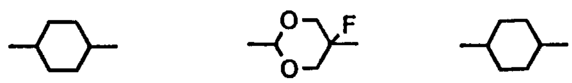 | | | $-OC_2H_5$ |
| 214 | $C_3H_7$— | 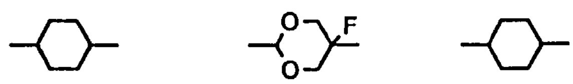 | | | $-C_3H_7$ |
| 215 | $C_3H_7$— | 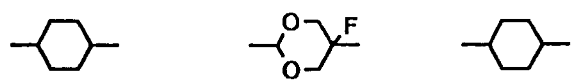 | | | $-C_2H_5$ |
| 216 | $C_3H_7$— | 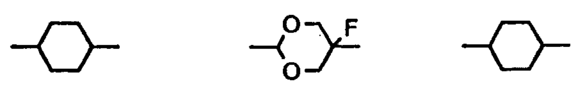 | | | $-C_5H_{11}$ |
| 217 | | 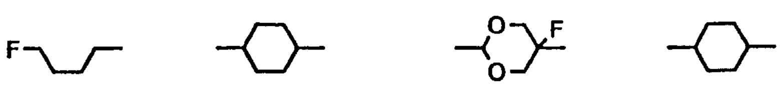 | | | $-C_2H_5$ |
| 218 | | 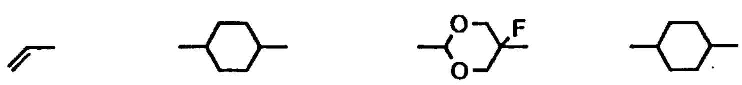 | | | $-C_3H_7$ |
| 219 | | 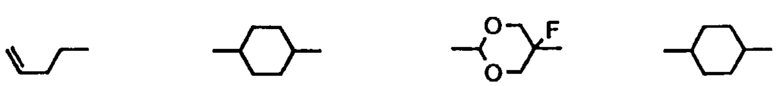 | | | $-C_2H_5$ |
| 220 | | 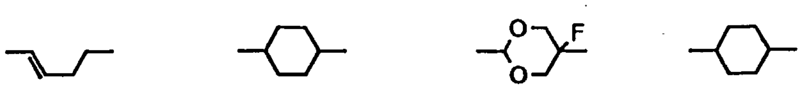 | | | $-C_2H_5$ |
| 221 | $C_3H_7O$— | 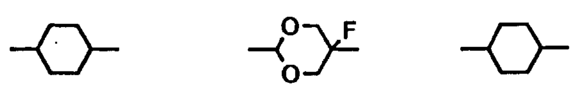 | | | $-C_2H_5$ |
| 222 | | 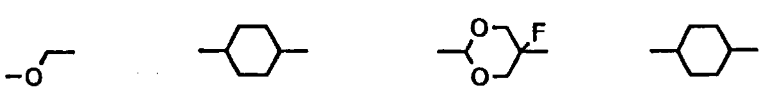 | | | $-C_2H_5$ |
| 223 | $C_3H_7$— | 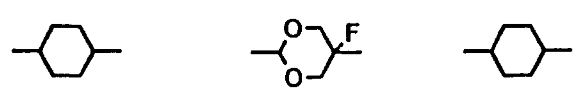 | | | $-OCF_2CFHCF_3$ |
| 224 | $C_3H_7$— | 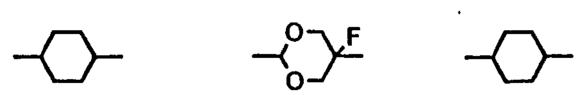 | | | $-OCF_2CF_2Cl$ |

| Compound No. | $R_1$—• | •$-(A_1)_k-Z_1-(A_2)_l-Z_2$—• | 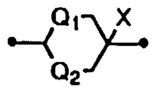 | •—$Z_3-(A_3)_m-Z_4-(A_4)_n$• | •—$R_2$ |
|---|---|---|---|---|---|
| 225 | $C_5H_{11}$— | 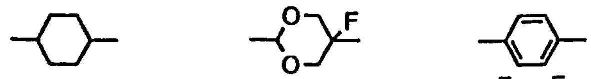 | | | $-C_2H_5$ |
| 226 | $C_3H_7$— | 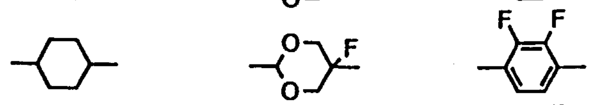 | | | $-C_2H_5$ |
| 227 | $C_5H_{11}$— | 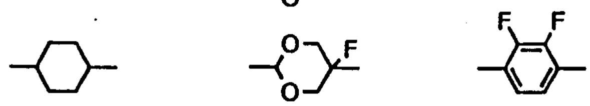 | | | $-OC_2H_5$ |
| 228 | $C_3H_7$— | 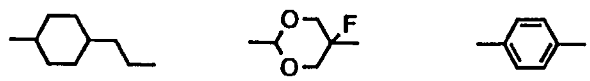 | | | $-C_3H_7$ |
| 229 | $C_3H_7$— | 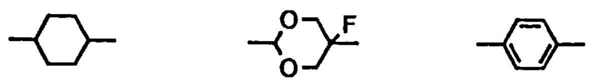 | | | $-C_2H_5$ |
| 230 | $C_3H_7$— | 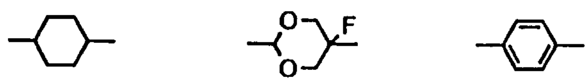 | | | $-C_5H_{11}$ |
| 231 | 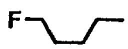 | 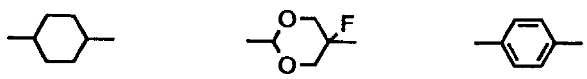 | | | $-C_2H_5$ |
| 232 | 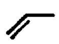 | 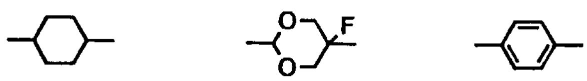 | | | $-C_3H_7$ |
| 233 | 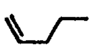 | 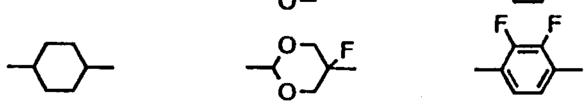 | | | $-C_2H_5$ |
| 234 | 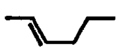 | 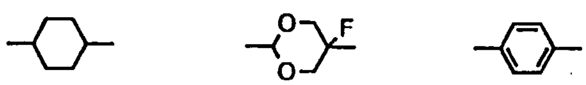 | | | $-C_2H_5$ |
| 235 | $C_3H_7O$— | 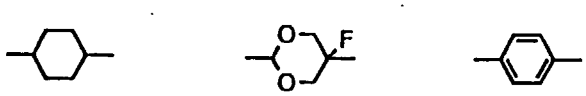 | | | $-C_2H_5$ |
| 236 | 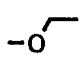 | 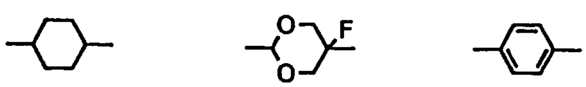 | | | $-C_2H_5$ |
| 237 | $C_3H_7$— | 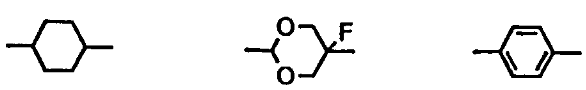 | | | $-OCF_2CFHCF_3$ |
| 238 | $C_3H_7$— | 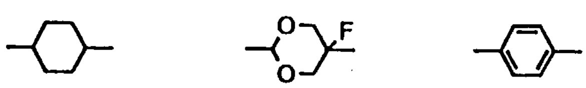 | | | $-OCF_2CF_2Cl$ |

| Compound No. | $R_1$— | 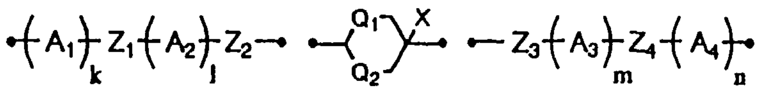 | —$R_2$ |
|---|---|---|---|
| 239 | $C_5H_{11}$— | 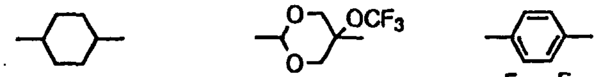 | $-C_2H_5$ |
| 240 | $C_3H_7$— | 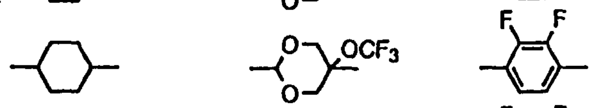 | $-C_2H_5$ |
| 241 | $C_5H_{11}$— | 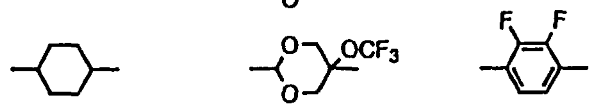 | $-OC_2H_5$ |
| 242 | $C_3H_7$— | 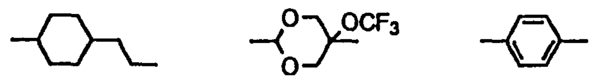 | $-C_3H_7$ |
| 243 | $C_3H_7$— | 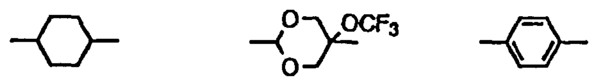 | $-C_2H_5$ |
| 244 | $C_3H_7$— | 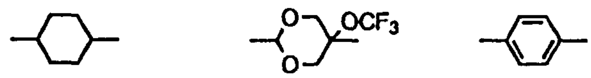 | $-C_5H_{11}$ |
| 245 | 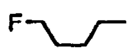 | 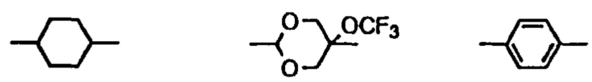 | $-C_2H_5$ |
| 246 | 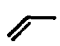 | 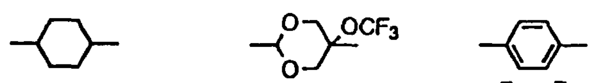 | $-C_3H_7$ |
| 247 | 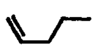 | 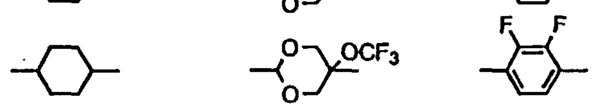 | $-C_2H_5$ |
| 248 | 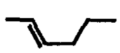 | 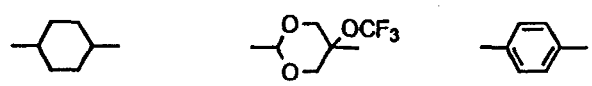 | $-C_2H_5$ |
| 249 | $C_3H_7O$— | 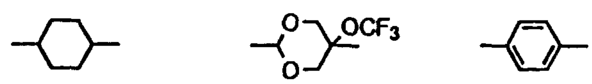 | $-C_2H_5$ |
| 250 | 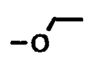 | 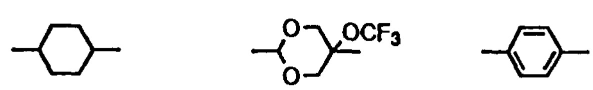 | $-C_2H_5$ |
| 251 | $C_3H_7$— | 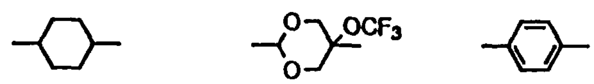 | $-OCF_2CFHCF_3$ |
| 252 | $C_3H_7$— | 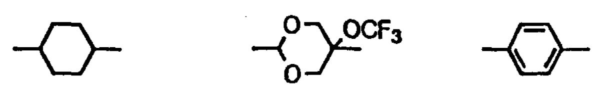 | $-OCF_2CF_2Cl$ |

| Compound No. | $R_1$—• | 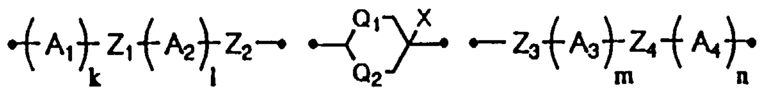 | •—$R_2$ |
|---|---|---|---|
| ·253 | $C_5H_{11}$— | 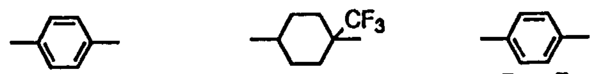 | $-C_2H_5$ |
| 254 | $C_3H_7$— | 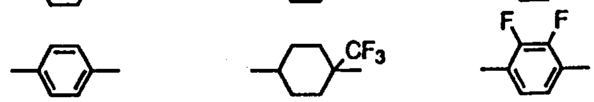 | $-C_2H_5$ |
| 255 | $C_5H_{11}$— | 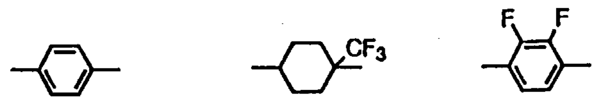 | $-OC_2H_5$ |
| 256 | $C_3H_7$— | 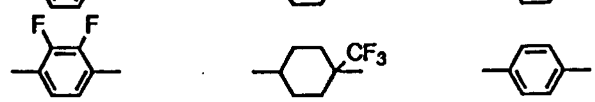 | $-C_3H_7$ |
| 257 | $C_3H_7$— | 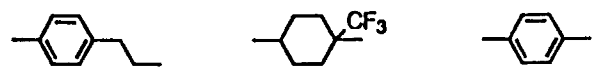 | $-C_2H_5$ |
| 258 | $C_3H_7$— | 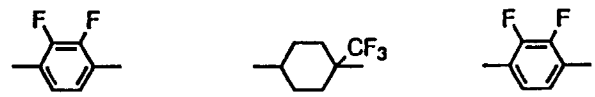 | $-C_5H_{11}$ |
| 259 | 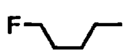 | 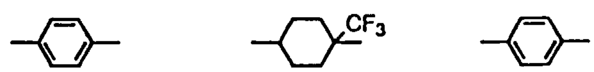 | $-C_2H_5$ |
| 260 | 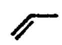 | 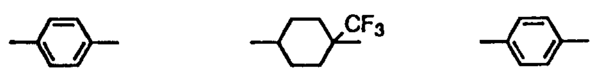 | $-C_3H_7$ |
| 261 | 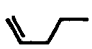 | 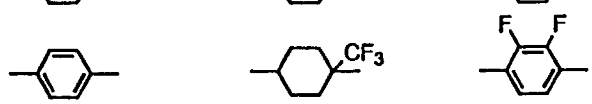 | $-C_2H_5$ |
| 262 | 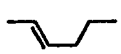 | 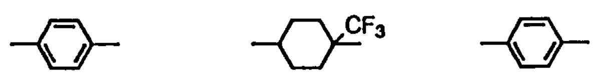 | $-C_2H_5$ |
| 263 | $C_3H_7O$— | 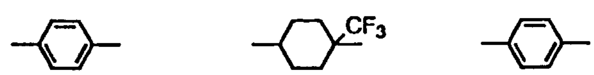 | $-C_2H_5$ |
| 264 | 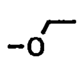 | 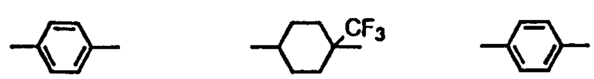 | $-C_2H_5$ |
| 265 | $C_3H_7$— | 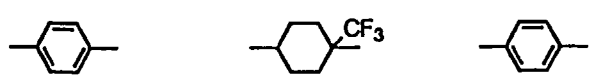 | $-OCF_2CFHCF_3$ |
| 266 | $C_3H_7$— | 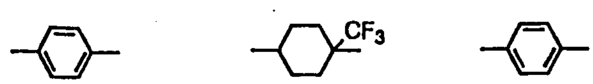 | $-OCF_2CF_2Cl$ |

| Compound No. | $R_1$ | Rings | | | $R_2$ |
|---|---|---|---|---|---|
| | 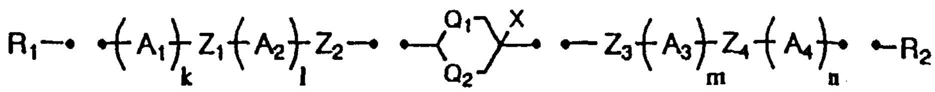 | | | | |
| 267 | $C_5H_{11}-$ | 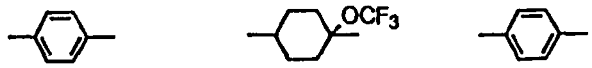 | | | $-C_2H_5$ |
| 268 | $C_3H_7-$ | 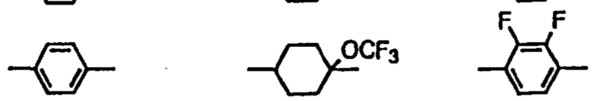 | | | $-C_2H_5$ |
| 269 | $C_5H_{11}-$ | 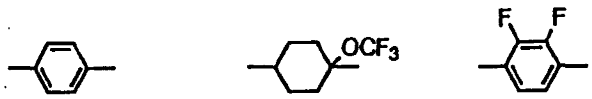 | | | $-OC_2H_5$ |
| 270 | $C_3H_7-$ | 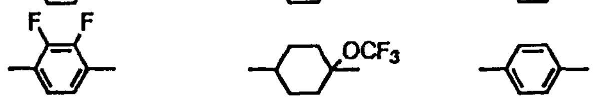 | | | $-C_3H_7$ |
| 271 | $C_3H_7-$ | 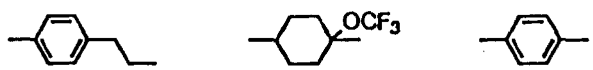 | | | $-C_2H_5$ |
| 272 | $C_3H_7-$ | 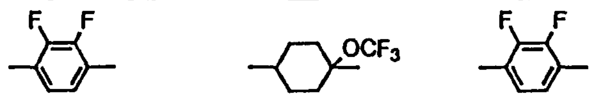 | | | $-C_5H_{11}$ |
| 273 | 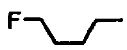 | 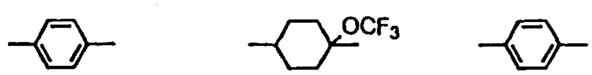 | | | $-C_2H_5$ |
| 274 | 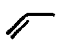 | 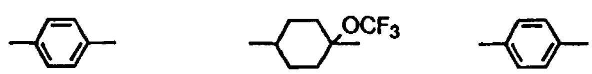 | | | $-C_3H_7$ |
| 275 | 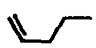 | 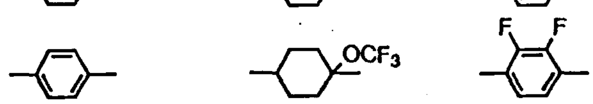 | | | $-C_2H_5$ |
| 276 | 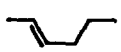 | 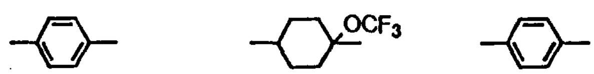 | | | $-C_2H_5$ |
| 277 | $C_3H_7O-$ | 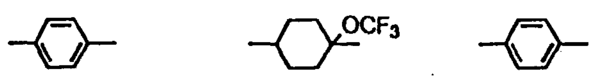 | | | $-C_2H_5$ |
| 278 | 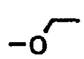 | 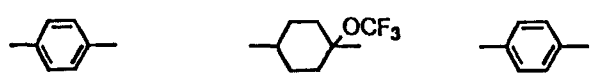 | | | $-C_2H_5$ |
| 279 | $C_3H_7-$ | 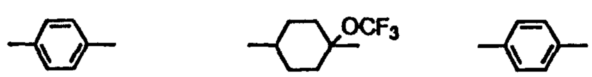 | | | $-OCF_2CFHCF_3$ |
| 280 | $C_3H_7-$ | 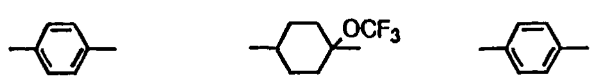 | | | $-OCF_2CF_2Cl$ |

| Compound No. | 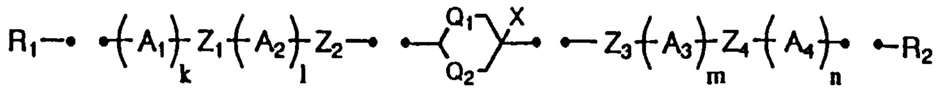 | | |
|---|---|---|---|
| 281 | $C_5H_{11}-$ | 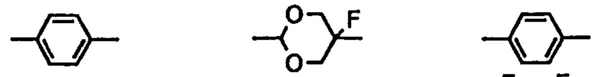 | $-C_2H_5$ |
| 282 | $C_3H_7-$ | 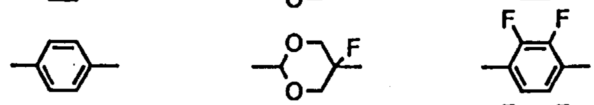 | $-C_2H_5$ |
| 283 | $C_5H_{11}-$ | 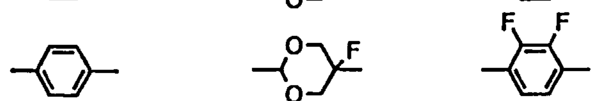 | $-OC_2H_5$ |
| 284 | $C_3H_7-$ | 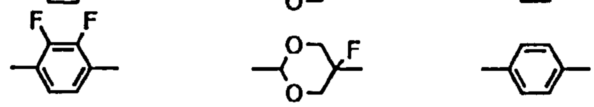 | $-C_3H_7$ |
| 285 | $C_3H_7-$ | 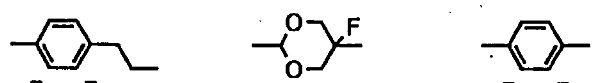 | $-C_2H_5$ |
| 286 | $C_3H_7-$ | 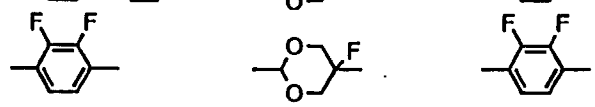 | $-C_5H_{11}$ |
| 287 | 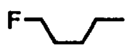 | 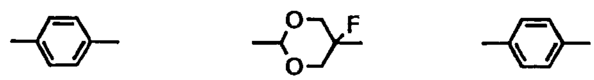 | $-C_2H_5$ |
| 288 | 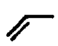 | 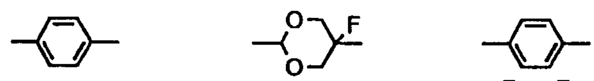 | $-C_3H_7$ |
| 289 | 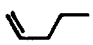 | 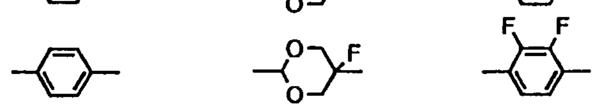 | $-C_2H_5$ |
| 290 | 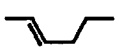 | 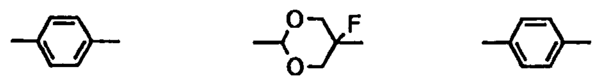 | $-C_2H_5$ |
| 291 | $C_3H_7O-$ | 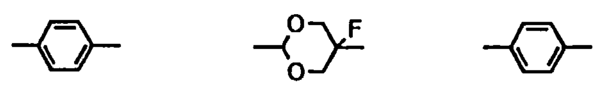 | $-C_2H_5$ |
| 292 | 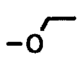 | 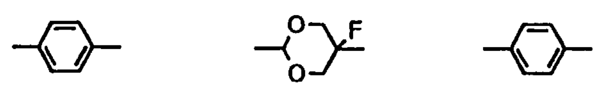 | $-C_2H_5$ |
| 293 | $C_3H_7-$ | 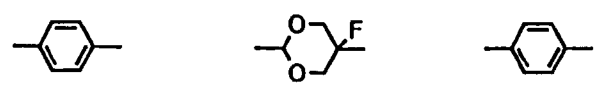 | $-OCF_2CFHCF_3$ |
| 294 | $C_3H_7-$ | 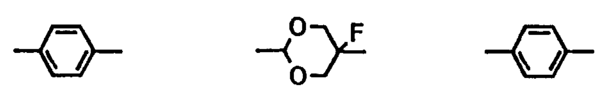 | $-OCF_2CF_2Cl$ |

| Compound No. | $R_1-$ | $-(A_1)_k-Z_1-(A_2)_l-Z_2-$ | 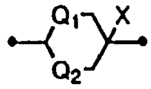 | $-Z_3-(A_3)_m-Z_4-(A_4)_n-$ | $-R_2$ |
|---|---|---|---|---|---|
| 295 | $C_5H_{11}-$ | 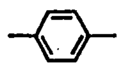 | 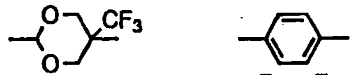 | | $-C_2H_5$ |
| 296 | $C_3H_7-$ | 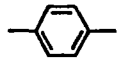 | 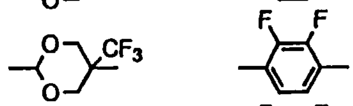 | | $-C_2H_5$ |
| 297 | $C_5H_{11}-$ | 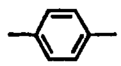 | 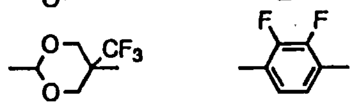 | | $-OC_2H_5$ |
| 298 | $C_3H_7-$ | 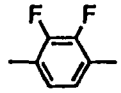 | 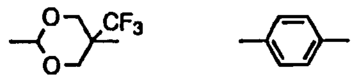 | | $-C_3H_7$ |
| 299 | $C_3H_7-$ | 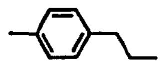 | 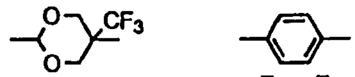 | | $-C_2H_5$ |
| 300 | $C_3H_7-$ | 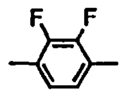 | 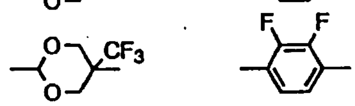 | | $-C_5H_{11}$ |
| 301 | 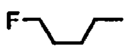 | 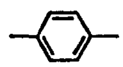 | 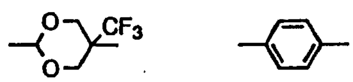 | | $-C_2H_5$ |
| 302 | 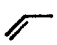 | 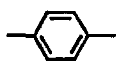 | 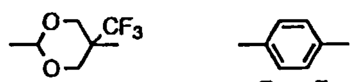 | | $-C_3H_7$ |
| 303 | 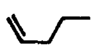 | 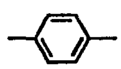 | 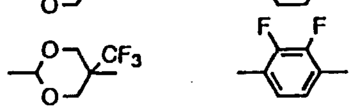 | | $-C_2H_5$ |
| 304 | 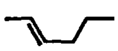 | 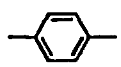 | 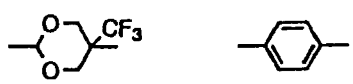 | | $-C_2H_5$ |
| 305 | $C_3H_7O-$ | 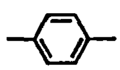 | 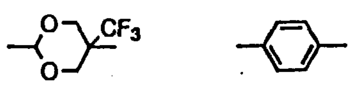 | | $-C_2H_5$ |
| 306 | 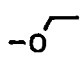 | 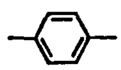 | 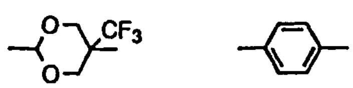 | | $-C_2H_5$ |
| 307 | $C_3H_7-$ | 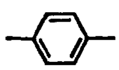 | 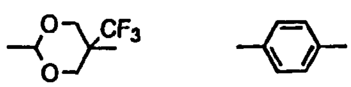 | | $-OCF_2CFHCF_3$ |
| 308 | $C_3H_7-$ | 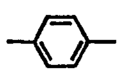 | 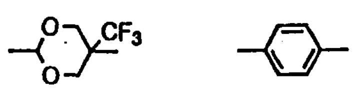 | | $-OCF_2CF_2Cl$ |

| Compound No. | $R_1$—• | •$\{A_1\}_k$-$Z_1$-$\{A_2\}_l$-$Z_2$—• | 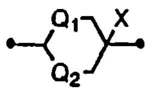 | •—$Z_3$-$\{A_3\}_m$-$Z_4$-$\{A_4\}_n$• | •—$R_2$ |
|---|---|---|---|---|---|
| 309 | $C_5H_{11}$— | 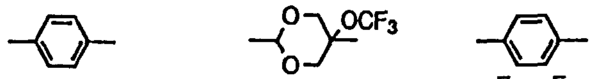 | | | $-C_2H_5$ |
| 310 | $C_3H_7$— | 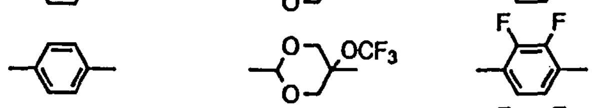 | | | $-C_2H_5$ |
| 311 | $C_5H_{11}$— | 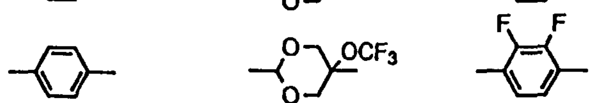 | | | $-OC_2H_5$ |
| 312 | $C_3H_7$— | 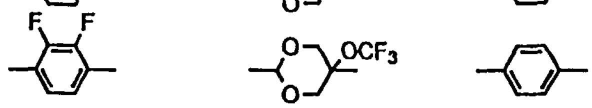 | | | $-C_3H_7$ |
| 313 | $C_3H_7$— | 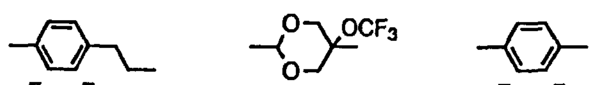 | | | $-C_2H_5$ |
| 314 | $C_3H_7$— | 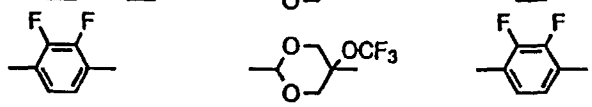 | | | $-C_5H_{11}$ |
| 315 | 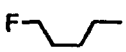 | 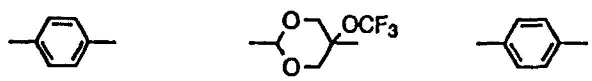 | | | $-C_2H_5$ |
| 316 | 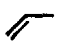 | 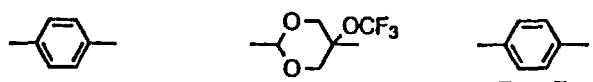 | | | $-C_3H_7$ |
| 317 | 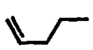 | 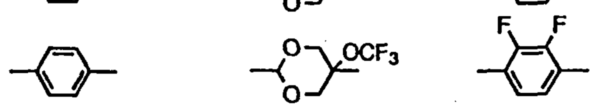 | | | $-C_2H_5$ |
| 318 | 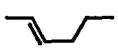 | 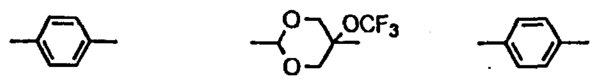 | | | $-C_2H_5$ |
| 319 | $C_3H_7O$— | 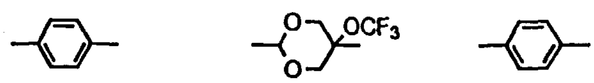 | | | $-C_2H_5$ |
| 320 | 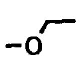 | 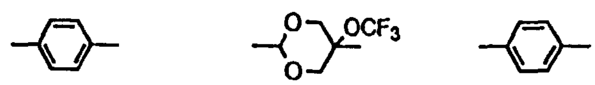 | | | $-C_2H_5$ |
| 321 | $C_3H_7$— | 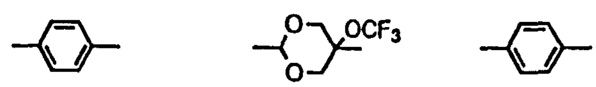 | | | $-OCF_2CFHCF_3$ |
| 322 | $C_3H_7$— | 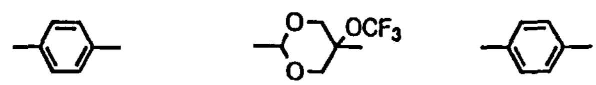 | | | $-OCF_2CF_2Cl$ |

| Compound No. | $R_1$—• | •-(A₁)ₖ-Z₁-(A₂)ₗ-Z₂—• | 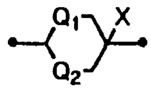 | •—$Z_3$-$(A_3)_m$-$Z_4$-$(A_4)_n$-• | •—$R_2$ |
|---|---|---|---|---|---|
| 323 | $C_5H_{11}$— | | 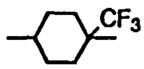 | 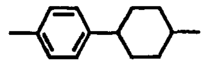 | $-C_2H_5$ |
| 324 | $C_3H_7$— | | 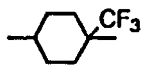 | 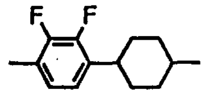 | $-C_2H_5$ |
| 325 | $C_5H_{11}$— | | 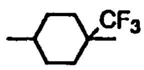 | 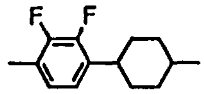 | $-OC_2H_5$ |
| 326 | $C_3H_7$— | | 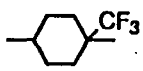 | 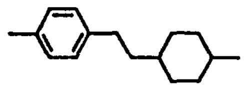 | $-C_3H_7$ |
| 327 | $C_3H_7$— | | 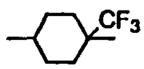 | 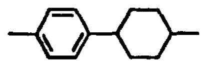 | $-C_2H_5$ |
| 328 | $C_3H_7$— | | 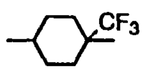 | 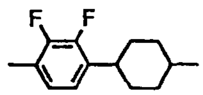 | $-C_5H_{11}$ |
| 329 | 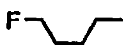 | | 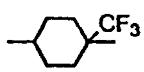 | 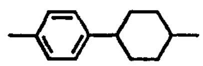 | $-C_2H_5$ |
| 330 | 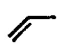 | | 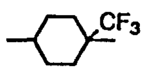 | 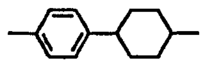 | $-C_3H_7$ |
| 331 | 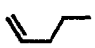 | | 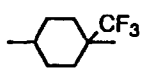 | 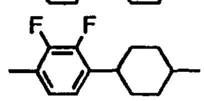 | $-C_2H_5$ |
| 332 | 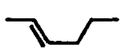 | | 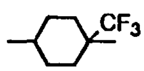 | 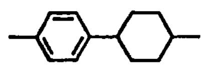 | $-C_2H_5$ |
| 333 | $C_3H_7O$— | | 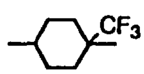 | 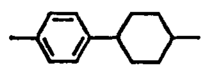 | $-C_2H_5$ |
| 334 | 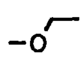 | | 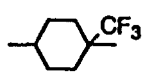 | 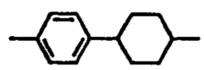 | $-C_2H_5$ |
| 335 | $C_3H_7$— | | 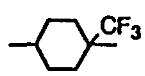 | 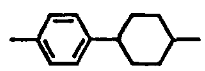 | $-OCF_2CFHCF_3$ |
| 336 | $C_3H_7$— | | 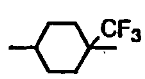 | 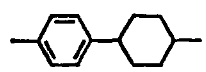 | $-OCF_2CF_2Cl$ |

| Compound No. | $R_1-$ | $-(A_1)_k-Z_1-(A_2)_l-Z_2-$ | 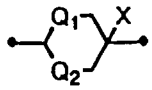 | $-Z_3-(A_3)_m-Z_4-(A_4)_n-$ | $-R_2$ |
|---|---|---|---|---|---|
| 337 | $C_5H_{11}-$ | | 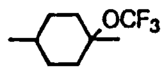 | 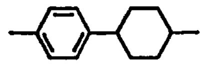 | $-C_2H_5$ |
| 338 | $C_3H_7-$ | | 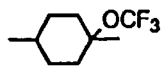 | 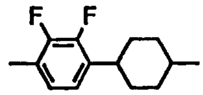 | $-C_2H_5$ |
| 339 | $C_5H_{11}-$ | | 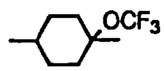 | 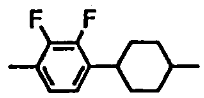 | $-OC_2H_5$ |
| 340 | $C_3H_7-$ | | 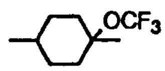 | 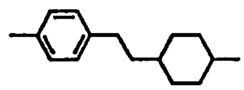 | $-C_3H_7$ |
| 341 | $C_3H_7-$ | | 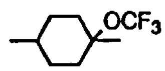 | 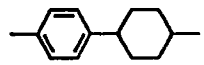 | $-C_2H_5$ |
| 342 | $C_3H_7-$ | | 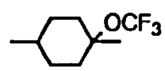 | 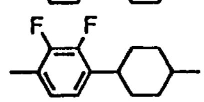 | $-C_5H_{11}$ |
| 343 | 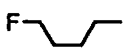 | | 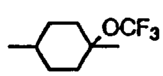 | 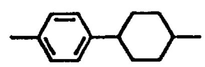 | $-C_2H_5$ |
| 344 | 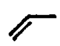 | | 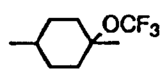 | 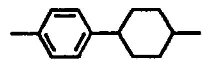 | $-C_3H_7$ |
| 345 | 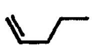 | | 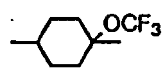 | 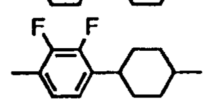 | $-C_2H_5$ |
| 346 | 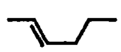 | | 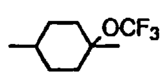 | 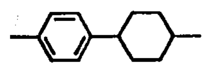 | $-C_2H_5$ |
| 347 | $C_3H_7O-$ | | 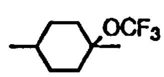 | 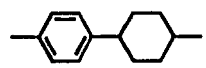 | $-C_2H_5$ |
| 348 | 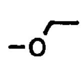 | | 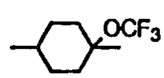 | 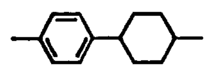 | $-C_2H_5$ |
| 349 | $C_3H_7-$ | | 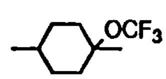 | 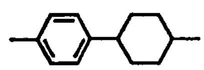 | $-OCF_2CFHCF_3$ |
| 350 | $C_3H_7-$ | | 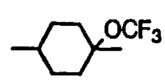 | 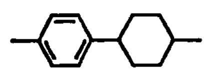 | $-OCF_2CF_2Cl$ |

| Compound No. | $R_1-\bullet$ | $\bullet\!-\!(A_1)_k-Z_1-(A_2)_l-Z_2-\bullet$ | 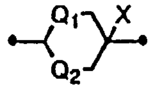 | $\bullet-Z_3-(A_3)_m-Z_4-(A_4)_n-\bullet$ | $\bullet-R_2$ |
|---|---|---|---|---|---|
| 351 | $C_5H_{11}-$ | | 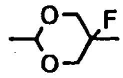 | 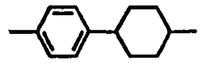 | $-C_2H_5$ |
| 352 | $C_3H_7-$ | | 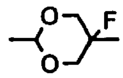 | 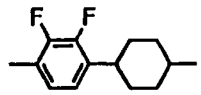 | $-C_2H_5$ |
| 353 | $C_5H_{11}-$ | | 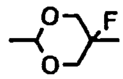 | 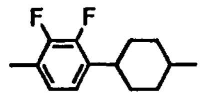 | $-OC_2H_5$ |
| 354 | $C_3H_7-$ | | 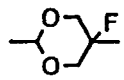 | 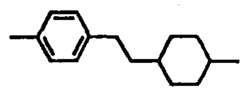 | $-C_3H_7$ |
| 355 | $C_3H_7-$ | | 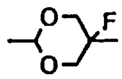 | 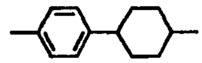 | $-C_2H_5$ |
| 356 | $C_3H_7-$ | | 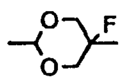 | 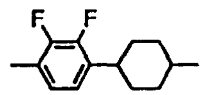 | $-C_5H_{11}$ |
| 357 | 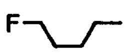 | | 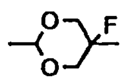 | 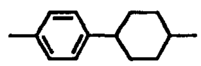 | $-C_2H_5$ |
| 358 | 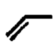 | | 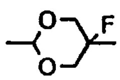 | 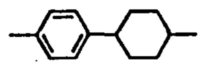 | $-C_3H_7$ |
| 359 | 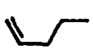 | | 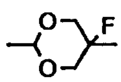 | 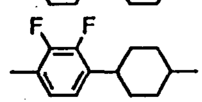 | $-C_2H_5$ |
| 360 | 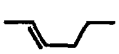 | | 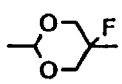 | 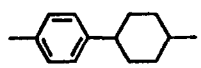 | $-C_2H_5$ |
| 361 | $C_3H_7O-$ | | 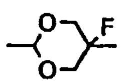 | 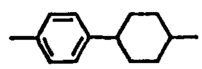 | $-C_2H_5$ |
| 362 | 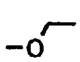 | | 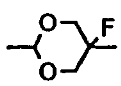 | 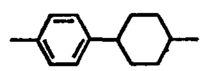 | $-C_2H_5$ |
| 363 | $C_3H_7-$ | | 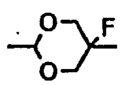 | 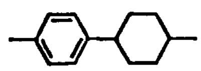 | $-OCF_2CFHCF_3$ |
| 364 | $C_3H_7-$ | | 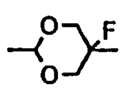 | 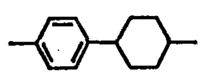 | $-OCF_2CF_2Cl$ |

| Compound No. | $R_1$—• | •$(A_1)_k$ $Z_1$ $(A_2)_l$ $Z_2$—• | 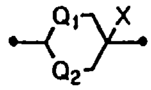 | •—$Z_3$ $(A_3)_m$ $Z_4$ $(A_4)_n$• | •—$R_2$ |
|---|---|---|---|---|---|
| 365 | $C_5H_{11}$— | | 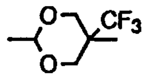 | 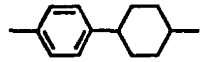 | $-C_2H_5$ |
| 366 | $C_3H_7$— | | 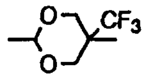 | 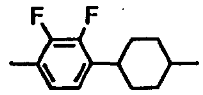 | $-C_2H_5$ |
| 367 | $C_5H_{11}$— | | 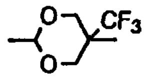 | 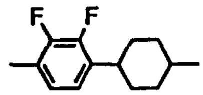 | $-OC_2H_5$ |
| 368 | $C_3H_7$— | | 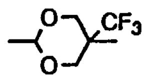 | 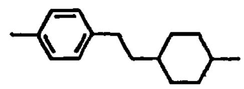 | $-C_3H_7$ |
| 369 | $C_3H_7$— | | 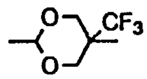 | 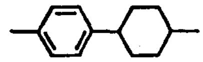 | $-C_2H_5$ |
| 370 | $C_3H_7$— | | 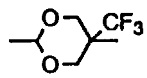 | 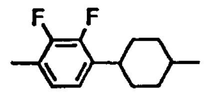 | $-C_5H_{11}$ |
| 371 | 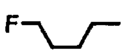 | | 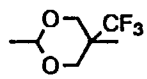 | 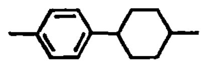 | $-C_2H_5$ |
| 372 | 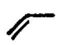 | | 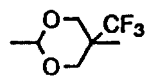 | 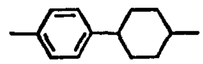 | $-C_3H_7$ |
| 373 | 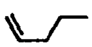 | | 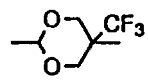 | 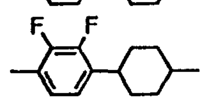 | $-C_2H_5$ |
| 374 | 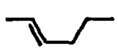 | | 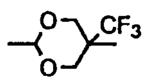 | 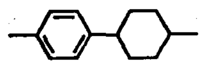 | $-C_2H_5$ |
| 375 | $C_3H_7O$— | | 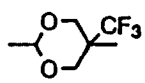 | 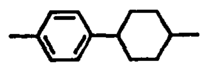 | $-C_2H_5$ |
| 376 | 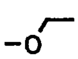 | | 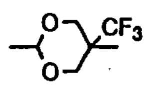 | 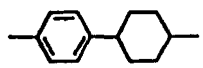 | $-C_2H_5$ |
| 377 | $C_3H_7$— | | 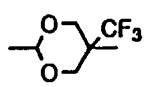 | 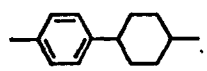 | $-OCF_2CFHCF_3$ |
| 378 | $C_3H_7$— | | 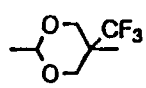 | 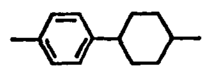 | $-OCF_2CF_2Cl$ |

| Compound No. | $R_1$—• | •–(A$_1$)$_k$–Z$_1$–(A$_2$)$_l$–Z$_2$—• | 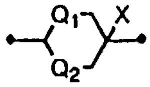 | •—Z$_3$–(A$_3$)$_m$–Z$_4$–(A$_4$)$_n$–• | •–$R_2$ |
|---|---|---|---|---|---|
| 379 | $C_5H_{11}$— | 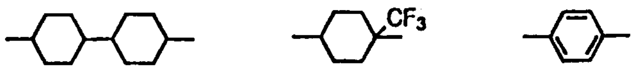 | | | $-C_2H_5$ |
| 380 | $C_3H_7$— | 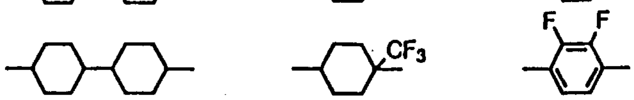 | | | $-C_2H_5$ |
| 381 | $C_5H_{11}$— | 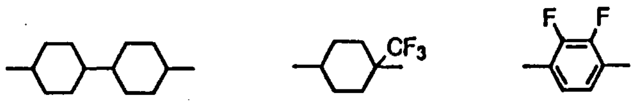 | | | $-OC_2H_5$ |
| 382 | $C_3H_7$— | 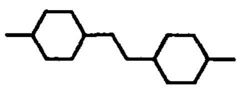 | | | $-C_3H_7$ |
| 383 | $C_3H_7$— | 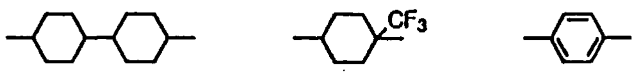 | | | $-C_2H_5$ |
| 384 | $C_3H_7$— | 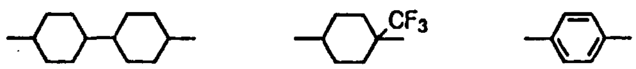 | | | $-C_5H_{11}$ |
| 385 | 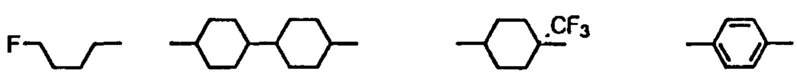 | | | | $-C_2H_5$ |
| 386 | 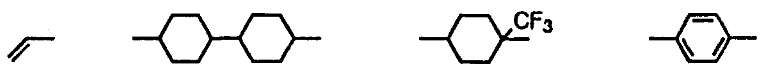 | | | | $-C_3H_7$ |
| 387 | 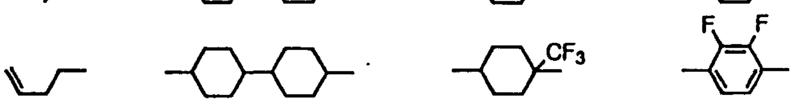 | | | | $-C_2H_5$ |
| 388 | 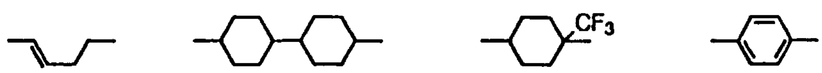 | | | | $-C_2H_5$ |
| 389 | $C_3H_7O$— | 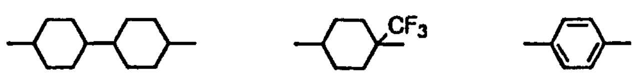 | | | $-C_2H_5$ |
| 390 | 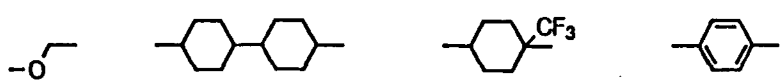 | | | | $-C_2H_5$ |
| 391 | $C_3H_7$— | 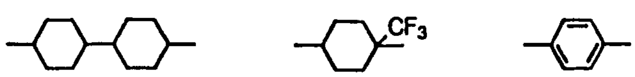 | | | $-OCF_2CFHCF_3$ |
| 392 | $C_3H_7$— | 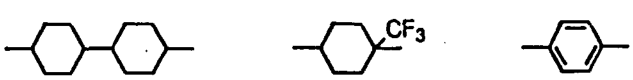 | | | $-OCF_2CF_2Cl$ |

| Compound No. | $R_1$ | | $R_2$ |
|---|---|---|---|
| | | 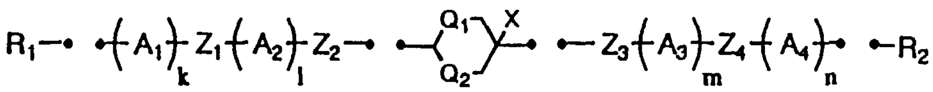 | |
| 393 | $C_5H_{11}-$ | 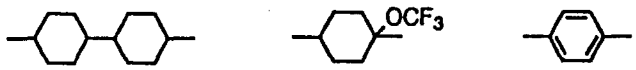 | $-C_2H_5$ |
| 394 | $C_3H_7-$ | 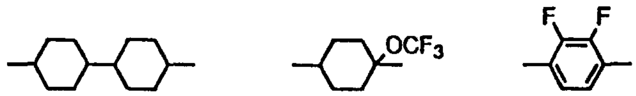 | $-C_2H_5$ |
| 395 | $C_5H_{11}-$ | 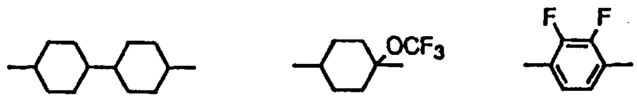 | $-OC_2H_5$ |
| 396 | $C_3H_7-$ | 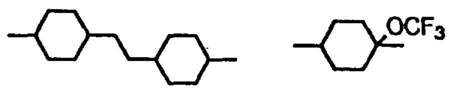 | $-C_3H_7$ |
| 397 | $C_3H_7-$ | 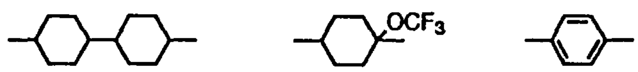 | $-C_2H_5$ |
| 398 | $C_3H_7-$ | 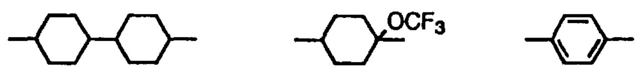 | $-C_5H_{11}$ |
| 399 | 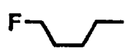 | 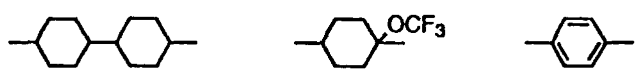 | $-C_2H_5$ |
| 400 | 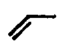 | 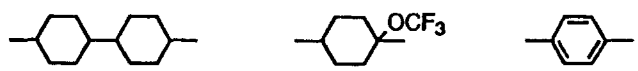 | $-C_3H_7$ |
| 401 | 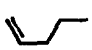 | 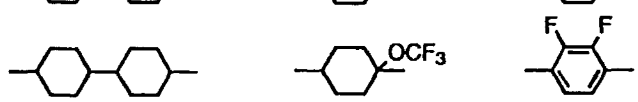 | $-C_2H_5$ |
| 402 | 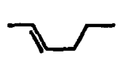 | 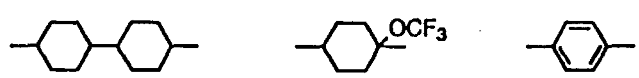 | $-C_2H_5$ |
| 403 | $C_3H_7O-$ | 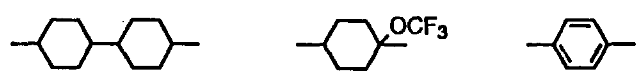 | $-C_2H_5$ |
| 404 | 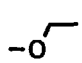 | 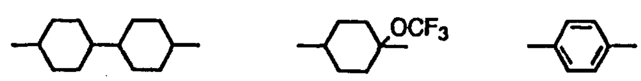 | $-C_2H_5$ |
| 405 | $C_3H_7-$ | 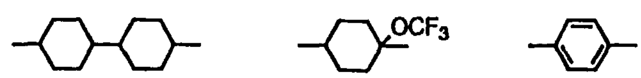 | $-OCF_2CFHCF_3$ |
| 406 | $C_3H_7-$ | 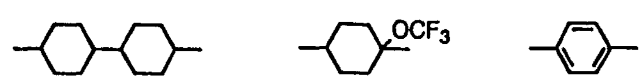 | $-OCF_2CF_2Cl$ |

| Compound No. | $R_1-$ | $-(A_1)_k-Z_1-(A_2)_l-Z_2-$ | 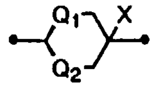 | $-Z_3-(A_3)_m-Z_4-(A_4)_n-$ | $-R_2$ |
|---|---|---|---|---|---|
| 407 | $C_5H_{11}-$ | 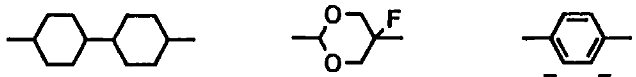 | | | $-C_2H_5$ |
| 408 | $C_3H_7-$ | 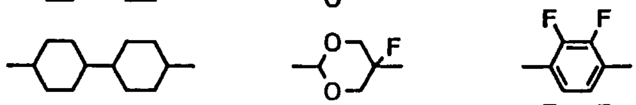 | | | $-C_2H_5$ |
| 409 | $C_5H_{11}-$ | 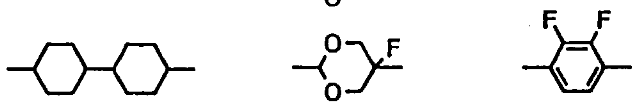 | | | $-OC_2H_5$ |
| 410 | $C_3H_7-$ | 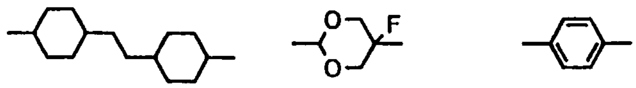 | | | $-C_3H_7$ |
| 411 | $C_3H_7-$ | 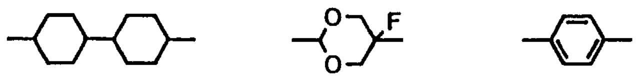 | | | $-C_2H_5$ |
| 412 | $C_3H_7-$ | 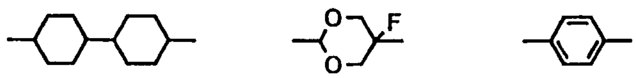 | | | $-C_5H_{11}$ |
| 413 | 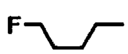 | 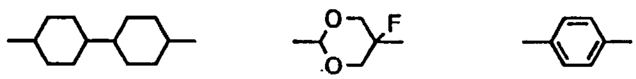 | | | $-C_2H_5$ |
| 414 | 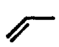 | 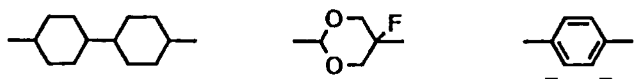 | | | $-C_3H_7$ |
| 415 | 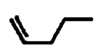 | 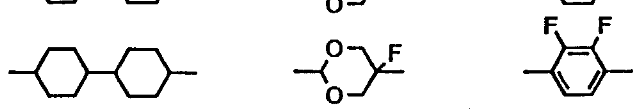 | | | $-C_2H_5$ |
| 416 | 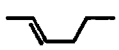 | 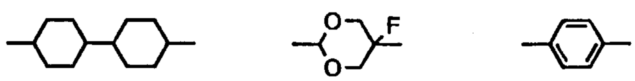 | | | $-C_2H_5$ |
| 417 | $C_3H_7O-$ | 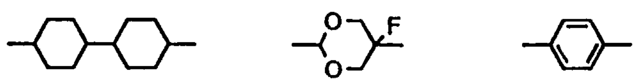 | | | $-C_2H_5$ |
| 418 | 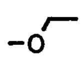 | 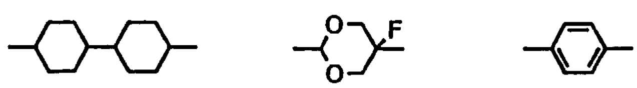 | | | $-C_2H_5$ |
| 419 | $C_3H_7-$ | 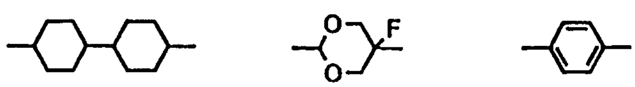 | | | $-OCF_2CFHCF_3$ |
| 420 | $C_3H_7-$ | 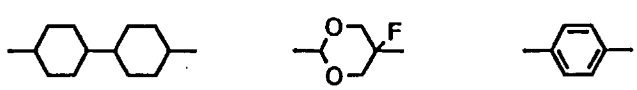 | | | $-OCF_2CF_2Cl$ |

| Compound No. | $R_1$—• | •$-(A_1)_k-Z_1-(A_2)_l-Z_2$—• | 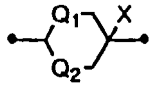 | •—$Z_3-(A_3)_m-Z_4-(A_4)_n$-• | •—$R_2$ |
|---|---|---|---|---|---|
| 421 | $C_5H_{11}$— | 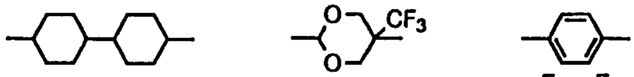 | | | $-C_2H_5$ |
| 422 | $C_3H_7$— | 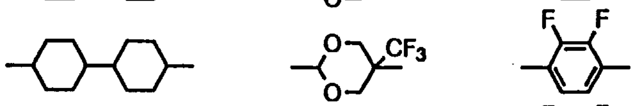 | | | $-C_2H_5$ |
| 423 | $C_5H_{11}$— | 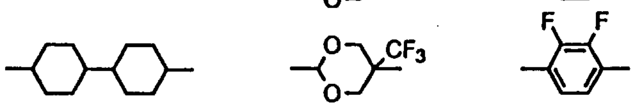 | | | $-OC_2H_5$ |
| 424 | $C_3H_7$— | 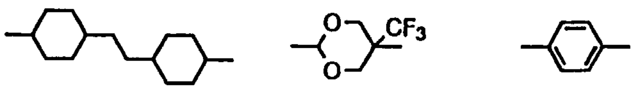 | | | $-C_3H_7$ |
| 425 | $C_3H_7$— | 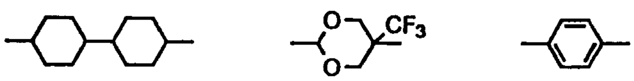 | | | $-C_2H_5$ |
| 426 | $C_3H_7$— | 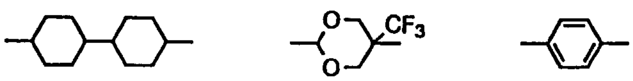 | | | $-C_5H_{11}$ |
| 427 | 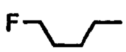 | 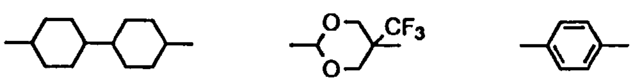 | | | $-C_2H_5$ |
| 428 | 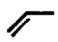 | 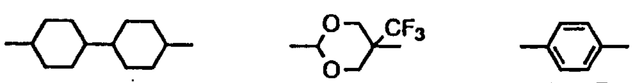 | | | $-C_3H_7$ |
| 429 | 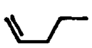 | 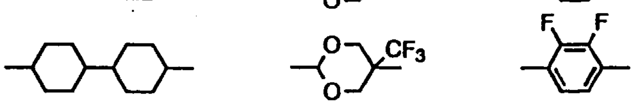 | | | $-C_2H_5$ |
| 430 | 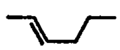 | 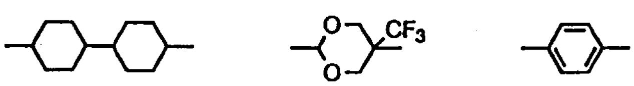 | | | $-C_2H_5$ |
| 431 | $C_3H_7O$— | 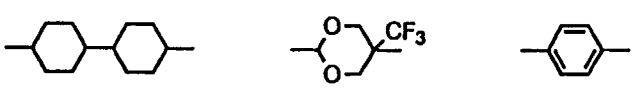 | | | $-C_2H_5$ |
| 432 | 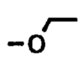 | 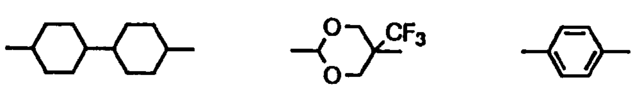 | | | $-C_2H_5$ |
| 433 | $C_3H_7$— | 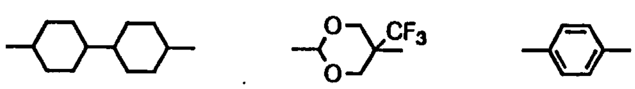 | | | $-OCF_2CFHCF_3$ |
| 434 | $C_3H_7$— | 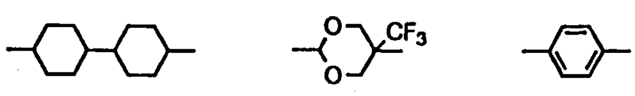 | | | $-OCF_2CF_2Cl$ |

| Compound No. | R1 | Structure | R2 |
|---|---|---|---|
| | | 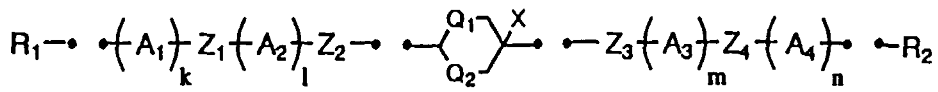 | |
| 435 | $C_5H_{11}$— | 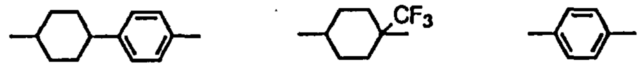 | $-C_2H_5$ |
| 436 | $C_3H_7$— | 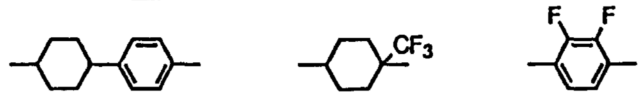 | $-C_2H_5$ |
| 437 | $C_5H_{11}$— | 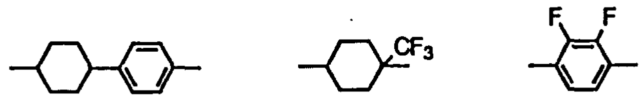 | $-OC_2H_5$ |
| 438 | $C_3H_7$— | 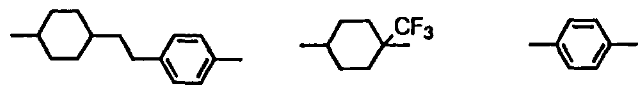 | $-C_3H_7$ |
| 439 | $C_3H_7$— | 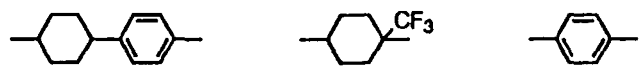 | $-C_2H_5$ |
| 440 | $C_3H_7$— | 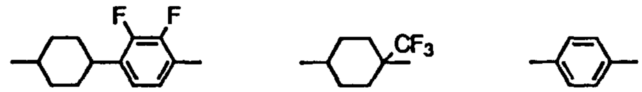 | $-C_5H_{11}$ |
| 441 | 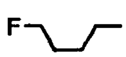 | 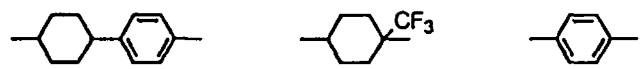 | $-C_2H_5$ |
| 442 | 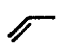 | 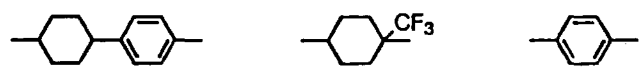 | $-C_3H_7$ |
| 443 | 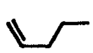 | 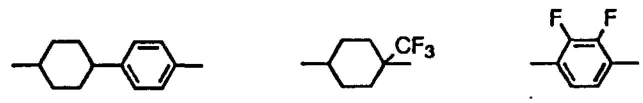 | $-C_2H_5$ |
| 444 | 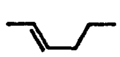 | 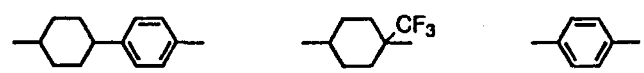 | $-C_2H_5$ |
| 445 | $C_3H_7O$— | 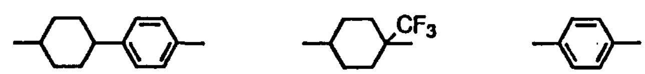 | $-C_2H_5$ |
| 446 | 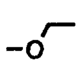 | 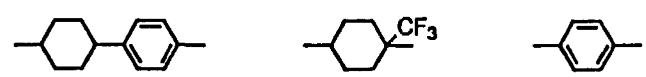 | $-C_2H_5$ |
| 447 | $C_3H_7$— | 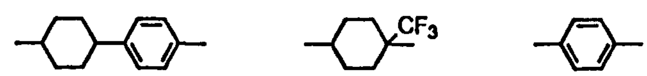 | $-OCF_2CFHCF_3$ |
| 448 | $C_3H_7$— | 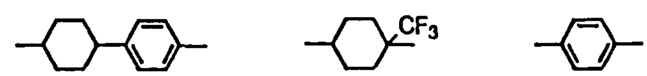 | $-OCF_2CF_2Cl$ |

| Compound No. | $R_1-\bullet$ | $\bullet-(A_1)_k-Z_1-(A_2)_l-Z_2-\bullet$ | 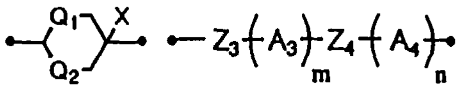 | | $\bullet-R_2$ |
|---|---|---|---|---|---|
| 449 | $C_5H_{11}-$ | 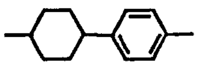 | 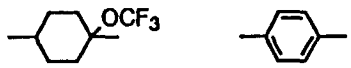 | | $-C_2H_5$ |
| 450 | $C_3H_7-$ | 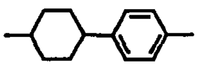 | 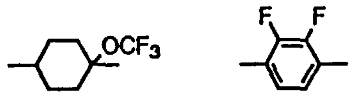 | | $-C_2H_5$ |
| 451 | $C_5H_{11}-$ | 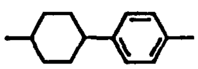 | 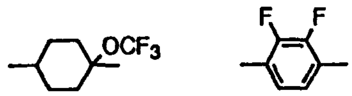 | | $-OC_2H_5$ |
| 452 | $C_3H_7-$ | 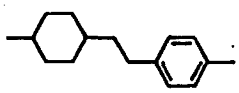 | 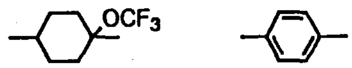 | | $-C_3H_7$ |
| 453 | $C_3H_7-$ | 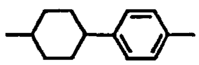 | 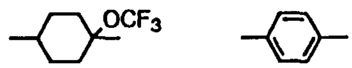 | | $-C_2H_5$ |
| 454 | $C_3H_7-$ | 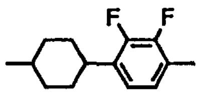 | 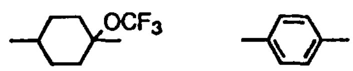 | | $-C_5H_{11}$ |
| 455 | 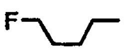 | 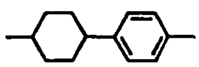 | 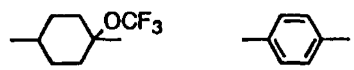 | | $-C_2H_5$ |
| 456 | 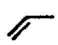 | 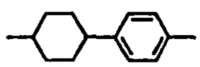 | 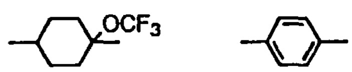 | | $-C_3H_7$ |
| 457 | 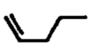 | 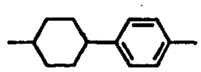 | 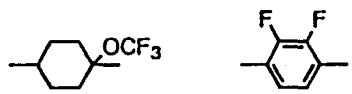 | | $-C_2H_5$ |
| 458 | 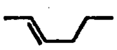 | 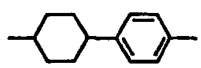 | 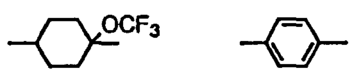 | | $-C_2H_5$ |
| 459 | $C_3H_7O-$ | 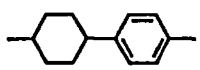 | 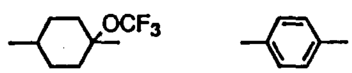 | | $-C_2H_5$ |
| 460 | 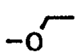 | 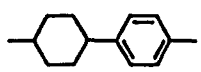 | 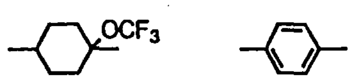 | | $-C_2H_5$ |
| 461 | $C_3H_7-$ | 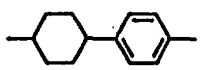 | 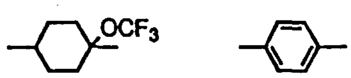 | | $-OCF_2CFHCF_3$ |
| 462 | $C_3H_7-$ | 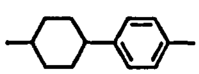 | 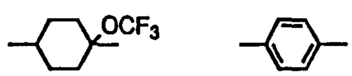 | | $-OCF_2CF_2Cl$ |

| Compound No. | $R_1$—• | •$(A_1)_k$$Z_1$$(A_2)_l$$Z_2$—• | 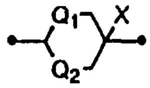 | •—$Z_3$$(A_3)_m$$Z_4$$(A_4)_n$• | •—$R_2$ |
|---|---|---|---|---|---|
| 463 | $C_5H_{11}$— | 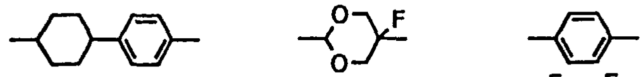 | | | $-C_2H_5$ |
| 464 | $C_3H_7$— | 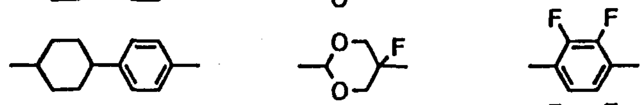 | | | $-C_2H_5$ |
| 465 | $C_5H_{11}$— | 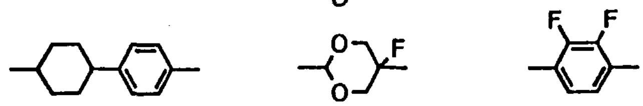 | | | $-OC_2H_5$ |
| 466 | $C_3H_7$— | 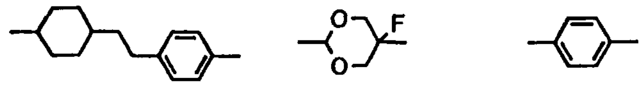 | | | $-C_3H_7$ |
| 467 | $C_3H_7$— | 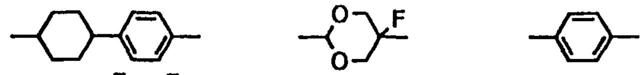 | | | $-C_2H_5$ |
| 468 | $C_3H_7$— | 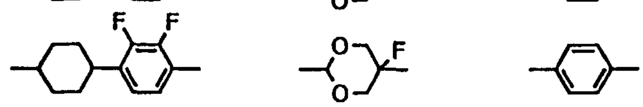 | | | $-C_5H_{11}$ |
| 469 | 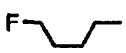 | 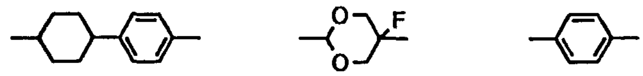 | | | $-C_2H_5$ |
| 470 | 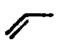 | 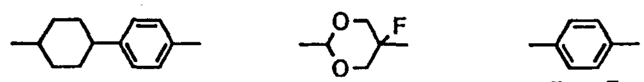 | | | $-C_3H_7$ |
| 471 | 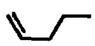 | 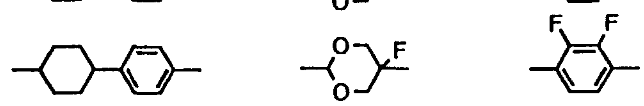 | | | $-C_2H_5$ |
| 472 | 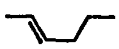 | 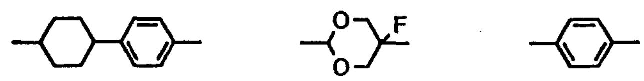 | | | $-C_2H_5$ |
| 473 | $C_3H_7O$— | 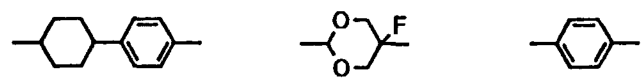 | | | $-C_2H_5$ |
| 474 | 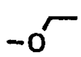 | 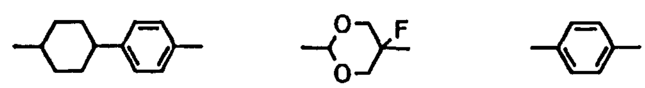 | | | $-C_2H_5$ |
| 475 | $C_3H_7$— | 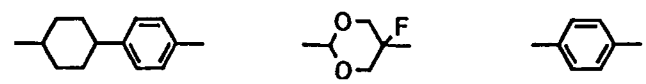 | | | $-OCF_2CFHCF_3$ |
| 476 | $C_3H_7$— | 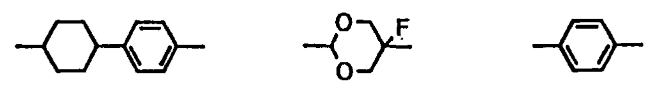 | | | $-OCF_2CF_2Cl$ |

| Compound No. | $R_1$—• | •$(A_1)_k$ $Z_1$ $(A_2)_l$ $Z_2$—• | 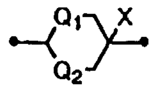 | •—$Z_3$ $(A_3)_m$ $Z_4$ $(A_4)_n$• | •—$R_2$ |
|---|---|---|---|---|---|
| 477 | $C_5H_{11}$— | 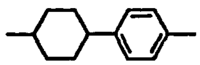 | 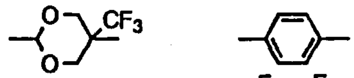 | | $-C_2H_5$ |
| 478 | $C_3H_7$— | 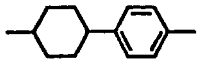 | 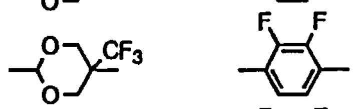 | | $-C_2H_5$ |
| 479 | $C_5H_{11}$— | 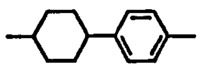 | 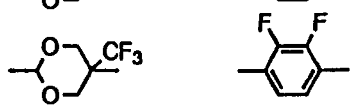 | | $-OC_2H_5$ |
| 480 | $C_3H_7$— | 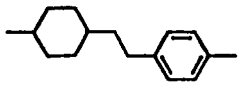 | 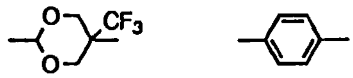 | | $-C_3H_7$ |
| 481 | $C_3H_7$— | 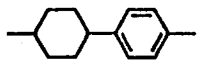 | 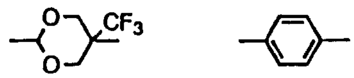 | | $-C_2H_5$ |
| 482 | $C_3H_7$— | 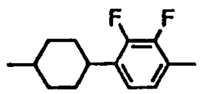 | 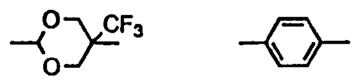 | | $-C_5H_{11}$ |
| 483 | 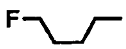 | 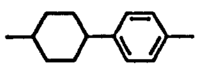 | 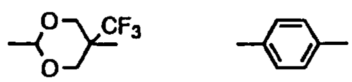 | | $-C_2H_5$ |
| 484 | 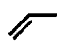 | 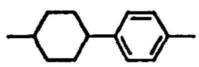 | 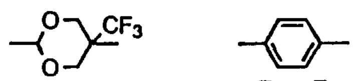 | | $-C_3H_7$ |
| 485 | 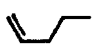 | 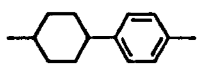 | 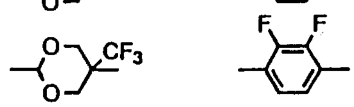 | | $-C_2H_5$ |
| 486 | 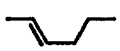 | 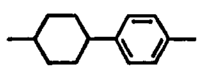 | 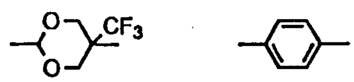 | | $-C_2H_5$ |
| 487 | $C_3H_7O$— | 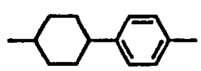 | 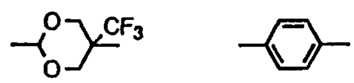 | | $-C_2H_5$ |
| 488 | 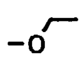 | 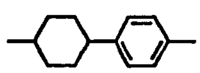 | 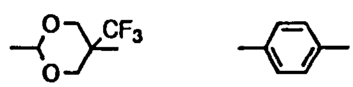 | | $-C_2H_5$ |
| 489 | $C_3H_7$— | 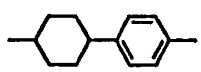 | 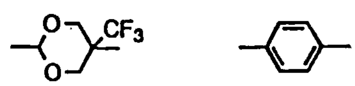 | | $-OCF_2CFHCF_3$ |
| 490 | $C_3H_7$— | 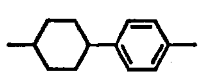 | 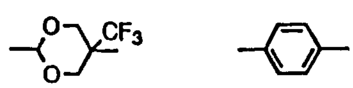 | | $-OCF_2CF_2Cl$ |

| Compound No. | $R_1$–• | •$\{A_1\}_k$–$Z_1$–$\{A_2\}_l$–$Z_2$–• | 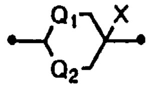 | •–$Z_3$–$\{A_3\}_m$–$Z_4$–$\{A_4\}_n$• | •–$R_2$ |
|---|---|---|---|---|---|
| 491 | $C_2H_5$– | 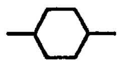 | 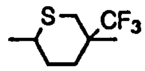 | | $-C_2H_5$ |
| 492 | $C_3H_7$– | 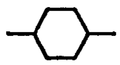 | 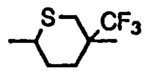 | | $-C_2H_5$ |
| 493 | $C_5H_{11}$– | 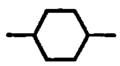 | 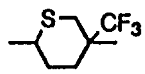 | | $-C_2H_5$ |
| 494 | $C_3H_7$– | 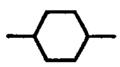 | 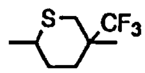 | | $-C_3H_7$ |
| 495 | $C_3H_7$– | 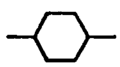 | 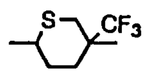 | | $-C_5H_{11}$ |
| 496 | $C_5H_{11}$– | 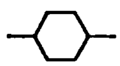 | 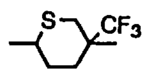 | | $-C_5H_{11}$ |
| 497 | 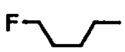 | 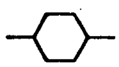 | 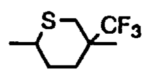 | | $-C_2H_5$ |
| 498 | 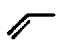 | 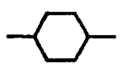 | 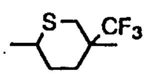 | | $-C_3H_7$ |
| 499 | 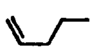 | 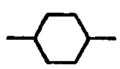 | 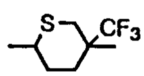 | | $-C_2H_5$ |
| 500 | 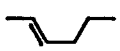 | 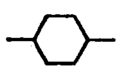 | 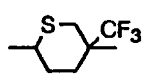 | | $-C_2H_5$ |
| 501 | $C_3H_7O$– | 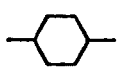 | 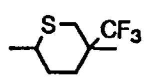 | | $-C_2H_5$ |
| 502 | 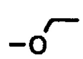 | 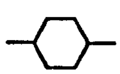 | 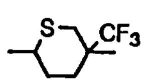 | | $-C_2H_5$ |
| 503 | $C_3H_7$– | 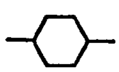 | 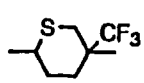 | | $-OCF_2CFHCF_3$ |
| 504 | $C_3H_7$– | 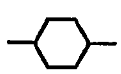 | 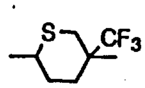 | | $-OCF_2CF_2Cl$ |

| Compound No. | $R_1$—• | •$-(A_1)_k-Z_1-(A_2)_l-Z_2$—• | 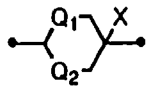 | •—$Z_3-(A_3)_m-Z_4-(A_4)_n$-• | •—$R_2$ |
|---|---|---|---|---|---|
| 505 | $C_2H_5$— | | 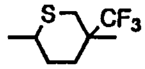 | 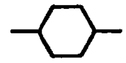 | $-C_2H_5$ |
| 506 | $C_3H_7$— | | 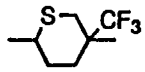 | 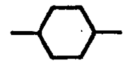 | $-C_2H_5$ |
| 507 | $C_5H_{11}$— | | 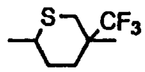 | 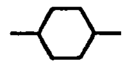 | $-C_2H_5$ |
| 508 | $C_3H_7$— | | 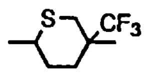 | 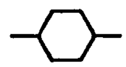 | $-C_3H_7$ |
| 509 | $C_3H_7$— | | 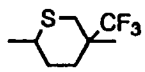 | 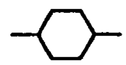 | $-C_5H_{11}$ |
| 510 | $C_5H_{11}$— | | 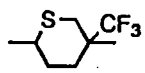 | 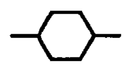 | $-C_5H_{11}$ |
| 511 | 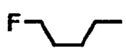 | | 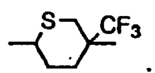 | 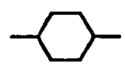 | $-C_2H_5$ |
| 512 | 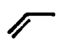 | | 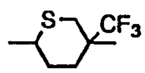 | 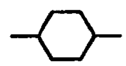 | $-C_3H_7$ |
| 513 | 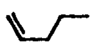 | | 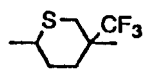 | 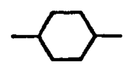 | $-C_2H_5$ |
| 514 | 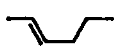 | | 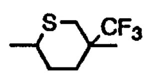 | 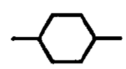 | $-C_2H_5$ |
| 515 | $C_3H_7O$— | | 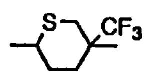 | 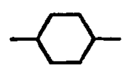 | $-C_2H_5$ |
| 516 | 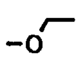 | | 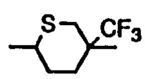 | 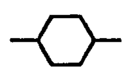 | $-C_2H_5$ |
| 517 | $C_3H_7$— | | 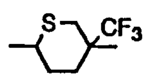 | 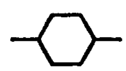 | $-OCF_2CFHCF_3$ |
| 518 | $C_3H_7$— | | 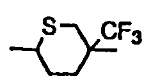 | 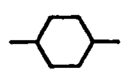 | $-OCF_2CF_2Cl$ |

| Compound No. | $R_1$— | 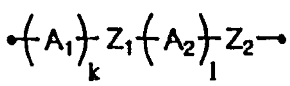 | 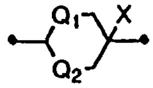 | 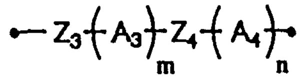 | —$R_2$ |
|---|---|---|---|---|---|
| 519 | $C_2H_5$— | 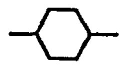 | 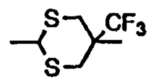 | | $-C_2H_5$ |
| 520 | $C_3H_7$— | 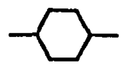 | 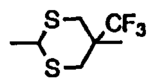 | | $-C_2H_5$ |
| 521 | $C_5H_{11}$— | 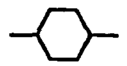 | 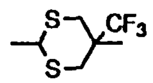 | | $-C_2H_5$ |
| 522 | $C_3H_7$— | 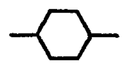 | 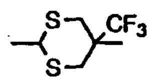 | | $-C_3H_7$ |
| 523 | $C_3H_7$— | 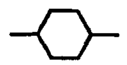 | 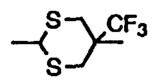 | | $-C_5H_{11}$ |
| 524 | $C_5H_{11}$— | 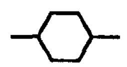 | 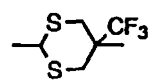 | | $-C_5H_{11}$ |
| 525 | 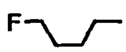 | 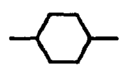 | 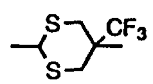 | | $-C_2H_5$ |
| 526 | 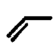 | 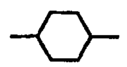 | 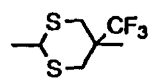 | | $-C_3H_7$ |
| 527 | 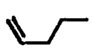 | 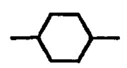 | 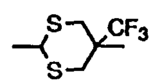 | | $-C_2H_5$ |
| 528 | 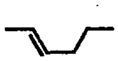 | 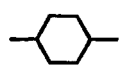 | 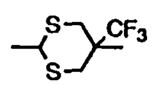 | | $-C_2H_5$ |
| 529 | $C_3H_7O$— | 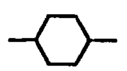 | 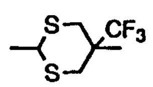 | | $-C_2H_5$ |
| 530 | 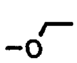 | 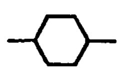 | 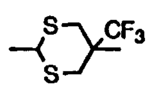 | | $-C_2H_5$ |
| 531 | $C_3H_7$— | 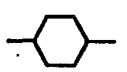 | 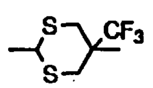 | | $-OCF_2CFHCF_3$ |
| 532 | $C_3H_7$— | 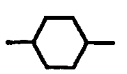 | 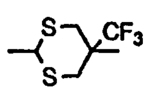 | | $-OCF_2CF_2Cl$ |

| Compound No. | $R_1$— | $-(A_1)_k-Z_1-(A_2)_l-Z_2-$ | 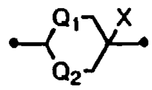 | $-Z_3-(A_3)_m-Z_4-(A_4)_n-$ | $-R_2$ |
|---|---|---|---|---|---|
| 533 | $C_2H_5$— | | 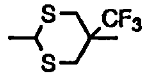 | 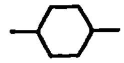 | $-C_2H_5$ |
| 534 | $C_3H_7$— | | 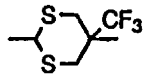 | 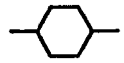 | $-C_2H_5$ |
| 535 | $C_5H_{11}$— | | 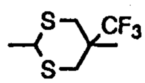 | 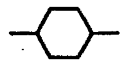 | $-C_2H_5$ |
| 536 | $C_3H_7$— | | 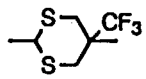 | 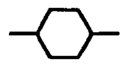 | $-C_3H_7$ |
| 537 | $C_3H_7$— | | 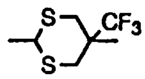 | 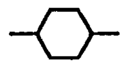 | $-C_5H_{11}$ |
| 538 | $C_5H_{11}$— | | 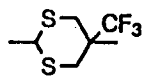 | 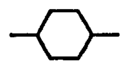 | $-C_5H_{11}$ |
| 539 | 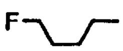 | | 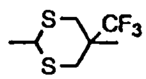 | 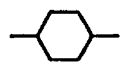 | $-C_2H_5$ |
| 540 | 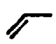 | | 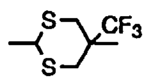 | 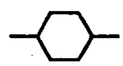 | $-C_3H_7$ |
| 541 | 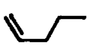 | | 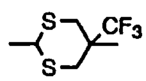 | 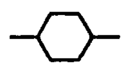 | $-C_2H_5$ |
| 542 | 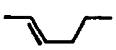 | | 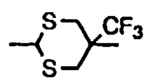 | 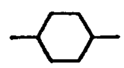 | $-C_2H_5$ |
| 543 | $C_3H_7O$— | | 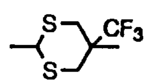 | 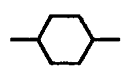 | $-C_2H_5$ |
| 544 | 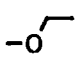 | | 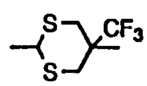 | 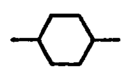 | $-C_2H_5$ |
| 545 | $C_3H_7$— | | 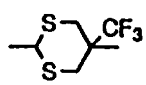 | 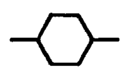 | $-OCF_2CFHCF_3$ |
| 546 | $C_3H_7$— | | 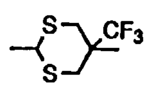 | 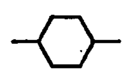 | $-OCF_2CF_2Cl$ |

| Compound No. | $R_1$—• | 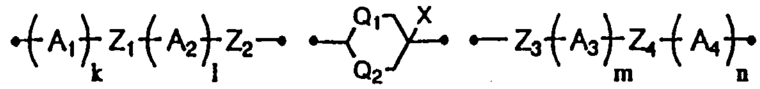 | •$-R_2$ |
|---|---|---|---|
| 547 | $C_5H_{11}$— | 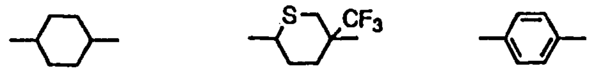 | $-C_2H_5$ |
| 548 | $C_3H_7$— | 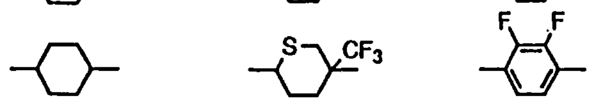 | $-C_2H_5$ |
| 549 | $C_5H_{11}$— | 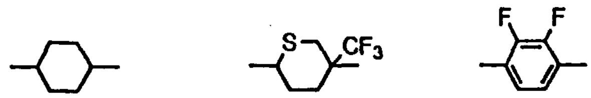 | $-OC_2H_5$ |
| 550 | $C_3H_7$— | 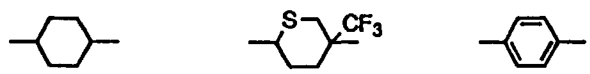 | $-C_3H_7$ |
| 551 | $C_3H_7$— | 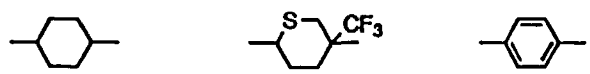 | $-C_2H_5$ |
| 552 | $C_3H_7$— | 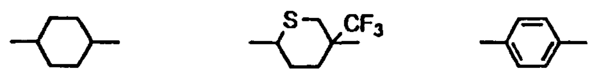 | $-C_5H_{11}$ |
| 553 | 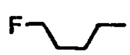 | 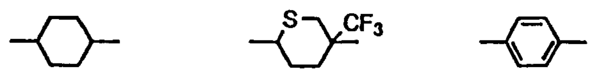 | $-C_2H_5$ |
| 554 | 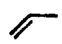 | 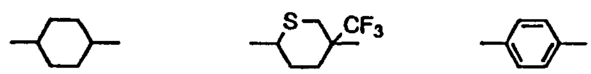 | $-C_3H_7$ |
| 555 | 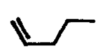 | 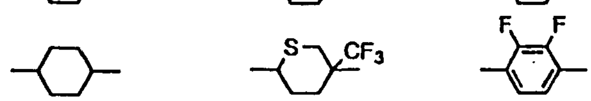 | $-C_2H_5$ |
| 556 | 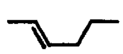 | 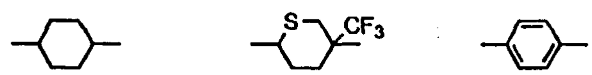 | $-C_2H_5$ |
| 557 | $C_3H_7O$— | 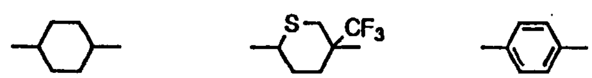 | $-C_2H_5$ |
| 558 | 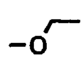 | 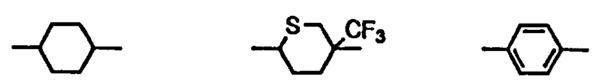 | $-C_2H_5$ |
| 559 | $C_3H_7$— | 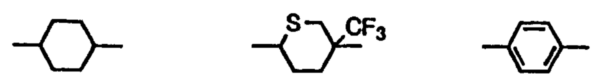 | $-OCF_2CFHCF_3$ |
| 560 | $C_3H_7$— | 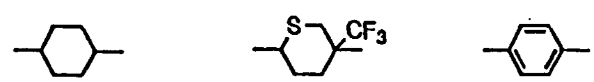 | $-OCF_2CF_2Cl$ |

| Compound No. | 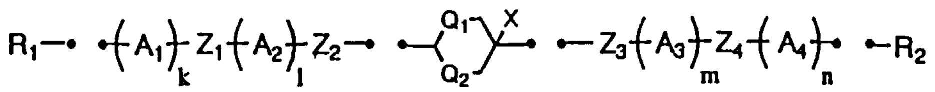 | | | | |
|---|---|---|---|---|---|
| 561 | $C_5H_{11}-$ | 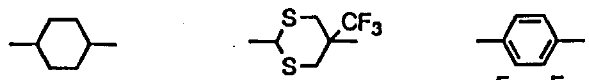 | | | $-C_2H_5$ |
| 562 | $C_3H_7-$ | 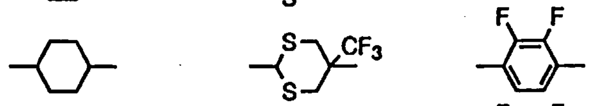 | | | $-C_2H_5$ |
| 563 | $C_5H_{11}-$ | 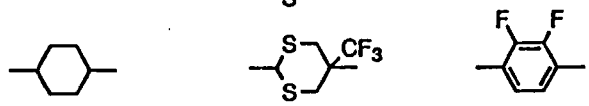 | | | $-OC_2H_5$ |
| 564 | $C_3H_7-$ | 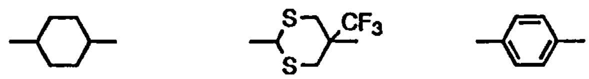 | | | $-C_3H_7$ |
| 565 | $C_3H_7-$ | 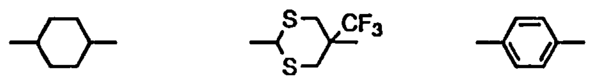 | | | $-C_2H_5$ |
| 566 | $C_3H_7-$ | 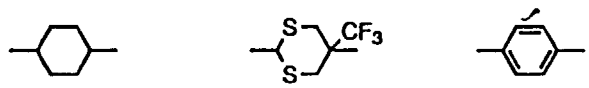 | | | $-C_5H_{11}$ |
| 567 | 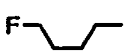 | 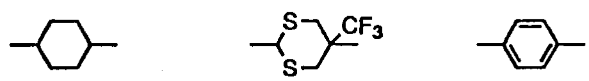 | | | $-C_2H_5$ |
| 568 | 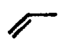 | 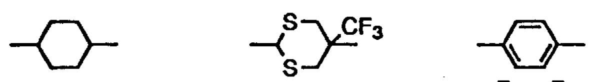 | | | $-C_3H_7$ |
| 569 | 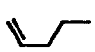 | 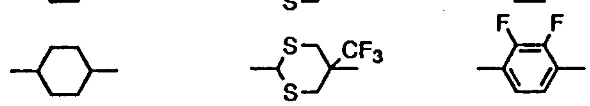 | | | $-C_2H_5$ |
| 570 | 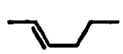 | 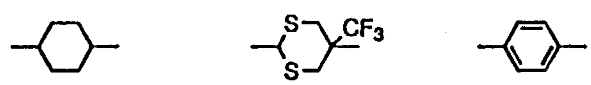 | | | $-C_2H_5$ |
| 571 | $C_3H_7O-$ | 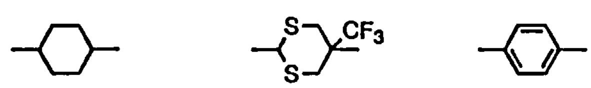 | | | $-C_2H_5$ |
| 572 | 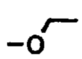 | 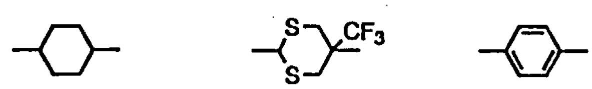 | | | $-C_2H_5$ |
| 573 | $C_3H_7-$ | 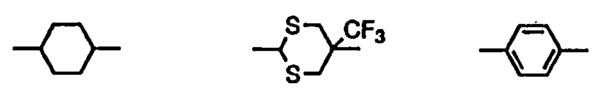 | | | $-OCF_2CFHCF_3$ |
| 574 | $C_3H_7-$ | 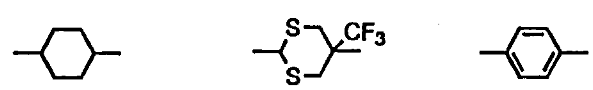 | | | $-OCF_2CF_2Cl$ |

Effect of the Invention

As clear from the above-mentioned Examples and Use Examples, the compounds according to the present invention have

1) high dielectric anisotropic values in minor axial direction,
2) high voltage-holding ratio and low temperature dependency, as well as

3) good miscibility with other known liquid crystalline compounds, particularly miscibility at low temperatures.

There can be provided liquid crystalline compounds and liquid crystalline compositions suitable as liquid crystalline materials showing negative dielectric anisotropic properties with high reliability, which also have high dielectric anisotropic values in minor axial directions, high voltage-holding ratio and low temperature-dependencies, by using the compounds according to the invention as components of the liquid crystalline compositions.

## Claims

1. A liquid crystalline compound expressed by the general formula (1)

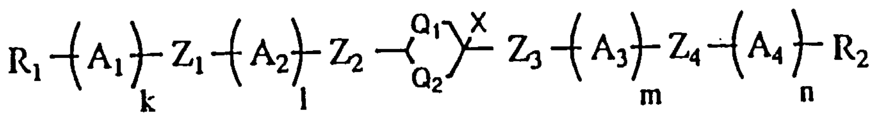 (1)

$$\bullet\!-A_5-Z_5-A_6-\!\bullet \qquad (1\text{-}1)$$

wherein, $R_1$ and $R_2$ are each independently denote alkyl group having from 1 to 10 carbon atom(s) in which one $CH_2$ group or more than one non-adjacent $CH_2$ groups may be substituted with (an) oxygen atom(s), (a) sulfur atom(s), $-SiH_2-$, -CH=CH- or -C≡C- and furthermore (an) optional hydrogen atom(s) in the group may be substituted with (a) halogen atom(s);

$Z_1$, $Z_2$, $Z_3$ and $Z_4$ each independently denote $-CH_2CH_2-$, -CH=CH-, -C≡C-, $-CH_2O-$, $-OCH_2-$, $-CF_2O-$, $-OCF_2-$, -COO-, -OCO-, $-(CH_2)_4-$or covalent bond;

$A_1$ and $A_3$ each independently denote 1,4-cyclohexylene group, 1,3-dioxane-2,5-diyl group, 1,4-phenylene group optionally substituted with one or more halogen atom(s), pyridine-2,5-diyl group, piperidine-1,4-diyl group, 1,4-bicyclo[2.2.2]octylene group, pyrimidine-2,5-diyl group, naphthalpne-2,6-diyl group, decahydronaphthalene-2,6-diyl group or 1,2,3,4-tetrahydronaphthalene-2,6-diyl group;

$A_2$ and $A_4$ each independently denote 1,4-cyclohexylene group, 1,3-dioxane-2,5-diyl group, 1,4-phenylene group optionally substituted with one or more halogen atom(s), pyridine-2,5-diyl group, piperidine-1,4-diyl group, 1,4-bicyclo[2.2.2]octylene group, pyrimidine-2,5-diyl group, naphthalene-2,6-diyl group, decahydronaphthalene-2,6-diyl group, 1,2,3,4-tetrahydronaphthalene-2,6-diyl group or a group expressed by the general formula (1-1);

$A_5$ and $A_6$ in the group each independently denote 1,4-cyclohexylene group, 1,3-dioxane-2,5-diyl group, 1,4-phenylene group optionally substituted with one or more halogen atom(s), pyridine-2,5-diyl group, piperidine-1,4-diyl group, 1,4-bicyclo[2.2.2]octylene group, pyrimidine-2,5-diyl group, naphthalene-2,6-diyl group, decahydronaphthalene-2,6-diyl group or 1,2,3,4-tetrahydronaphthalene-2,6-diyl group;

$Z_5$ denotes $-CH_2CH_2-$, -CH=CH-, -C≡C-, $-CH_2O-$, $-OCH_2-$, $-CF_2O-$, $-OCF_2-$, -COO-, -OCO-, - $(CH_2)_4$- or single bond;

$Q_1$ and $Q_2$ each independently denote $CH_2$ group, (an) oxygen atom(s) or (a) sulfur atom(s);

X denotes halogen atom, cyano group, haloalkyl group having from 1 to 10 carbon atom(s) or haloalkoxy group if either or both of $Q_1$ and $Q_2$ being (an) oxygen atom(s) or (a) sulfur atom(s) and denotes haloalkyl groups or haloalkoxy group having from 1 to 10 carbon atom(s) if both of $Q_1$ and $Q_2$ being $CH_2$ groups;

k, l, m and n each independently 0 or 1, provided that $l+k+m+n \leqq 3$ ,

provided that each elements constituting the compound may be substituted with their isotopic elements.

2. A liquid crystalline compound according to Claim 1, wherein both of $Q_1$ and $Q_2$ being $CH_2$ groups in the general formula (1).

3. A liquid crystalline compound according to Claim 2, wherein both of $Q_1$ and $Q_2$ being $CH_2$ groups and X being $CF_3$ in the general formula (1).

4. A liquid crystalline compound according to Claim 2, wherein both of $Q_1$ and $Q_2$ being $CH_2$ groups and X being

$OCF_3$ group in the general formula (1).

**5.** A liquid crystalline compound according to Claim 1, wherein both of $Q_1$ and $Q_2$ being oxygen atoms in the general formula (1).

**6.** A liquid crystalline compound according to Claim 5, wherein both of $Q_1$ and $Q_2$ being oxygen atoms and X being a halogen atom in the general formula (1).

**7.** A liquid crystalline compound according to Claim 1, wherein both of $Q_1$ and $Q_2$ being sulfur atoms in the general formula (1).

**8.** A liquid crystalline compound according to Claim 1, wherein both of $Q_1$ being $CH_2$ groups and $Q_2$ being an oxygen atom in the general formula (1).

**9.** A liquid crystalline compound according to Claim 1, wherein both of $Q_1$ being $CH_2$ groups and $Q_2$ being a sulfur atom in the general formula (1).

**10.** A liquid crystalline composition characterized in that one or more compound(s) expressed by the general formula (1) is(are) contained.

**11.** A liquid crystalline composition characterized in that one or more compound(s) according to Claims 1 to 9 is(are) contained as the first component, and one or more compound(s) selected from the group consisting of the general formulae (2), (3) and (4):

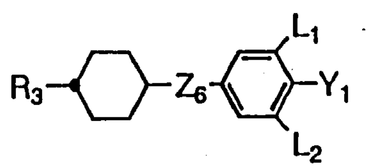

(2)

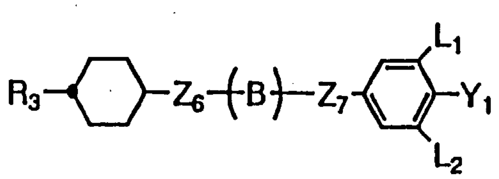

(3)

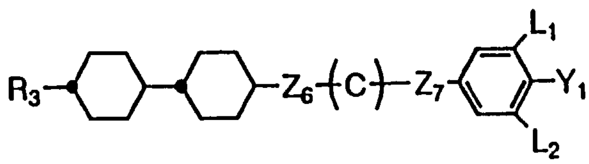

(4)

wherein, $R_3$ denotes an alkyl group having from 1 to 10 carbon atom(s) in which (an) optional non-adjacent methylene group(s) in the alkyl group may be substituted with (an) oxygen atom(s) or -CH=CH- and also (an) optional hydrogen atom(s) in the group may be substituted with (a) fluorine atom(s);
$Y_1$ denotes fluorine atom, chlorine atom, $OCF_3$, $OCF_2H$, $CF_3$, $CF_2H$, $CFH_2$, $OCF_2CF_2H$ or $OCF_2CFHCF_3$;
$L_1$ and $L_2$ each independently denote hydrogen atom or fluorine atom; $Z_6$ and $Z_7$ each independently denote 1,2-ethylene group, 1,4-butylene group, -COO-, $-CF_2O-$, $-OCF_2-$, -CH=CH- or covalent bond;
ring B denotes trans-1,4-cyclohexylene, 1,3-dioxane-2,5-diyl or 1,4-phenylene in which (a) hydrogen atom(s) may be substituted with (a) fluorine atom(s);
ring C denotes trans-1,4-cyclohexylene or 1,4-phenylene in which (a) hydrogen atom(s) may be substituted with (a) fluorine atom(s); and
each atoms used in the formulae can include their isotopes, is(are) contained as the second component.

**12.** A liquid crystalline composition characterized in that one or more compound(s) according to Claims 1 to 9 is(are) contained as the first component, and one or more compound(s) selected from the group consisting of the general formulae (5) and (6):

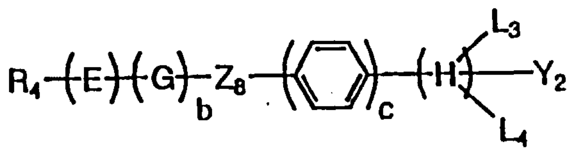

(5)

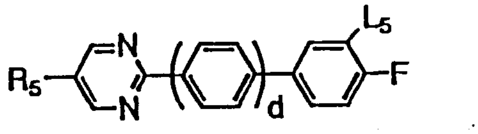

(6)

wherein, $R_4$ and $R_5$ each independently denote alkyl group having from 1 to 10 carbon atom(s) in which (an) optional non-adjacent methylene group(s) in the alkyl group may be substituted with (an) oxygen atom(s) or -CH=CH- and also (an) optional hydrogen atom(s) in the group may be substituted with (a) fluorine atom(s);
$Y_2$ denotes -CN group or -C≡C-CN;
ring E denotes trans-1,4-cyclohexylene, 1,4-phenylene, 1,3-dioxane-2,5-diyl or pyrimidine-2,5-diyl;
ring G denotes trans-1,4-cyclohexylene, 1,4-phenylene in which (a) hydrogen atom(s) may be substituted with (a) fluorine atom(s) or pyrimidine-2,5-diyl;
ring H denotes trans-1,4-cyclohexylene or 1,4-phenylene;
$Z_8$ denotes 1,2-ethylene group, -COO- or covalent bond;
$L_3$, $L_4$ and $L_5$ each independently donote hydrogen atom or fluorine atom;
b, c and d each independently denote 0 or 1; and
each atoms used in the formulae can include their isotopes, is(are) contained as the second component.

**13.** A liquid crystalline composition characterized in that one or more compound(s) according to Claims 1 to 9 is(are) contained as the first component, and one or more compound(s) selected from the group consisting of the general formulae (7), (8) and (9):

$$R_6\text{-}(I)\text{-}Z_9\text{-}(J)\text{-}Z_{10}\text{—}R_7 \quad (7)$$

$$R_6\text{-}(I)\text{-}Z_9\text{-}(J)\text{-}Z_{10}\text{-}(K)\text{-}R_7 \quad (8)$$

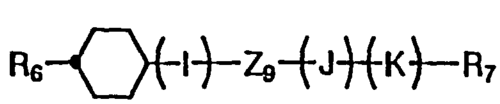

(9)

wherein, $R_6$ and $R_7$ each independently denote alkyl group having from 1 to 10 carbon atom(s) in which (an) optional non-adjacent methylene group(s) in the alkyl group may be substituted with (an) oxygen atom(s) or -CH=CH- and also (an) optional hydrogen atom(s) in the group may be substituted with (a) fluorine atom(s);
I, J and K each independently denote trans-1,4-cyclohexylene, pyrimidine-2,5-diyl or 1,4-phenylene in which (a) hydrogen atom(s) may be substituted with (a) fluorine atom(s);
$Z_4$ and $Z_5$ each independently denote -C≡C-, -COO-, $-CH_2CH_2-$, -CH=CH- or covalent bond; and
each atoms used in the formulae can include their isotopes, is(are) contained as the second component.

**14.** A liquid crystalline composition characterized in that one compound according to Claims 1 to 9 is contained as the first component, and one or more compound(s) selected from the group consisting of the general formulae (10), (11) and (12):

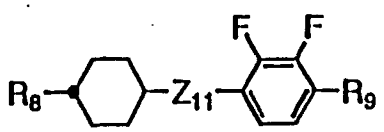

(10)

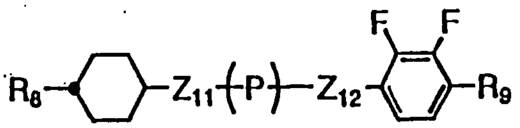

(11)

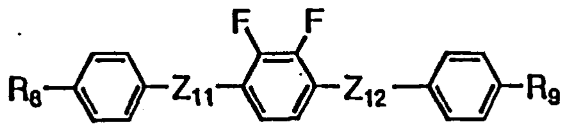

(12)

wherein, $R_8$ and $R_9$ each independently denote alkyl group having from 1 to 10 carbon atom(s) in which (an) optional non-adjacent methylene group(s) in the alkyl group may be substituted with (an) oxygen atom(s) or -CH=CH- and also (an) optional hydrogen atom(s) in the group may be substituted with (a) fluorine atom(s);
P each independently denote trans-1,4-cyclohexylene or 1,4-phenylene; $Z_{11}$ and $Z_{12}$ each independently denote $-CH_2CH_2-$, $-CH_2O-$ or covalent bond; and
each atoms used in the formulae can include their isotopes, is(are) contained as the second component.

**15.** A liquid crystalline composition characterized in that one or more compound(s) according to Claims 1 to 9 is(are) contained as the first component, one or more compound(s) selected from the group consisting of the general formulae (7), (8) and (9) is(are) contained as the second component, as well as one or more compound(s) selected from the group consisting of the general formulae (10), (11) and (12) is(are) contained as the third component.

**16.** A liquid crystalline composition characterized in that one or more compound(s) according to Claims 1 to 9 is (are) contained as the first component, one or more compound(s) selected from the group consisting of the general formulae (2), (3) and (4) is(are) contained as the second component, as well as one or more compound(s) selected from the group consisting of the general formulae (7), (8) and (9) is(are) contained as the third component.

**17.** A liquid crystalline composition characterized in that one or more compound(s) according to Claims 1 to 9 is(are) contained as the first component, one or more compound(s) selected from the group consisting of the general formulae (5) and (6) is(are) contained as the second component, as well as one or more compound(s) selected from the group consisting of the general formulae (7), (8) and (9) is(are) contained as the third component.

**18.** A liquid crystalline composition characterized in that one or more compound(s) according to Claims 1 to 9 being contained as the first component, one or more compound(s) selected from the group consisting of the general formulae (2), (3) and (4) is(are) contained as the second component, one or more compound(s) selected from the group consisting of the general formulae (5) and (6) is(are) contained as the third component, as well as one or more compound(s) selected from the group consisting of the general formulae (7), (8) and (9) is(are) contained as the fourth component.

**19.** A liquid crystalline composition characterized in that one or more optically active compound(s) is(are) contained in addition to any of the liquid crystalline composition according to Claims 10 to 18.

**20.** A liquid crystalline display element constituted by using the liquid crystalline composition according to any of Claims 10 to 19.

## INTERNATIONAL SEARCH REPORT

International application No.
PCT/JP97/03503

A. CLASSIFICATION OF SUBJECT MATTER Int. Cl[6] C07C22/08, 43/172, 43/192, 43/225, C07D239/26, 309/04, 309/06, 319/06, 335/02, 339/08, C09K19/30, 19/34, 19/42, G02F1/13
According to International Patent Classification (IPC) or to both national classification and IPC

B. FIELDS SEARCHED

Minimum documentation searched (classification system followed by classification symbols) Int. Cl[6] C07C22/08, 43/172, 43/192, 43/225, C07D239/26, 309/04, 309/06, 319/06, 335/02, 339/08, C09K19/30, 19/34, 19/42, G02F1/13

Documentation searched other than minimum documentation to the extent that such documents are included in the fields searched

Electronic data base consulted during the international search (name of data base and, where practicable, search terms used)
CAS ONLINE

C. DOCUMENTS CONSIDERED TO BE RELEVANT

| Category* | Citation of document, with indication, where appropriate, of the relevant passages | Relevant to claim No. |
|---|---|---|
| A | JP, 5-505828, A (Hoechst AG.), August 26, 1993 (26. 08. 93) & WO, 92/12218, A1 & US, 5370823, A | 1 - 20 |
| A | JP, 6-56718, A (Dainippon Ink & Chemicals Inc.), March 1, 1994 (01. 03. 94)(Family: none) | 1 - 20 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| December 12, 1997 (12. 12. 97) | December 24, 1997 (24. 12. 97) |
| Name and mailing address of the ISA/ Japanese Patent Office | Authorized officer |
| Facsimile No. | Telephone No. |

Form PCT/ISA/210 (second sheet) (July 1992)